# EUROPEAN PATENT APPLICATION

(11) **EP 4 382 521 A1**
(43) Date of publication of application: **12.06.2024**
(21) Application number: 22852319.7
(22) Date of filing: 04.08.2022
(51) Int. Cl.: C07D 401/12, C07D 405/14, C07D 409/14, C07D 417/14, A61K 31/495, A61P 25/00, A61P 25/02, A61P 25/04, A61P 25/08, A61P 25/16, A61P 25/22, A61P 25/24

(54) **REGULATOR CONTAINING TRICYCLIC DERIVATIVE, PREPARATION METHOD THEREFOR AND APPLICATION THEREOF**

(30) Priority: 05.08.2021 CN 202110897787
(71) Applicant: Shujing Biopharma Co., Ltd, Shanghai 201208 (CN); Jiangsu NHWA Pharmaceutical Co., Ltd, Xuzhou, Jiangsu 221000 (CN)
(72) Inventor: QIN, Jun, Shanghai 201208 (CN); WENG, Yangyang, Shanghai 201208 (CN); CAI, Jin, Shanghai 201208 (CN); WAN, Zehong, Shanghai 201208 (CN)
(74) Representative: Lavoix
(86) International application number: PCT/CN2022/110371
(87) International publication number: WO 2023/011608

(57) **Abstract**

The present application relates to a regulator containing a tricyclic derivative, a preparation method therefor and an application thereof, and in particular to a compound represented by general formula (Ia), a preparation method therefor and a pharmaceutical composition containing the compound, and an application thereof in the preparation of drugs for preventing and/or treating central nervous system diseases and/or peripheral diseases related to mammalian 5-hydroxytryptamine and/or dopamine and/or norepinephrine neurotransmitter transmission.

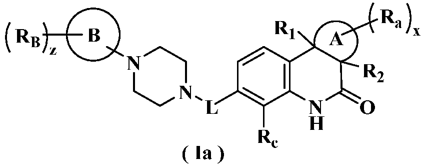

## Description

The present application claims priority to Chinese Patent Application No. 2021108977876 filed on Aug. 5, 2021, which is incorporated herein by reference in its entirety.

### TECHNICAL FIELD

The present invention belongs to the field of pharmaceutical chemistry, and particularly relates to a regulator containing a tricyclic derivative, a preparation method therefor, and use thereof.

### BACKGROUND

Schizophrenia is a disease characterized by severely schizophrenic cognition and emotion, manifesting as the influence on the basic behavior of a human, such as language, thinking, feeling, and self-perception. The symptoms of the disease are of a wide range, and the most common symptoms are mental disorders, such as hallucinations, delusions, and illusions. About 1% of people all over the world suffer from schizophrenia, while only 5% of all treated patients can eventually recover completely. In addition, complications, such as anxiety disorder, depression, or psychotropic drug abuse, are often caused by schizophrenia.

Drugs used to treat schizophrenia are collectively referred to as anti-schizophrenic drugs. Typical anti-schizophrenic drugs, i.e., conventional anti-schizophrenic drugs, are the first generation anti-schizophrenic drugs, which exert pharmacological effects mainly by blocking dopamine D₂ receptors. The typical anti-schizophrenic drugs include chlorpromazine, fluphenazine, haloperidol, thiothixene, stelazine, trilafon, mellaril, and the like. They are groundbreaking in treating positive symptoms of schizophrenia, but fail to treat negative symptoms and cognitive disorder. The typical anti-schizophrenic drugs generally have severe extrapyramidal syndrome (EPS) and tardive dyskinesia (TD) side effects and are ineffective in one third of schizophrenic patients.

In the 1990s, the second generation anti-schizophrenic drugs, referred to as atypical anti-schizophrenic drugs, i.e., novel anti-schizophrenic drugs, were introduced. The second generation anti-schizophrenic drugs commonly used in clinic include clozapine, risperidone, olanzapine, quetiapine, ziprasidone, and the like. Although their respective pharmacological effects are not completely consistent, they have a common pharmacological characteristic that the affinity for 5-hydroxytryptamine (5-HT) receptors (5-HT_{1A}, 5-HT_{2A}, 5-HT_{2B}, and 5-HT_{2C}) is far higher than that for D₂ receptors. Compared with the first generation anti-schizophrenic drugs, the second generation anti-schizophrenic drugs have more advantages in clinical effects. Not only are they as effective as traditional anti-schizophrenic drugs for positive symptoms, but they are also effective for negative symptoms and cognitive disorder symptoms, have a broader spectrum of action, and can significantly reduce the tendency of causing EPS and TD. However, these drugs still have adverse reactions such as QTc interval prolongation, hyperprolactinemia, weight gain, abnormal heart metabolism, and cardiovascular side effects. Therefore, it is a research hotspot to find drugs that are effective for positive and negative symptoms and cognitive disorder of schizophrenia and have small side effects.

The 5-hydroxytryptamine system plays an important role in regulating the functions of the prefrontal cortex (PFC), including emotion control, cognitive behavior, and working memory. The pyramidal neurons and GABA interneurons of PFC comprise several 5-hydroxytryptamine receptor subtypes, 5-HT_{1A} and 5-HT_{2A}, with particularly high densities. It has recently been demonstrated that the PFC and NMDA receptor channels are targets for 5-HT_{1AR}, and that these two receptors regulate cortical excitatory neurons and thus affect cognitive function. In fact, various preclinical data suggest that 5-HT_{1AR} may be a new therapeutic target for the development of anti-schizophrenic drugs. The high affinity for 5-HT_{1AR} and low EPS side effects of atypical anti-schizophrenic drugs (such as clozapine) suggest that the 5-hydroxytryptamine system plays an important role in regulating the functions of the prefrontal cortex (PFC), including emotion control, cognitive behavior, and working memory. The pyramidal neurons and GABA interneurons of PFC comprise several 5-hydroxytryptamine receptor subtypes, 5-HT_{1A} and 5-HT_{2A}, with particularly high densities. Recent research has shown that 5-HT_{1A} agonists are associated with atypical anti-schizophrenic drug therapy and can ameliorate the negative symptoms and cognitive disorder. In the treatment of schizophrenia with the atypical anti-schizophrenic drug clozapine, 5-HT_{2A} has been found to play a major role, involving various aspects of perception, emotion regulation, and motion control. Blocking of the 5-HT_{2A} receptor can regulate the function of dopamine, thereby ameliorating the positive symptoms of schizophrenia. In addition, 5-HT_{2B} receptor is closely associated with cardiovascular abnormalities, such as abnormal heart metabolism, pulmonary hypertension, fibrosis, and valvular disease, and the 5-HT_{2C} receptor is closely associated with weight gain.

The distribution of D₃ receptors in the brain is mainly and selectively distributed in the limbic system. There are two major DA neural pathways in the brain. One is nigrostriatal pathway regulating and controlling motor functions, and the other is mesencephalic ventral tegmental area-nucleus accumbens-prefrontal cortex DA pathway, which is closely associated with learning cognition and emotional activities, and its dysfunction will cause the abnormality of mental activities. This DA pathway is also the major pathway of reward effects in the brain. D_{3R} is distributed in both of the DA neural pathways, and has a complex interaction with other DA receptor subtypes, thus it may be the target of antipsychotic drug therapy. Selective D₃ receptor antagonists can ameliorate dyskinesia caused by the administration of levodopa in patients with Parkinson's disease. Therefore, the search for an anti-schizophrenic drug which binds to multiple receptors and has fewer side effects is of great significance for clinical treatment.

Aripiprazole is another atypical anti-schizophrenic drug widely used in the clinic. It has a partial agonistic effect on a D₂ receptor, an agonistic effect on 5-HT_{1A} receptor, an antagonistic effect on 5-HT_{2A} receptor, and lower affinity for 5-hydroxytryptamine (5-HT) receptors (5-HT_{1A} and 5-HT_{2A}) than for the D₂ receptor. Due to its unique pharmacological mechanism, aripiprazole is not only clinically effective in treating the positive symptoms of schizophrenia, but also can improve the negative symptoms and cognitive disorder of patients, and does not cause side effects such as extrapyramidal syndrome, and tardive dyskinesia. Although aripiprazole has some advantages in efficacy as a new generation of atypical anti-schizophrenic drugs, it still has many adverse reactions in clinic. More than 10% of patients have adverse reactions including weight gain, headache, dizziness, akathisia, insomnia, and gastrointestinal discomfort, which will cause the patients to stop taking the drug and the conditions to recur. In addition, drugs for the negative symptoms of schizophrenia (referring to defects in normal emotional reactions and other thinking processes) have been currently applied in clinic, improving the negative symptoms of some patients, but overall, the effect is limited, and many patients cannot recover and restore normal social functions due to the negative symptoms, making it difficult to return to normal social work. In addition, the treatment of cognitive disorder is also a key point of the current treatment of schizophrenia, which affects verbal memory, semantic processing ability, and attention function of most patients suffering from schizophrenia, and the anti-schizophrenic drugs currently under development or on the market have very limited improvement on the cognitive function.

In addition to the problems described above, the current anti-schizophrenic drugs are still in a dilemma for the treatment of refractory schizophrenia. The refractory schizophrenia refers to a type of patients who cannot achieve ideal efficacy despite being treated according to a general method. This type of patients have been treated with three anti-schizophrenic drugs having different active ingredients, with a sufficient amount of drugs and sufficient courses of treatment, but the treatment response is not good or the patients are unable to tolerate the adverse reactions of the anti-schizophrenic drugs, or even if the patients have sufficient maintenance or preventive treatment, the conditions continue to recur or worsen. Therefore, the anti-refractory schizophrenia drugs have always been a challenge in clinical drug research and an urgent direction to be overcome.

Currently, RP5063 of Reviva, which has completed phase II clinical trials and is in the preparation of phase III clinical trials, is an atypical anti-schizophrenic drug and has an anti-schizophrenic effect by regulating the functions of dopamine and serotonin systems. It is a partial agonist of D₂ and D₃ receptors, an agonist of the 5-HT_{1A} receptor, and an antagonist of the 5-HT_{2A} receptor, and it exhibits antagonistic effects against 5-HT_{2B}, 5-HT_{2C}, and 5-HT₇. Compared with typical anti-schizophrenic drugs, RP5063, as a multi-target receptor regulator, has a certain improvement effect on the positive symptoms, negative symptoms, and cognitive disorder of schizophrenia, and can reduce the risk of some adverse reactions.

Therefore, there is an urgent need to develop an anti-schizophrenic drug which has a good and continuously effective treatment effect on the negative symptoms, improves the cognitive function of patients, can effectively treat refractory schizophrenia, has relatively low adverse drug reactions (such as extrapyramidal symptoms, tardive dyskinesia, hyperprolactinemia, weight gain, nausea and vomiting, abnormal heart metabolism, and cardiovascular abnormalities), and acts on multiple targets, so as to meet the huge market demand.

### SUMMARY

For the technical problem to be solved, the present invention provides a regulator containing a tricyclic derivative, a preparation method therefor, and use thereof.

The present invention aims to provide a compound of general formula (Ia), a stereoisomer thereof, or a pharmaceutically acceptable salt thereof: 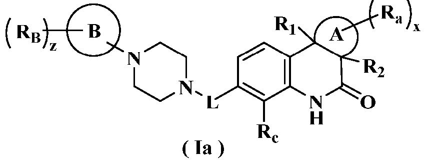 wherein:
L is -(CH₂)ₙ₁-, -(CH₂)ₙ₁O(CH₂)ₙ₂-, -(CH₂)ₙ₁S(CH₂)ₙ₂-, -(CH₂)ₙ₁NH(CH₂)ₙ₂-, -(CH₂)ₙ₁C(O)(CH₂)ₙ₂-, - (CH₂)ₙ₁S(O)₂(CH₂)ₙ₂-, -(CH₂)ₙ₁C(O)O(CH₂)ₙ₂-, -(CH₂)ₙ₁OC(O)(CH₂)ₙ₂-, -(CH₂)ₙ₁C(O)S(CH₂)ₙ₂-, - (CH₂)ₙ₁SC(O)(CH₂)ₙ₂-, -(CH₂)ₙ₁C(O)NH(CH₂)ₙ₂-, or -(CH₂)ₙ₁NHC(O)(CH₂)ₙ₂-;
ring A is C₃₋₆ cycloalkyl or 3- to 6-membered heterocyclyl;
Rₐ is hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb};
R₁ and R₂ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb};
ring B is C₆₋₁₂ aryl, 5- to 12-membered heteroaryl, C₆₋₁₂ arylo C₃₋₈ cycloalkyl, C₆₋₁₂ arylo 4- to 8-membered heterocyclyl, or C₆₋₁₂ arylo 5- to 10-membered heteroaryl;
R_{B} is hydrogen, deuterium, halogen, hydroxy, cyano, amino, C₁₋₆ alkoxy, C₁₋₆ alkyl, C₁₋₆ deuterated alkyl, C₁₋₆ haloalkyl, oxo, C(O)ORₐₐ, -NRₐₐR_{bb}, or -C(O)NRₐₐR_{bb};
R_{c} is hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb};
Rₐₐ and R_{bb} are each independently hydrogen, deuterium, hydroxy, cyano, amino, C₁₋₆ alkoxy, C₁₋₆ alkyl, C₁₋₆ deuterated alkyl, or C₁₋₆ haloalkyl;
x is an integer from 0 to 4;
z is an integer from 0 to 5; and
n1 and n2 are each independently an integer from 0 to 6.

In a further preferred embodiment of the present invention, L is -(CH₂)ₙ₁O-, -(CH₂)ₙ₁OCH₂-, - (CH₂)₂O(CH₂)ₙ₂-, -(CH₂)ₙ₁S-, -(CH₂)ₙ₁SCH₂-, -(CH₂)₂S(CH₂)ₙ₂-, -(CH₂)ₙ₁C(O)NH-, -(CH₂)ₙ₁C(O)NHCH₂-, - (CH₂)ₙ₁NHC(O)CH₂-, or -(CH₂)₂NHC(O)(CH₂)ₙ₂-, preferably -(CH₂)ₙ₁O-, -(CH₂)ₙ₁S-, or -(CH₂)ₙ₁C(O)NH-, more preferably -(CH₂)ₙ₁O-, and further preferably -(CH₂)₃O-, -(CH₂)₄O-, or -(CH₂)₅O- (the O end being linked to the benzene ring).

In a further preferred embodiment of the present invention, ring A is C₃₋₆ cycloalkyl, preferably 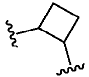 or

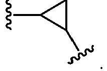

In a further preferred embodiment of the present invention, Rₐ is hydrogen, deuterium, halogen, or C₁₋₆ alkyl, preferably hydrogen, deuterium, fluorine, chlorine, bromine, or C₁₋₃ alkyl, more preferably hydrogen, deuterium, fluorine, or methyl, and further preferably hydrogen, deuterium, or fluorine.

In a further preferred embodiment of the present invention, R₁ and R₂ are each independently hydrogen, deuterium, halogen, cyano, or C₁₋₆ alkyl, preferably hydrogen, deuterium, fluorine, chlorine, bromine, cyano, or C₁₋₃ alkyl, and more preferably hydrogen, deuterium, fluorine, cyano, or methyl.

In a further preferred embodiment of the present invention, 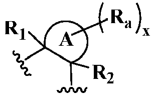 is a group as follows:

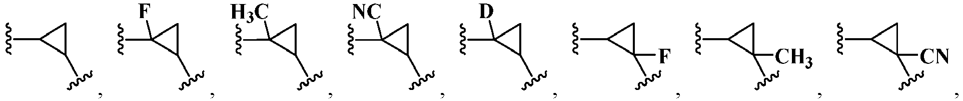

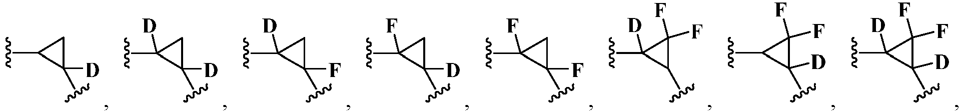

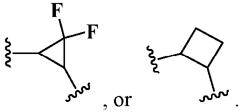

In a further preferred embodiment of the present invention, ring B is 5- to 6-membered heteroaryl, phenyl, benzo C₃₋₈ cycloalkyl, benzo 4- to 8-membered heterocyclyl, or benzo 5- to 6-membered heteroaryl, preferably 5- to 6-membered heteroaryl, phenyl, benzo C₅₋₆ cycloalkyl, benzo 5- to 6-membered heterocyclyl, or benzo 5- to 6-membered heteroaryl, and more preferably a group as follows:

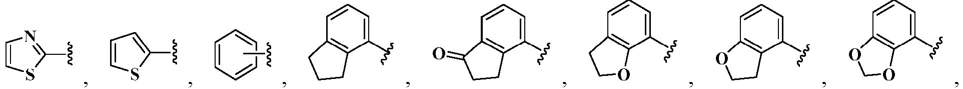

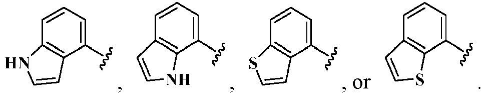

In a further preferred embodiment of the present invention, R_{B} is hydrogen, deuterium, halogen, cyano, hydroxy, oxo, C₁₋₃ alkoxy, or C₁₋₃ alkyl, preferably hydrogen, deuterium, fluorine, chlorine, bromine, cyano, hydroxy, oxo, methoxy, or methyl, and more preferably hydrogen, fluorine, chlorine, cyano, hydroxy, oxo, methoxy, or methyl.

In a further preferred embodiment of the present invention, R_{c} is hydrogen, deuterium, or halogen, preferably hydrogen, deuterium, fluorine, chlorine, or bromine, and more preferably hydrogen or fluorine.

In a further preferred embodiment of the present invention, Rₐₐ and R_{bb} are each independently selected from hydrogen, deuterium, C₁₋₆ alkyl, or C₁₋₆ deuterated alkyl, preferably hydrogen, deuterium, C₁₋₃ alkyl, or C₁₋₃ deuterated alkyl, and more preferably hydrogen, deuterium, methyl, ethyl, propyl, or isopropyl.

In a further preferred embodiment of the present invention, 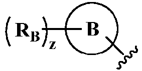 is a group as follows:

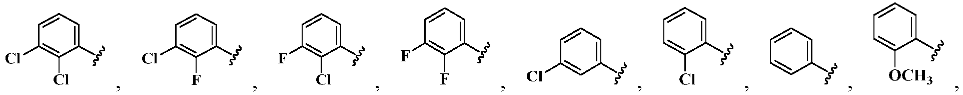

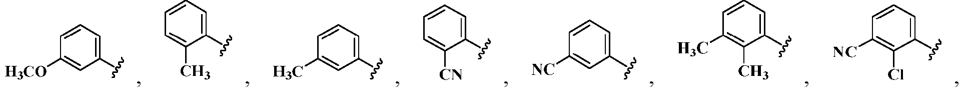

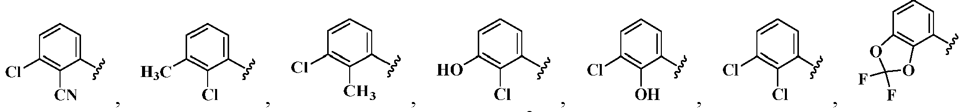

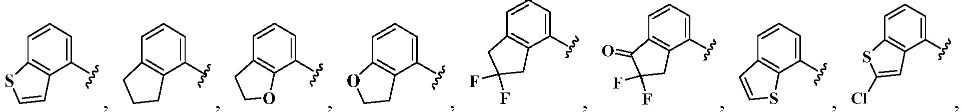

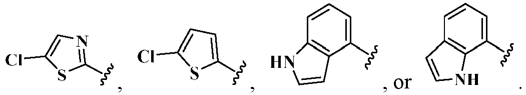

In a further preferred embodiment of the present invention, z is an integer from 0 to 3.

In a further preferred embodiment of the present invention, n1 is an integer from 3 to 5, preferably 4.

In a further preferred embodiment of the present invention, n2 is 0.

The present invention further provides a compound of general formula (I), a stereoisomer thereof, or a pharmaceutically acceptable salt thereof: 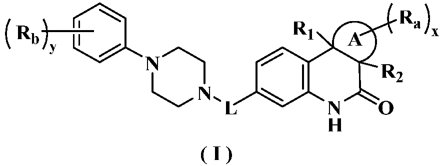 wherein:
L is -(CH₂)ₙ₁-, -(CH₂)ₙ₁O(CH₂)ₙ₂-, -(CH₂)ₙ₁S(CH₂)ₙ₂-, -(CH₂)ₙ₁NH(CH₂)ₙ₂-, -(CH₂)ₙ₁C(O)(CH2)ₙ₂-, - (CH₂)ₙ₁S(O)₂(CH₂)ₙ₂-, -(CH₂)ₙ₁C(O)O(CH₂)ₙ₂-, -(CH₂)ₙ₁OC(O)(CH₂)ₙ₂-, -(CH₂)ₙ₁C(O)S(CH₂)ₙ₂-, - (CH₂)ₙ₁SC(O)(CH₂)ₙ₂-, -(CH₂)ₙ₁C(O)NH(CH₂)ₙ₂-, or -(CH₂)ₙ₁NHC(O)(CH₂)ₙ₂-, preferably -(CH₂)ₙ₁O-, - (CH₂)ₙ₁OCH₂-, -(CH₂)₂O(CH₂)ₙ₂-, -(CH₂)ₙ₁S-, -(CH₂)ₙ₁SCH₂-, -(CH₂)₂S(CH₂)ₙ₂-, -(CH₂)ₙ₁C(O)NH-, - (CH₂)ₙ₁C(O)NHCH₂-, -(CH₂)ₙ₁NHC(O)CH₂-, or -(CH₂)₂NHC(O)(CH₂)ₙ₂-, more preferably -(CH₂)ₙ₁O-, - (CH₂)ₙ₁S-, or -(CH₂)ₙ₁C(O)NH-, further preferably -(CH₂)ₙ₁O-, and particularly preferably -(CH₂)₃O-, - (CH₂)₄O-, or -(CH₂)₅O- (the O end being linked to the benzene ring);
ring A is C₃₋₆ cycloalkyl or 3- to 6-membered heterocyclyl, preferably C₃₋₆ cycloalkyl, and more preferably 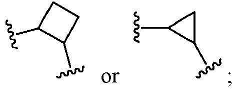
Rₐ is hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb}, preferably hydrogen, deuterium, halogen, or C₁₋₆ alkyl, more preferably hydrogen, deuterium, fluorine, chlorine, bromine, or C₁₋₃ alkyl, further preferably hydrogen, deuterium, fluorine, or methyl, and particularly preferably hydrogen, deuterium, or fluorine;
R₁ and R₂ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb}, preferably hydrogen, deuterium, halogen, cyano, or C₁₋₆ alkyl, more preferably hydrogen, deuterium, fluorine, chlorine, bromine, cyano, or C₁₋₃ alkyl, and further preferably hydrogen, deuterium, fluorine, cyano, or methyl;
R_{b} is hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb}, preferably hydrogen, deuterium, halogen, cyano, C₁₋₃ alkoxy, or C₁₋₃ alkyl, more preferably hydrogen, deuterium, fluorine, chlorine, bromine, cyano, methoxy, or methyl, and further preferably hydrogen, fluorine, chlorine, cyano, methoxy, or methyl;
Rₐₐ and R_{bb} are each independently hydrogen, deuterium, hydroxy, cyano, amino, C₁₋₆ alkoxy, C₁₋₆ alkyl, C₁₋₆ deuterated alkyl, or C₁₋₆ haloalkyl, preferably hydrogen, deuterium, C₁₋₆ alkyl, or C₁₋₆ deuterated alkyl, more preferably hydrogen, deuterium, C₁₋₃ alkyl, or C₁₋₃ deuterated alkyl, and further preferably hydrogen, deuterium, methyl, ethyl, propyl, or isopropyl;
x is an integer from 0 to 4;
y is an integer from 0 to 5, preferably an integer from 0 to 2; and
n1 and n2 are each independently an integer from 0 to 6; n1 is preferably an integer from 3 to 5, and more preferably 4; n2 is preferably 0.

In a further preferred embodiment of the present invention, R_{b} is hydroxy.

In a further preferred embodiment of the present invention, 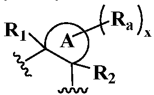 is a group as follows:

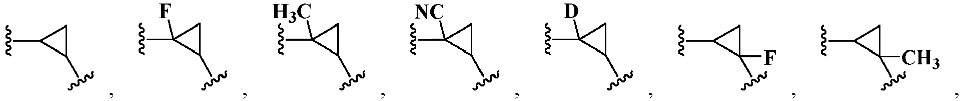

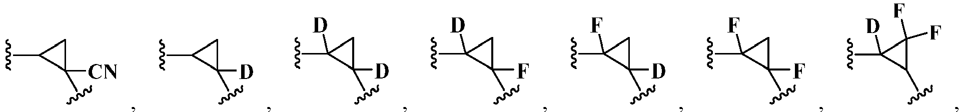

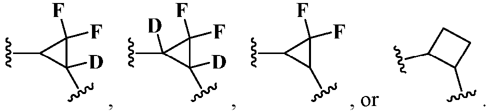

In a further preferred embodiment of the present invention, 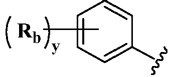 is a group as follows:

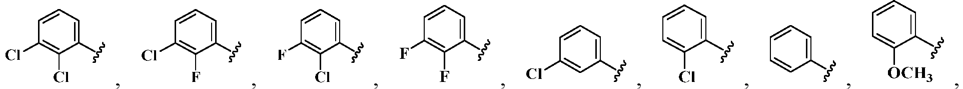

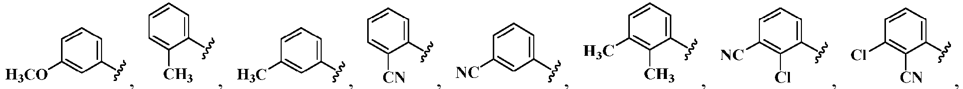

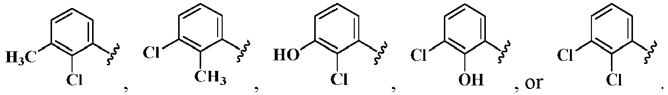

In a further preferred embodiment of the present invention, general formula (I) is further represented by general formula (II): 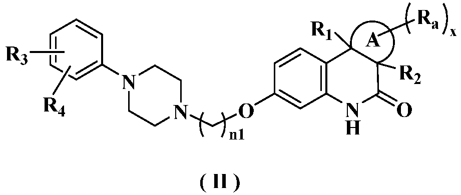 wherein:
R₃ and R₄ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb},
preferably hydrogen, deuterium, halogen, cyano, C₁₋₃ alkoxy, or C₁₋₃ alkyl,
more preferably hydrogen, deuterium, fluorine, chlorine, bromine, cyano, methoxy, or methyl,
and further preferably hydrogen, fluorine, chlorine, cyano, methoxy, or methyl;
Rₐₐ and R_{bb} are each independently hydrogen, deuterium, hydroxy, cyano, amino, C₁₋₆ alkoxy, C₁₋₆ alkyl, C₁₋₆ deuterated alkyl, or C₁₋₆ haloalkyl,
preferably hydrogen, deuterium, C₁₋₆ alkyl, or C₁₋₆ deuterated alkyl,
more preferably hydrogen, deuterium, C₁₋₃ alkyl, or C₁₋₃ deuterated alkyl,
and further preferably hydrogen, deuterium, methyl, ethyl, propyl, or isopropyl; and
n1 is an integer from 2 to 6, preferably an integer from 3 to 5, and more preferably 4;
ring A, R₁, R₂, Rₐ, x, and 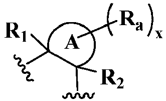 are as defined above.

In a further preferred embodiment of the present invention, R₃ and R₄ are each independently hydroxy.

In a further preferred embodiment of the present invention, 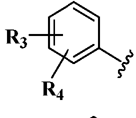 is a group as follows:

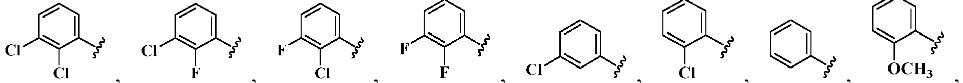

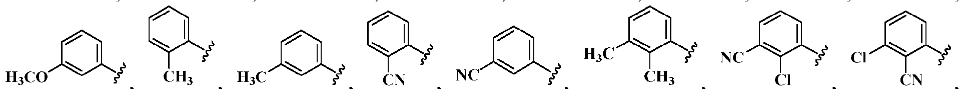

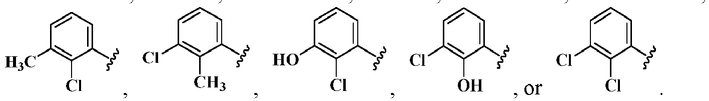

In a further preferred embodiment of the present invention, general formula (Ia) is further represented by general formula (III): 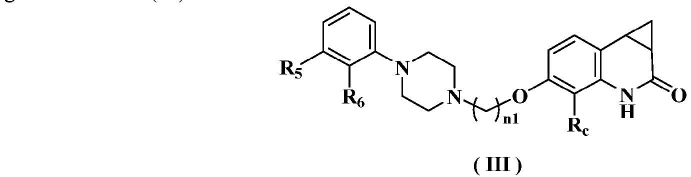 wherein:
R₅ and R₆ are each independently hydrogen, deuterium, halogen, cyano, hydroxy, C₁₋₃ alkoxy, or C₁₋₃ alkyl, preferably hydrogen, deuterium, fluorine, chlorine, bromine, cyano, hydroxy, methoxy, or methyl, and further preferably hydrogen, fluorine, chlorine, cyano, hydroxy, methoxy, or methyl;
or R₅ and R₆ are linked to the carbon atom to which they are attached to form C₃₋₈ cycloalkyl, 4- to 8-membered heterocyclyl, or 5- to 6-membered heteroaryl, the C₃₋₈ cycloalkyl, 4- to 8-membered heterocyclyl, and 5- to 6-membered heteroaryl optionally being further substituted with one or more substituents selected from hydrogen, deuterium, oxo and halogen,
preferably to form C₃₋₅ cycloalkyl, 5- to 6-membered heterocyclyl, or 5- to 6-membered heteroaryl, the C₃₋₅ cycloalkyl, 5- to 6-membered heterocyclyl, and 5- to 6-membered heteroaryl optionally being further substituted with one or more substituents selected from hydrogen, deuterium, oxo, fluorine, chlorine, and bromine,
more preferably to form a group as follows: 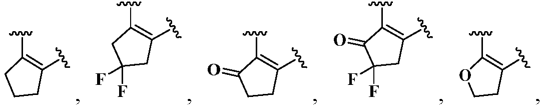 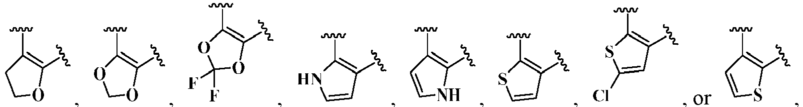
and further preferably to form a group as follows: 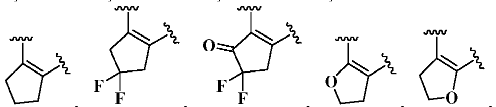 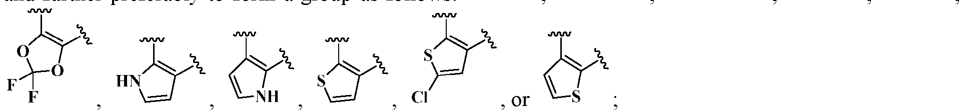
R_{c} is hydrogen, deuterium, or halogen, preferably hydrogen, deuterium, fluorine, chlorine, or bromine, and more preferably hydrogen or fluorine;
n1 is an integer from 2 to 6, preferably an integer from 3 to 5, and more preferably 4.

In a further preferred embodiment of the present invention, 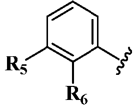 is a group as follows:

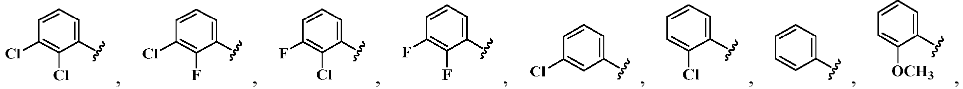

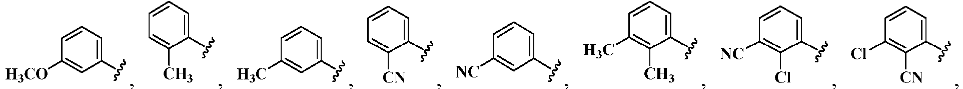

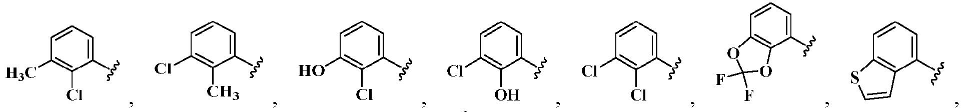

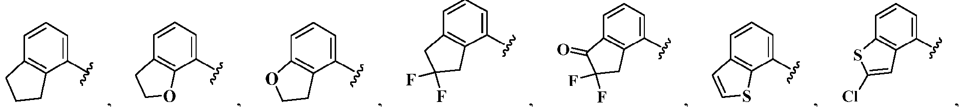

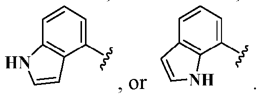

In a further preferred embodiment of the present invention, the compound is of any one of the following structures:

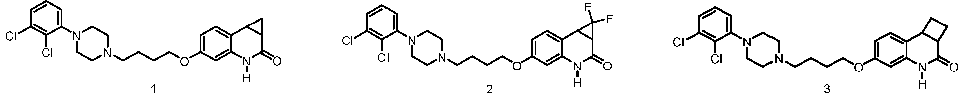

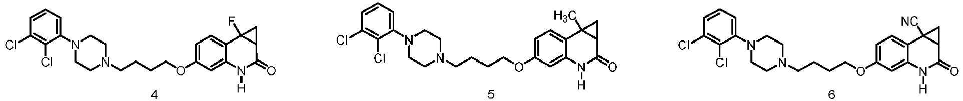

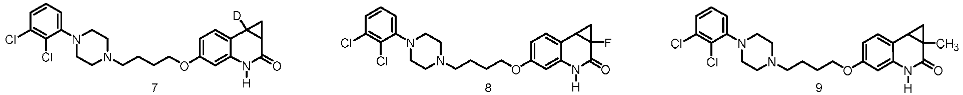

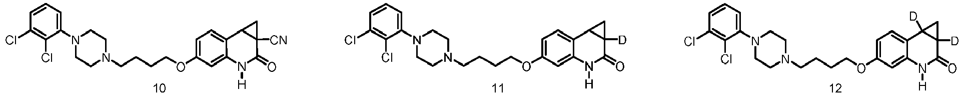

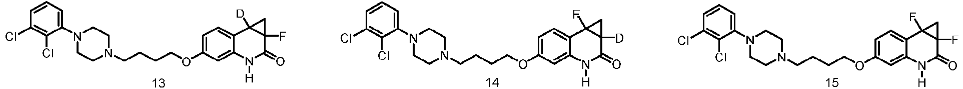

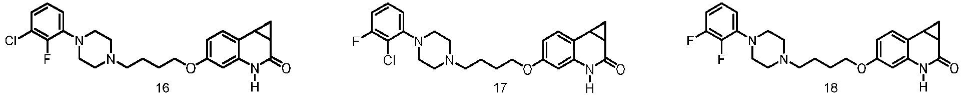

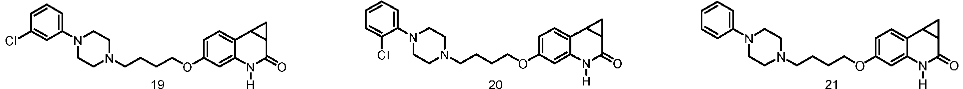

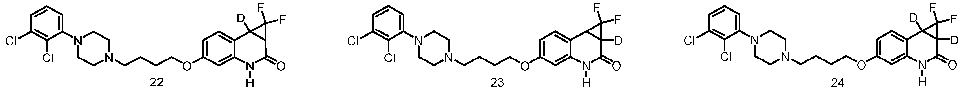

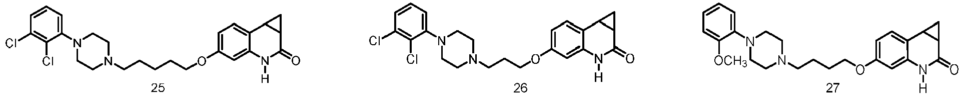

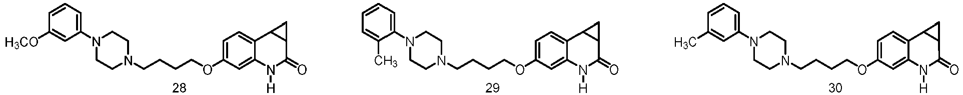

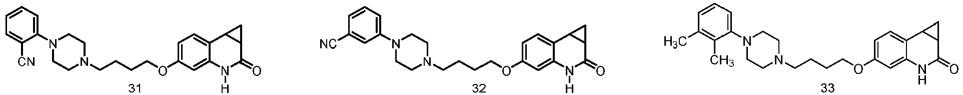

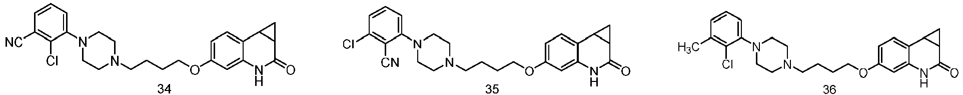

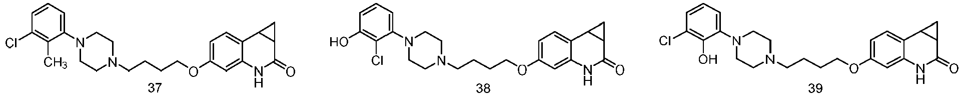

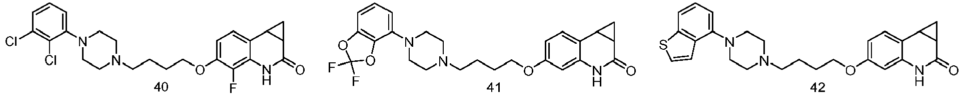

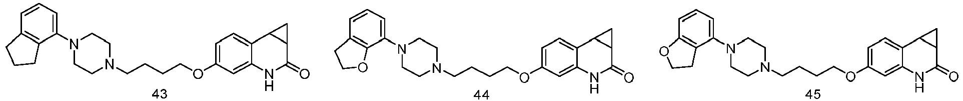

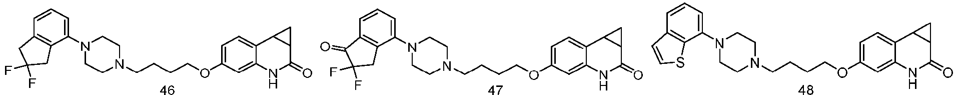

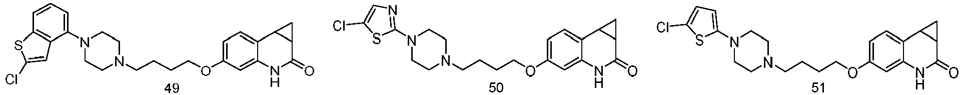

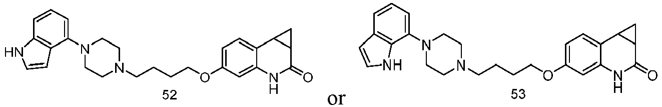

In one embodiment of the present invention, the compound may be of any one of the following structures:

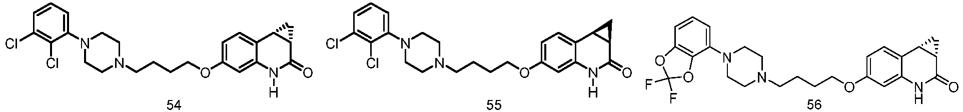

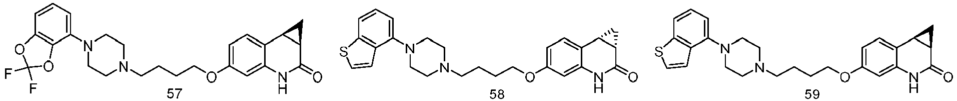

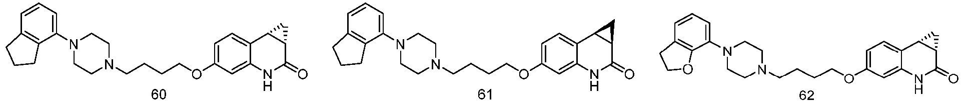

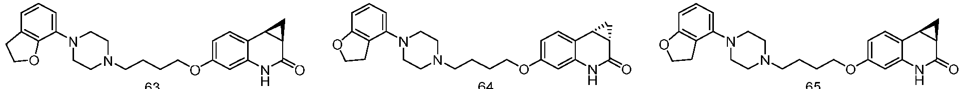

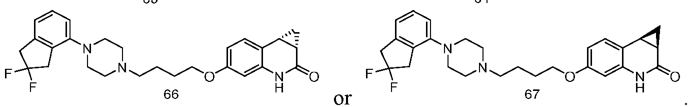

In one embodiment of the present invention, the compound of general formula (Ia) may be obtained from compound 1 under the following chiral resolution conditions: 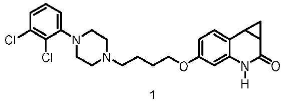
chromatographic column: DAICEL CHIRALCEL OJ column;
mobile phase: A: supercritical CO₂, B: methanol (containing 0.1% dimethylamine);
gradient: A (%V/V):B (%V/V) = 60:40; flow rate: 1.5 mL/min; detection wavelength: 214 nm;
the compound of general formula (Ia) is collected at a retention time of 2.38 min or 3.01 min.

In one embodiment of the present invention, the compound of general formula (Ia) may be obtained from compound 41 under the following chiral resolution conditions: 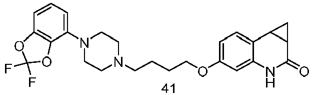
chromatographic column: DAICEL CHIRALPAK AS column;
mobile phase: A: supercritical CO₂, B: methanol (containing 0.1% dimethylamine);
gradient: A (%V/V):B (%V/V) = 60:40; flow rate: 1.5 mL/min; detection wavelength: 214 nm;
the compound of general formula (Ia) is collected at a retention time of 0.80 min or 1.86 min.

In one embodiment of the present invention, the compound of general formula (Ia) may be obtained from compound 42 under the following chiral resolution conditions: 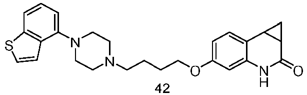
chromatographic column: DAICEL CHIRALPAK AS column;
mobile phase: A: supercritical CO₂, B: methanol (containing 0.1% dimethylamine);
gradient: A (%V/V):B (%V/V) = 60:40; flow rate: 1.5 mL/min; detection wavelength: 214 nm;
the compound of general formula (Ia) is collected at a retention time of 1.64 min or 4.62 min.

In one embodiment of the present invention, the compound of general formula (Ia) may be obtained from compound 43 under the following chiral resolution conditions: 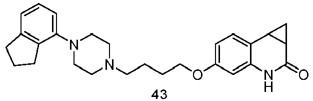
chromatographic column: DAICEL CHIRALPAK AS column;
mobile phase: A: supercritical CO₂, B: methanol (containing 0.1% dimethylamine);
gradient: A (%V/V):B (%V/V) = 60:40; flow rate: 1.5 mL/min; detection wavelength: 214 nm;
the compound of general formula (Ia) is collected at a retention time of 1.05 min or 2.72 min.

In one embodiment of the present invention, the compound of general formula (Ia) may be obtained from compound 44 under the following chiral resolution conditions: 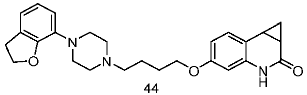
chromatographic column: DAICEL CHIRALPAK AS column;
mobile phase: A: supercritical CO₂, B: methanol (containing 0.1% dimethylamine);
gradient: A (%V/V):B (%V/V) = 60:40; flow rate: 1.5 mL/min; detection wavelength: 214 nm;
the compound of general formula (Ia) is collected at a retention time of 1.75 min or 5.22 min.

In one embodiment of the present invention, the compound of general formula (Ia) may be obtained from compound 45 under the following chiral resolution conditions: 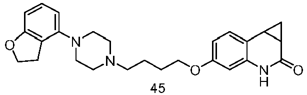
chromatographic column: DAICEL CHIRALPAK AS column;
mobile phase: A: supercritical CO₂, B: methanol (containing 0.1% dimethylamine);
gradient: A (%V/V):B (%V/V) = 60:40; flow rate: 1.5 mL/min; detection wavelength: 214 nm;
the compound of general formula (Ia) is collected at a retention time of 2.25 min or 7.94 min.

In one embodiment of the present invention, the compound of general formula (Ia) may be obtained from compound 46 under the following chiral resolution conditions: 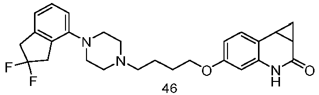
chromatographic column: DAICEL CHIRALPAK AS column;
mobile phase: A: supercritical CO₂, B: methanol (containing 0.1% dimethylamine);
gradient: A (%V/V):B (%V/V) = 60:40; flow rate: 1.5 mL/min; detection wavelength: 214 nm;
the compound of general formula (Ia) is collected at a retention time of 1.24 min or 4.02 min.

The present invention further provides a method for preparing a compound of general formula (Ia), a stereoisomer thereof, or a pharmaceutically acceptable salt thereof, comprising the following step: 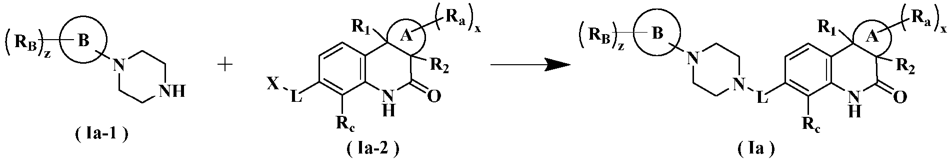
reacting a compound of general formula (Ia-1) with a compound of general formula (Ia-2) through a nucleophilic substitution reaction or reductive amination reaction to prepare the compound of general formula (Ia), the stereoisomer thereof, or the pharmaceutically acceptable salt thereof;
wherein:
   X is halogen, preferably fluorine, chlorine, bromine, or iodine, and more preferably bromine;
   ring A, ring B, L, R₁, R₂, Rₐ, R_{B}, R_{c}, x, and z are as described above.

The present invention further provides a method for preparing a compound of general formula (I), a stereoisomer thereof, or a pharmaceutically acceptable salt thereof, comprising the following step: 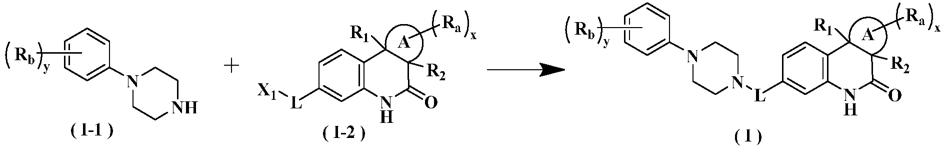
reacting a compound of general formula (I-1) with a compound of general formula (I-2) through a nucleophilic substitution reaction or reductive amination reaction to prepare the compound of general formula (I), the stereoisomer thereof, or the pharmaceutically acceptable salt thereof;
wherein:
   X₁ is halogen, preferably fluorine, chlorine, bromine, or iodine, and more preferably bromine;
   ring A, L, R₁, R₂, Rₐ, R_{b}, x, and y are as described above.

The present invention further relates to a pharmaceutical composition comprising a therapeutically effective dose of any one of the compounds, the stereoisomers thereof, or the pharmaceutically acceptable salts thereof, and one or more pharmaceutically acceptable carriers or excipients.

The present invention further relates to use of any one of the compounds, the stereoisomers thereof, or the pharmaceutically acceptable salts thereof, or the pharmaceutical composition thereof in the preparation of a drug. The drug is selected from one or more of 5-hydroxytryptamine, dopamine, and norepinephrine receptor regulators.

The present invention further relates to use of any one of the compounds, the stereoisomers thereof, or the pharmaceutically acceptable salts thereof, or the pharmaceutical composition thereof in the preparation of a drug for preventing and/or treating a central nervous system disease and/or a peripheral disease associated with the transmission of one or more neurotransmitters selected from 5-hydroxytryptamine, dopamine, and norepinephrine in a mammal.

The present invention further relates to a method for preventing and/or treating a central nervous system disease and/or a peripheral disease associated with the transmission of one or more neurotransmitters selected from 5-hydroxytryptamine, dopamine, and norepinephrine in a mammal, comprising administering a therapeutically effective dose of any one of the compounds, the stereoisomers thereof, or the pharmaceutically acceptable salts thereof, or the pharmaceutical composition thereof to the mammal.

In some embodiments, the central nervous system disease involved in the present invention is selected from one or more of schizophrenia, epilepsy, pain, depression, anxiety, bipolar disorder, sleep disorder, neurodegenerative disease, Huntington's chorea, cognitive disorder, memory impairment, Alzheimer's disease, Parkinson's disease, post-traumatic stress syndrome, addictive diseases, withdrawal syndrome, autism, Down's syndrome, attention deficit hyperactivity disorder, Reye's syndrome, attention deficit hyperactivity disorder, panic disorder, obsessive-compulsive disorder, and sleep disorder; the peripheral disease is pulmonary fibrosis and/or pulmonary hypertension.

### Detailed Description of the Invention

Unless otherwise defined, all technical and scientific terms used herein have the same meaning as that commonly understood by those of ordinary skill in the art to which the present invention belongs. In the event of a contradiction, the definition provided in the present application will control. When referring to a trade name, it is intended to refer to its corresponding commercial product or its active ingredient. All patents, published patent applications and publications cited herein are incorporated herein by reference.

In the present invention, the linking order of L is not particularly limited, and those skilled in the art may determine the linking order of L according to the understanding of the compounds in the present invention, for example, in the compound of general formula (Ia) or the compound of general formula (I), when L is - (CH₂)₄O-, the O end may be linked to the benzene ring or may be linked to the piperazine ring.

The term "alkyl" refers to a linear or branched saturated aliphatic hydrocarbon group consisting of carbon atoms and hydrogen atoms, which is linked to the rest of the molecule via a single bond. The "alkyl" may have 1-8 carbon atoms, i.e., "Ci-Cs alkyl", e.g., C₁₋₄ alkyl, C₁₋₃ alkyl, C₁₋₂ alkyl, C₃ alkyl, C₄ alkyl, C₁₋₆ alkyl, or C₃₋₆ alkyl. Non-limiting examples of the alkyl include, but are not limited to, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof. The alkyl may be optionally substituted or unsubstituted. When it is substituted, the substituent may be substituted at any available linking site, and the substituent is preferably one or more of the following groups independently selected from alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the alkyl is substituted with a substituent, the substituent is not further substituted.

The term "cycloalkyl" refers to a saturated or partially unsaturated monocyclic or polycyclic hydrocarbon substituent. The cycloalkyl contains 3 to 20 carbon atoms, preferably 3 to 12 carbon atoms, more preferably 3 to 8 carbon atoms, further preferably 3 to 6 carbon atoms, and most preferably 3 to 5 carbon atoms, or contains 5 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; polycyclic cycloalkyl includes spiro cycloalkyl, fused cycloalkyl, and bridged cycloalkyl.

The term "spiro cycloalkyl" refers to a 5- to 20-membered polycyclic group in which monocyclic rings share one carbon atom (referred to as the spiro atom). It may contain one or more double bonds, but none of the rings has a fully conjugated π-electron system. It is preferably 6- to 14-membered, and is more preferably 7-to 10-membered. According to the number of spiro atoms shared among the rings, the spiro cycloalkyl may be monospiro cycloalkyl, bispiro cycloalkyl, or polyspiro cycloalkyl, preferably monospiro cycloalkyl or bispiro cycloalkyl, and more preferably 3-membered/6-membered, 3-membered/5-membered, 4-membered/4-membered, 4-membered/5-membered, 4-membered/6-membered, 5-membered/5-membered, or 5-membered/6-membered monospiro cycloalkyl. Spiro heterocycloalkyl in which monospiro cycloalkyl shares a spiro atom with heterocycloalkyl is also included.

The term "fused cycloalkyl" refers to a 5- to 20-membered all-carbon polycyclic group in which each ring in the system shares a pair of adjacent carbon atoms with other rings in the system, wherein one or more rings may contain one or more double bonds, but none of the rings has a fully conjugated π-electron system. It is preferably 6- to 14-membered, and is more preferably 7- to 10-membered. According to the number of constituent rings, the fused cycloalkyl may be bicyclic, tricyclic, tetracyclic, or polycyclic fused cycloalkyl, preferably bicyclic or tricyclic fused cycloalkyl, and more preferably 5-membered/5-membered or 5-membered/6-membered bicycloalkyl.

The term "bridged cycloalkyl" refers to a 5- to 20-membered all-carbon polycyclic group in which any two rings share two carbon atoms that are not directly linked to each other, wherein these rings contain one or more double bonds, but none of the rings has a fully conjugated π-electron system. It is preferably 6- to 14-membered, and is more preferably 7- to 10-membered. According to the number of constituent rings, the bridged cycloalkyl may be bicyclic, tricyclic, tetracyclic, or polycyclic bridged cycloalkyl, preferably bicyclic, tricyclic, or tetracyclic bridged cycloalkyl, and more preferably bicyclic or tricyclic bridged cycloalkyl.

The cycloalkyl as described above may be fused to an aryl, heteroaryl, or heterocycloalkyl ring. The cycloalkyl may be optionally substituted or unsubstituted. When it is substituted, the substituent may be substituted at any available linking site, and the substituent is preferably one or more of the following groups independently selected from alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the cycloalkyl is substituted with a substituent, the substituent is not further substituted.

The term "heterocyclyl" refers to a saturated or unsaturated monocyclic or polycyclic hydrocarbon substituent containing 3 to 20 ring atoms, wherein one or more of the ring atoms are heteroatoms selected from nitrogen, oxygen, and S(O)ₘ (wherein m is an integer from 0 to 2), excluding a cyclic portion of -O-O-, -O-S-, or -S-S-, and the remaining ring atoms are carbon atoms. Preferably, the heterocyclyl contains 3 to 12 ring atoms, of which 1 to 4 are heteroatoms; more preferably, the heterocyclyl contains 3 to 6 ring atoms, of which 1 to 4 are heteroatoms selected from N, O, and S, or 4 to 8 ring atoms, of which 1 to 4 are heteroatoms selected from N, O, and S; further preferably, the heterocyclyl is 3- to 6-membered heterocyclyl containing 1 to 4 or 1 to 2 atoms selected from N, O, and S; most preferably, the heterocyclyl is 5- to 6-membered heterocyclyl containing 1 to 4 or 1 to 2 atoms selected from N, O, or S. "Hetero" in the heterocyclyl represents 1 to 4 heteroatoms selected from N, O, and S(O)ₘ, wherein m is an integer from 0 to 2.

Non-limiting examples of monocyclic heterocyclyl include oxetanyl, thietanyl, pyrrolidinyl, imidazolidinyl, tetrahydrofuranyl, tetrahydrothienyl, tetrahydropyranyl, dihydroimidazolyl, dihydrofuranyl, dihydropyrazolyl, piperidinyl, piperazinyl, morpholinyl, 1,3-dioxolanyl, 2,2-difluoro-1,3-dioxolanyl, cyclopentanonyl, 2,2-difluorocyclopentanonyl, azepinyl, 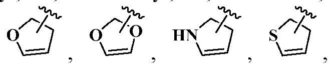 or the like.

Non-limiting examples of polycyclic heterocyclyl include spiro heterocyclyl, fused heterocyclyl, and bridged heterocyclyl, wherein the spiro heterocyclyl, fused heterocyclyl, and bridged heterocyclyl involved are optionally linked to other groups via single bonds, or are further ortho-fused to other cycloalkyl, heterocyclyl, aryl, and heteroaryl via any two or more atoms of the ring.

The term "spiro heterocyclyl" refers to a 5- to 20-membered polycyclic heterocyclyl group in which monocyclic rings share one atom (referred to as the spiro atom), wherein one or more of the ring atoms are heteroatoms selected from nitrogen, oxygen, and S(O)ₘ (wherein m is an integer from 0 to 2), and the remaining ring atoms are carbon atoms. It may contain one or more double bonds, but none of the rings has a fully conjugated π-electron system. It is preferably 6- to 14-membered, and is more preferably 7- to 10-membered. According to the number of spiro atoms shared among the rings, the spiro heterocyclyl may be monospiro heterocyclyl, bispiro heterocyclyl, or polyspiro heterocyclyl, preferably monospiro heterocyclyl or bispiro heterocyclyl, and more preferably 3-membered/6-membered, 3-membered/5-membered, 4-membered/4-membered, 4-membered/5-membered, 4-membered/6-membered, 5-membered/5-membered, or 5-membered/6-membered monospiro heterocyclyl. "Hetero" in the spiro heterocyclyl represents 1 to 4 heteroatoms selected from N, O, and S(O)ₘ, wherein m is an integer from 0 to 2.

The term "fused heterocyclyl" refers to a 5- to 20-membered polycyclic heterocyclyl group in which each ring in the system shares a pair of adjacent atoms with the other rings in the system, wherein one or more of the ring atoms are heteroatoms selected from nitrogen, oxygen, or S(O)ₘ (wherein m is an integer from 0 to 2), and the remaining ring atoms are carbon atoms, wherein one or more of the rings may contain one or more double bonds, but none of the rings has a fully conjugated *π*-electron system. It is preferably 6- to 14-membered, and is more preferably 7- to 10-membered. According to the number of constituent rings, the fused heterocyclyl may be bicyclic, tricyclic, tetracyclic, or polycyclic fused heterocyclyl, preferably bicyclic or tricyclic fused heterocyclyl, and more preferably 5-membered/5-membered or 5-membered/6-membered bicyclic fused heterocyclyl. "Hetero" in the fused heterocyclyl represents 1 to 4 heteroatoms selected from N, O, and S(O)ₘ, wherein m is an integer from 0 to 2.

The term "bridged heterocyclyl" refers to a 5- to 20-membered polycyclic heterocyclyl group in which any two rings share two atoms that are not directly linked to each other, wherein one or more of the ring atoms are heteroatoms selected from nitrogen, oxygen, or S(O)ₘ (wherein m is an integer from 0 to 2), and the remaining ring atoms are carbon atoms, wherein these rings contain one or more double bonds, but none of the rings has a fully conjugated π-electron system. It is preferably 6- to 14-membered, and is more preferably 7- to 10-membered. According to the number of constituent rings, the bridged heterocyclyl may be bicyclic, tricyclic, tetracyclic, or polycyclic bridged heterocyclyl, preferably bicyclic, tricyclic, or tetracyclic bridged heterocyclyl, and more preferably bicyclic or tricyclic bridged heterocyclyl. "Hetero" in the bridged heterocyclyl represents 1 to 4 heteroatoms selected from N, O, and S(O)ₘ, wherein m is an integer from 0 to 2.

The heterocyclyl as described above may be fused to an aryl, heteroaryl, or cycloalkyl ring. The heterocyclyl may be optionally substituted or unsubstituted. When it is substituted, the substituent may be substituted at any available linking site, and the substituent is preferably one or more of the following groups independently selected from alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the heterocyclyl is substituted with a substituent, the substituent is not further substituted.

The term "aryl" refers to a 6- to 14-membered, preferably 6- to 12-membered all-carbon monocyclic or fused polycyclic group having a conjugated π-electron system, such as phenyl and naphthyl, more preferably, phenyl. The aryl may be fused to a heteroaryl, heterocyclyl or cycloalkyl ring, for example, C₆₋₁₂ arylo 5- to 10-membered heteroaryl, C₆₋₁₂ arylo C₃₋₈ cycloalkyl, C₆₋₁₂ arylo 4- to 8-membered heterocyclyl, preferably benzo 5- to 10-membered heteroaryl, benzo C₃₋₈ cycloalkyl, or benzo 4- to 8-membered heterocyclyl, more preferably benzo C₃₋₈ cycloalkyl, benzo 4- to 8-membered heterocyclyl, or benzo 5- to 6-membered heteroaryl, further preferably benzo 5- to 6-membered heteroaryl, benzo C₄₋₆ cycloalkyl, or benzo 4- to 6-membered heterocyclyl, and particularly preferably benzo 5- to 6-membered heteroaryl, benzo C₅₋₆ cycloalkyl, and benzo 5- to 6-membered heterocyclyl. Its non-limiting examples include: 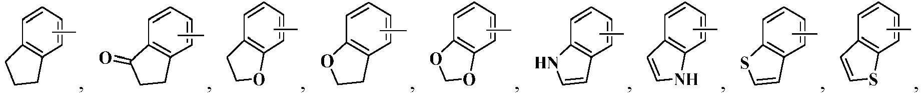 and the like.

The aryl may be optionally substituted or unsubstituted. When it is substituted, the substituent may be substituted at any available linking site, and the substituent is preferably one or more of the following groups independently selected from alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the aryl is substituted with a substituent, the substituent is not further substituted.

The term "heteroaryl" refers to a heteroaromatic system containing 1 to 4 heteroatoms and 5 to 14 ring atoms, wherein the heteroatoms are selected from oxygen, sulfur, nitrogen, and the like. The heteroaryl is preferably 5- to 12-membered, and more preferably 5- to 6-membered, such as pyrrolyl, imidazolyl, furanyl, pyranyl, thienyl, thiazolyl, thiadiazolyl, pyrazolyl, oxazolyl, oxadiazolyl, triazolyl, tetrazolyl, pyridinyl, pyrimidinyl, pyridazinyl, and pyrazinyl. The heteroaryl includes those in which the heteroaryl ring as described above is fused to an aryl, cycloalkyl, or heterocyclyl ring, wherein the ring linked to the parent structure is the heteroaryl ring. The heteroaryl may be optionally substituted or unsubstituted. When it is substituted, the substituent may be substituted at any available linking site, and the substituent is preferably one or more of the following groups independently selected from alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the heteroaryl is substituted with a substituent, the substituent is not further substituted. "Hetero" in the heteroaryl represents 1 to 4 heteroatoms selected from N, O, and S.

The term "alkoxy" refers to -O-(alkyl) or -O-(unsubstituted cycloalkyl), wherein the alkyl and cycloalkyl are as defined above. Non-limiting examples of alkoxy include: methoxy, ethoxy, propoxy, butoxy, cyclopropyloxy, cyclobutoxy, cyclopentyloxy, cyclohexyloxy, and the like. The alkoxy may be optionally substituted or unsubstituted. When it is substituted, the substituent may be substituted at any available linking site, and the substituent is preferably one or more of the following groups independently selected from alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the alkoxy is substituted with a substituent, the substituent is not further substituted.

The term "alkylthio" refers to -S-(alkyl) or -S-(unsubstituted cycloalkyl), wherein the alkyl and cycloalkyl are as defined above. Non-limiting examples of alkylthio include: methylthio, ethylthio, propylthio, butylthio, cyclopropylthio, cyclobutylthio, cyclopentylthio, cyclohexylthio, and the like. The alkylthio may be optionally substituted or unsubstituted. When it is substituted, the substituent may be substituted at any available linking site, and the substituent is preferably one or more of the following groups independently selected from alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the alkylthio is substituted with a substituent, the substituent is not further substituted.

The term "halo", "halogen", or "halogenated" should be understood to refer to fluorine (F), chlorine (Cl), bromine (Br), or iodine (I) atom, preferably fluorine, chlorine, or bromine atom.

The term "deuterated alkyl" refers to alkyl substituted with one or more deuterium, wherein the alkyl is as defined above.

The term "haloalkyl" refers to alkyl substituted with one or more halogens, wherein the alkyl is as defined above.

The term "alkenyl" refers to an alkenyl group, also referred to as an alkene group, wherein the alkene may be further substituted with other related groups.

The term "alkynyl" refers to (CH≡C-), wherein the alkynyl may be further substituted by other related groups.
"Hydroxy" refers to -OH.
"Amino" refers to -NH₂.
"Cyano" refers to -CN.
"Nitro" refers to -NO₂.
"Sulfydryl" refers to -SH.
"Carboxyl" refers to -C(O)OH.
"Oxo" refers to =O.
"DMB" refers to 3,4-dimethoxybenzyl.
"PMB" refers to *p*-methoxybenzyl.
"Boc" refers to *tert*-butoxycarbonyl.
"DMF" refers to *N,N*-dimethylformamide.
"THF" refers to tetrahydrofuran.
"TFA" refers to trifluoroacetic acid.
"DCM" refers to dichloromethane.
"DIPEA" refers to *N,N*-diisopropylethylamine.
"Pd₂dba₃" refers to tris(dibenzylideneacetone)dipalladium(0).
"Ruphos" refers to 2-dicyclohexylphosphonium-2',6'-diisopropoxy-1,1'-biphenyl.
"LiHMDS" refers to lithium bis(trimethylsilyl)amide.
"*m*-CPBA" refers to *m*-chloroperoxybenzoic acid.
"DAST" refers to diethylaminosulfur trifluoride.
"NCS" refers to *N*-chlorosuccinimide.
""BuLi" refers to *n*-butyllithium. "*^{t}*BuONa" refers to sodium *tert*-butoxide.
"TfOH" refers to trifluoromethanesulfonic acid.
"P^{t}Bu₃·BF₄" refers to tri-*tert*-butylphosphine tetrafluoroborate.
"Et₃SiH" refers to triethylsilane.
"*p*-TsOH" refers to *p*-toluenesulfonic acid.
"TsCl" refers to *p*-toluenesulfonyl chloride.
"Mg (dust)" refers to magnesium chips.
"Selectfluor" refers to 1-chloromethyl-4-fluoro-1,4-diazabicyclo[2.2.2]octane bis(tetrafluoroborate).

The terms "comprise", "include", "have", "contain", or "relate" and other variations thereof herein are inclusive or open-ended and do not exclude additional unrecited elements or method steps. It should be understood by those skilled in the art that terms described above such as "comprise" encompass the meaning of "consist of".

The term "one or more" or similar expressions "at least one" may indicate, for example, 1, 2, 3, 4, 5, 6, 7, 8, 9, 10 or more.

When the lower and upper limits of a range of values are disclosed, any value falling within the range and any included range are specifically disclosed. In particular, each range of values disclosed herein is to be understood as indicating each value and range encompassed within the broader range.

As used herein, "Z" and "-Z-" both refer to the same particular group, which may be used interchangeably. The expression "m-n" as used herein refers to the range of m to n as well as to sub-ranges consisting of point values therein and the point values. For example, the expression "C₂-C₈" or "C₂₋₈" encompasses a range of 2 to 8 carbon atoms and should be understood as also encompassing any sub-range and each point value therein, e.g., C₂-C₅, C₃-C₄, C₂-C₆, C₃-C₆, C₄-C₆, C₄-C₇, C₄-C₈, and the like, as well as C₂, C₃, C₄, C₅, C₆, C₇, C₈, and the like. For example, the expression "C₃-C₁₀" or "C₃₋₁₀" should also be understood in a similar manner, for example, it can encompass any sub-range and point value therein, e.g., C₃-C₉, C₆-C₉, C₆-C₈, C₆-C₇, C₇-C₁₀, C₇-C₉, C₇-C₈, C₈-C₉, etc., and C₃, C₄, C₅, C₆, C₇, C₈, C₉, C₁₀, etc. For another example, the expression "C₁-C₆" or "C₁₋₆" encompasses a range of 1 to 6 carbon atoms and should be understood as also encompassing any sub-range and each point value therein, e.g., C₂-C₅, C₃-C₄, C₁-C₂, C₁-C₃, C₁-C₄, C₁-C₅, C₁-C₆, and the like, as well as C₁, C₂, C₃, C₄, C₅, C₆, and the like. For another example, the expression "three to ten membered" should be understood as encompassing any sub-range and each point value therein, e.g., three to five membered, three to six membered, three to seven membered, three to eight membered, four to five membered, four to six membered, four to seven membered, four to eight membered, five to seven membered, five to eight membered, six to seven membered, six to eight membered, nine to ten membered, etc., and three membered, four membered, five membered, six membered, seven membered, eight membered, nine membered, ten membered, etc. Other similar expressions herein should also be understood in a similar manner.

The expressions "X is selected from A, B, or C", "X is selected from A, B, and C", "X is A, B, or C", "X is A, B, and C", and the like as used herein all carry the same meaning, i.e., X may be any one or more of A, B, and C.

The term "optional" or "optionally" refers to that the subsequently described event or circumstance may occur or may not occur. The description includes instances where the event or circumstance occurs and instances where the event or circumstance does not occur. For example, "cycloalkyl optionally substituted with alkyl" means that alkyl may, but does not necessarily exist and that the description includes instances where the cycloalkyl is or is not substituted with alkyl.

The terms "substitution" and "substituted" mean that one or more (e.g., 1, 2, 3, or 4) hydrogens on a specified atom is replaced with a selection from the designated group, with the proviso that the normal valency of the atom specified is not exceeded and that the replacement results in a stable compound. A combination of substituents and/or variables is permissible only if the combination results in a stable compound. When it is stated that a certain substituent is absent, it should be understood that the substituent may be one or more hydrogen atoms, provided that the structure enables the compound to reach a stable state. When it is stated that each carbon atom in a group may optionally be replaced by a heteroatom, the proviso is that the normal valency of all atoms in the group in the present case is not exceeded, and that a stable compound is formed. If a substituent is described as "optionally substituted", the substituent may be unsubstituted or may be substituted. If an atom or group is described as optionally substituted with one or more substituents in the list of substituents, one or more hydrogens on the atom or group can be replaced with an independently selected and optional substituent. When the substituent is oxo (namely =O), it means that two hydrogen atoms are replaced. Unless otherwise indicated, as used herein, the connecting point of a substituent may be from any suitable position of the substituent.

When a bond of a substituent is shown to pass through a bond connecting two atoms in the ring, the substituent may be bonded to any one of the ring-forming atoms in the substitutable ring.

When any variable (e.g., R), as well as labeled variables (e.g., R₁, R₂, R₃, R₄, R₅, P₆, R₇, etc.) occurs more than once in a composition or structure of a compound, the variable is independently defined in each case at each occurrence. For example, if a group is substituted with 0, 1, 2, 3 or 4 R substituents, the group can be optionally substituted with up to four R substituents, and each R substituent is independently defined in each case.

The term "substituted" means that one or more hydrogen atoms on a compound or group are replaced by other atoms or groups, with the proviso that stable valence states or compounds are formed. The expression "unsubstituted" may also be understood as "not substituted". It should be understood that when a substituent is hydrogen, this may also mean that the corresponding group is "unsubstituted" or "not substituted".

The compounds of the present invention may exist in a form of a specific geometric isomer or stereoisomer. All such compounds are contemplated herein, including *cis* and *trans* isomers, (-)- and (+)-enantiomers, (R)- and (S)-enantiomers, diastereoisomers, (D)-isomers, (L)-isomers, and racemic mixtures and other mixtures thereof, such as an enantiomer or diastereomer enriched mixture, all of which are encompassed within the scope of the present invention. Additional asymmetric carbon atoms may be present in substituents such as an alkyl group. All these isomers and mixtures thereof are encompassed within the scope of the present invention. In certain embodiments, preferred compounds are those isomer compounds that exhibit superior biological activity. Purified or partially purified isomers and stereoisomers, or racemic or diastereomeric mixtures of the compound of the present invention are also encompassed within the scope of the present invention. Purification and isolation of such materials can be accomplished by standard techniques known in the art.

All hydrogen atoms described in the present invention may be substituted with isotope deuterium, and any hydrogen atom in the compounds of the examples to which the present invention relates may also be substituted with a deuterium atom.

The term "pharmaceutically acceptable" substance refers to those substances which are, within the scope of sound medical judgment, suitable for use in contact with the tissues of patients without undue toxicity, irritation, allergic response and other problems, commensurate with a reasonable benefit to risk ratio, and effective for their intended use.

The term "pharmaceutically acceptable salt" refers to salts of the compounds of the present invention, which are safe and effective for use in the body of a mammal and possess the requisite biological activities.

The term "pharmaceutical composition" refers to a mixture containing one or more of the compounds described herein or a physiologically/pharmaceutically acceptable salt or pro-drug thereof, and other chemical components, for example physiologically/pharmaceutically acceptable carriers or excipients. The pharmaceutical composition is intended to promote the administration to an organism, so as to facilitate the absorption of the active ingredient, thereby exerting biological activity.

The term "pharmaceutically acceptable carrier" refers to those substances which do not have a significant irritating effect on organisms and do not impair the biological activity and properties of the active compound. The "pharmaceutically acceptable carrier" includes, but is not limited to a glidant, a sweetener, a diluent, a preservative, a dye/colorant, a flavoring agent, a surfactant, a wetting agent, a dispersant, a disintegrant, a stabilizer, a solvent or an emulsifier.

The terms "administration" or "administering" and the like refer to those methods which enable a compound or composition to be delivered to a desired site of biological action. Those methods include, but are not limited to, oral administration or parenteral (including intraventricular, intravenous, subcutaneous, intraperitoneal, intramuscular, intravascular injection or infusion), topical, rectal administrations, and the like. Those methods especially include injection or oral administration.

As used herein, the term "treat", "treating" or "treatment" includes alleviating, reducing, or ameliorating a disease or symptom; preventing other symptoms; ameliorating or preventing metabolic factors underlying a symptom; inhibiting a disease or symptom, e.g., arresting the development of a disease or symptom; alleviating a disease or symptom; promoting the alleviation of a disease or symptom; or halting the signs of a disease or symptom, and extends to include prevention. The "treat", "treating" or "treatment" also includes achieving a therapeutic benefit and/or a prophylactic benefit. The therapeutic benefit refers to eradication or amelioration of a condition being treated. In addition, the therapeutic benefit is achieved by eradicating or ameliorating one or more physiological signs associated with an underlying disease, and amelioration of the underlying disease in the subject is observed, although the subject may still be afflicted with the underlying disease. The prophylactic benefit refers to the use of a composition by a patient to prevent the risk of a disease or the administration of a composition by a patient when the patient develops one or more physiological conditions of a disease, although the disease has not yet been diagnosed.

The term "active ingredient", "therapeutic agent", "active substance" or "active agent" refers to a chemical entity that is effective in treating a target disorder, disease, or condition. The term "nervous and mental disease" refers to a general term for neurological diseases and psychiatric diseases, including neurological diseases and/or psychiatric diseases.

For a drug, drug unit or active ingredient, the term "effective amount", "therapeutically effective amount" or "prophylactically effective amount" refers to an amount of a drug or agent that is sufficient to provide the desired effect with acceptable side effects. The determination of the effective amount varies from person to person. It depends on the age and general condition of a subject, as well as the particular active substance used. The appropriate effective amount in a case may be determined by those skilled in the art in the light of routine tests.

As used herein, an "individual" includes a human or non-human animal. An exemplary human individual includes a human individual (referred to as patients) with a disease (e.g., a disease described herein) or a normal individual. As used herein, a "non-human animal" includes all vertebrates, such as non-mammals (e.g., birds, amphibians, and reptiles) and mammals, such as non-human primates, livestock and/or domesticated animals (e.g., sheep, dogs, cats, cows, pigs, etc.).

The following detailed description is intended to illustrate non-limiting embodiments and to enable others skilled in the art to more fully understand the technical scheme of the present invention, its principles, and its practical application, so that others skilled in the art may modify and implement the present invention in various forms to allow it to be optimally adapted to the particular use contemplated.

### Beneficial Effects

*In vitro* receptor functional assays show that the compounds of the present invention have an agonistic effect on dopamine D₂ (D_{2L} and D_{2S}) receptors, have an agonistic effect on 5-HT_{1A} receptor, have an antagonistic effect on 5-HT_{2A} and 5-HT_{2B} receptors, and have the effects of treating or preventing nervous and mental diseases, particularly an anti-schizophrenic effect.

The compounds of the present invention have an agonistic effect on dopamine D₂ (D_{2L} and D_{2S}) receptors, have good efficacy on positive symptoms of schizophrenia, and can reduce side effects such as EPS, hyperprolactinemia, and tardive dyskinesia; the compounds have an agonistic effect on 5-HT_{1A} receptor, and can improve negative symptoms and cognitive disorder; the compounds have an antagonistic effect on 5-HT_{2A} receptor, and can directly or indirectly regulate the function of dopamine to exert the anti-schizophrenia effect, i.e., to down-regulate hyperactive dopamine activity and improve positive symptoms. Meanwhile, the compounds of the present invention have an antagonistic effect on 5-HT_{2B} receptor, and can reduce cardiovascular side effects such as pulmonary hypertension, fibrosis, and valvular disease caused by the activation of the 5-HT_{2B} receptor.

In addition, the compounds of the present invention also have good pharmacokinetic properties.

### DETAILED DESCRIPTION

The present invention will be further illustrated with reference to the following specific examples. It should be understood that these examples are merely intended to illustrate the present invention rather than limit the scope of the present invention. Furthermore, it should be understood that various changes or modifications of the present invention can be made by those skilled in the art after reading the teachings of the present invention, and these equivalents also fall within the scope of the appended claims of the present application.

### EXAMPLES

Embodiments of the present invention will be described in detail below with reference to examples, but those skilled in the art will appreciate that the following examples are only illustrative of the present invention and should not be construed as limiting the scope of the present invention. Experimental procedures without specified conditions in the examples are conducted according to conventional conditions or conditions recommended by the manufacturer. Reagents or instruments without specified manufacturers used herein are conventional products that are commercially available. The proportions or percentages used herein are calculated by weight, unless otherwise specified.

The compound structure of the present invention is determined by nuclear magnetic resonance (NMR) and/or liquid chromatography-mass spectrometry (LC-MS).

The NMR chemical shift (δ) is given in parts per million (ppm). The NMR determination is conducted by using an AVANCE III600 nuclear magnetic resonance apparatus, with deuterated dimethyl sulfoxide (*d₆*-DMSO), deuterated methanol (CD₃OD), and deuterated chloroform (CDCl₃) as determination solvents, and tetramethylsilane (TMS) as an internal standard.

The LC-MS determination was conducted by using a Japan Shimadzu LCMS2020 mass spectrometer. HPLC analysis was performed by using a Japan Shimadzu LC20A liquid chromatograph.

The Yantai Jiangyou silica gel plate was adopted as a thin layer chromatography silica gel plate. The specification adopted by the TLC was 0.2 mm ± 0.03 mm, and the specification adopted by the thin layer chromatography for product separation and purification was 0.4 mm-0.5 mm.

### Synthesis of intermediates

### 5-(4-Bromobutoxy)-1,1a,3,7b-tetrahydro-2H-cyclopropa[c]quinolin-2-one

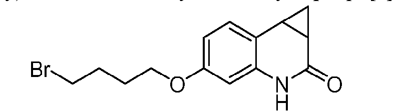

### Snthetic route:

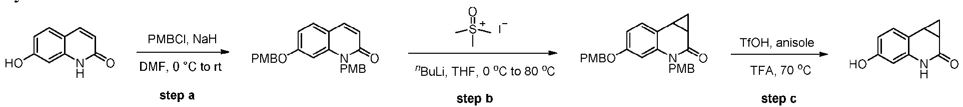

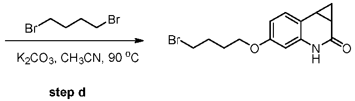

### Step a: Synthesis of 1-(4-methoxybenzyl)-7-((4-methoxybenzyl)oxy)quinolin-2(1H)-one

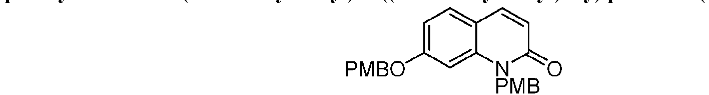

Sodium hydride (24.8 g, 620.5 mmol) was added portionwise to a solution of 7-hydroxyquinolin-2(1H)-one (40.0 g, 248.2 mmol) in *N,N*-dimethylformamide (800 mL) at 0 °C. After the mixed solution was reacted at this temperature for 30 min, 4-methoxybenzyl chloride (97.2 g, 620.5 mmol) was slowly added dropwise, and then the reaction solution was warmed to room temperature overnight. After the reaction was completed, the reaction solution was poured into ice water, and a large amount of solid was precipitated. The mixture was filtered, and the filtrate was extracted with ethyl acetate. The organic phase was washed with water and saturated brine, dried over anhydrous sodium sulfate, filtered, concentrated, and combined with the filter cake. The crude product was slurried (with petroleum ether: ethyl acetate = 3:1) and purified to give the target product (85.0 g, 85% yield).

### Step b: Synthesis of 3-(4-methoxybenzyl)-5-((4-methoxybenzyl)oxy)-1,1a,3,7b-tetrahydro-2H-cyclopropa[c]quinolin-2-one

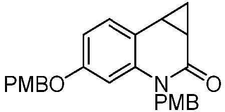

*n*-Butyllithium (237.1 mL, 2.5 M in *n*-hexane, 592.8 mmol) was slowly added dropwise to a solution of trimethylsulfoxonium iodide (139.8 g, 635.2 mmol) in tetrahydrofuran (1200 mL) at 0 °C, and the mixed solution was reacted at this temperature for 30 min. A solution of 1-(4-methoxybenzyl)-7-((4-methoxybenzyl)oxy)quinolin-2(1H)-one (85.0 g, 211.7 mmol) in tetrahydrofuran (200 mL) was then added, and the mixed solution was reacted at 80 °C for 4 h. After the reaction was completed, the reaction solution was poured into ice water, and a large amount of solid was precipitated. The mixture was filtered, and the filtrate was extracted with ethyl acetate. The organic phase was washed with saturated brine, dried over anhydrous sodium sulfate, filtered, concentrated, and combined with the filter cake. The crude product was slurried with ethyl acetate and purified to give the target product (60 g, 68% yield).

### Step c: Synthesis of 5-hydroxy-1,1a,3,7b-tetrahydro-2H-cyclopropa[c]quinolin-2-one

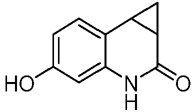

3-(4-Methoxybenzyl)-5-((4-methoxybenzyl)oxy)-1,1a,3,7b-tetrahydro-2H-cyclopropa[c]quinolin-2-one (6.0 g, 14.4 mmol), trifluoroacetic acid (60 mL), anisole (6 mL), and trifluoromethanesulfonic acid (6 mL) each were added to a reaction flask, and then the mixed solution was reacted at 70 °C for 2 h. After the reaction solution was concentrated, the crude product was directly purified by column chromatography (petroleum ether:ethyl acetate = 1:2) to give the target product (2.5 g, 100% yield).

### Step d: Synthesis of 5-(4-bromobutoxy)-1,1a,3,7b-tetrahydro-2H-cyclopropa[c]quinolin-2-one

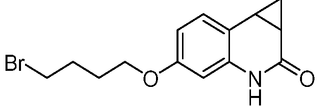

5-Hydroxy-1,1a,3,7b-tetrahydro-2H-cyclopropa[c]quinolin-2-one (2.5 g, 14.4 mmol), 1,4-dibromobutane (9.4 g, 43.3 mmol), potassium carbonate (6.0 g, 43.3 mmol), and acetonitrile (60 mL) each were added to a reaction flask, and the mixed solution was reacted at 90 °C for 3 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 2:1) to give the target product (2.8 g, 63% yield).

¹H NMR (400 MHz, CDCl3): δ 9.14 (s, 1H), 7.21 (d, *J* = 8.4 Hz, 1H), 6.53 (dd, *J* = 8.4, 2.4 Hz, 1H), 6.38 (d, *J* = 2.4 Hz, 1H), 3.95 (t, *J* = 6.0 Hz, 2H), 3.47 (t, *J* = 6.8 Hz, 2H), 2.49-2.44 (m, 1H), 2.15-2.09 (m, 1H), 2.07-2.01 (m, 2H), 1.94-1.88 (m, 2H), 1.61-1.55 (m, 1H), 0.65 (q, *J* = 4.8 Hz, 1H).

MS Found: 309.9[M+H]⁺.

### General synthesis scheme for some compounds of the examples of the present invention:

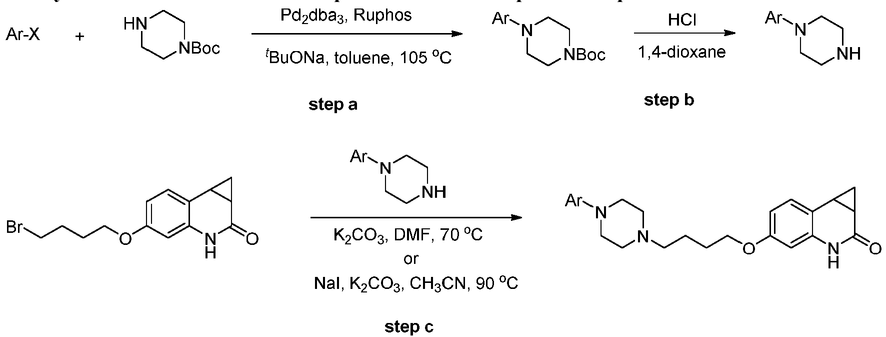

### Step a: General synthesis method for tert-butyl 4-arylpiperazine-1-carboxylate

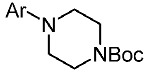

Tris(dibenzylideneacetone)dipalladium(0) (5 mol%), 2-dicyclohexylphosphonium-2',6'-diisopropoxy-1,1'-biphenyl (20 mol%), sodium *tert*-butoxide (1.5 eq), *tert*-butyl piperazine-1-carboxylate (1.5 eq), and the corresponding solid aryl halide (1.0 eq) each were added to a dry reaction flask, and then the reaction flask was purged three times with nitrogen in a vacuum pump. Extra-dry toluene (0.1 M) was added under nitrogen atmosphere (if the aryl halide was liquid, the aryl halide was added to the reaction system after toluene was added), and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography to give the corresponding target product.

### Step b: General synthesis method for 1-arylpiperazine hydrochloride

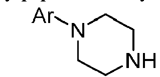

*tert*-Butyl 4-arylpiperazine-1-carboxylate (1.5 eq) and a solution of hydrochloric acid in 1,4-dioxane (0.1 M) each were added to a reaction flask, and the mixed solution was reacted at room temperature until the starting materials disappeared. After the reaction was completed, the reaction solution was concentrated to give the corresponding crude 1-arylpiperazine hydrochloride, which was directly used in the next step without purification.

### Step c: General synthesis method for 5-(4-(4-arylpiperazin-1-yl)butoxy)-1,1a,3,7b-tetrahydro-2H-cyclopropa[c]quinolin-2-one

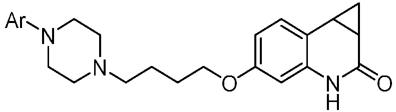

5-(4-Bromobutoxy)-1,1a,3,7b-tetrahydro-2H-cyclopropa[c]quinolin-2-one (1.5 eq), 1-arylpiperazine hydrochloride (1.0 eq), potassium carbonate (3.0 eq), and *N,N*-dimethylformamide (0.1 M) each were added to a reaction flask, and the mixed solution was reacted at 70 °C until the starting materials disappeared. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography to give the corresponding target product.

### Example 1

### 5-(4-(4-(2,3-Dichlorophenyl)piperazin-1-yl)butoxy)-3,7b-dihydro-1H-cyclopropa[c]quinolin-2(1aH)-one

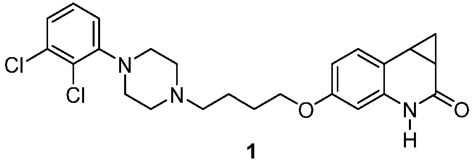

### Synthesis scheme:

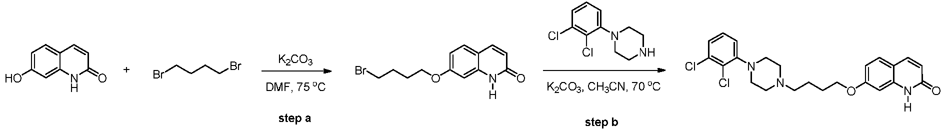

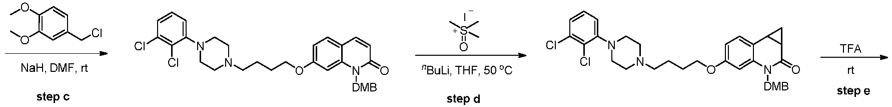

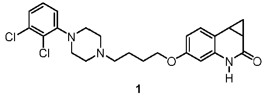

### Step a: Synthesis of 7-(4-bromobutoxy)quinolin-2(1H)-one

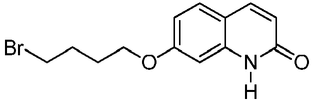

7-Hydroxyquinolin-2(1H)-one (8 g, 0.049 mol), 1,4-dibromobutane (11.7 g, 0.054 mol), potassium carbonate (20.6 g, 0.162 mol), and *N,N*-dimethylformamide (100 mL) were added sequentially to a reaction flask, and the mixed solution was heated and reacted at 75 °C for 16 h. After the reaction was completed, the reaction solution was extracted with ethyl acetate (300 mL). The organic phase was washed with water (300 mL), then dried over anhydrous sodium sulfate, filtered, and concentrated. The crude product was purified by column chromatography (petroleum ether: ethyl acetate = 3:7) to give the target product (4.8 g, 37% yield).

### Step b: Synthesis of 7-(4-(4-(2,3-dichlorophenyl)piperazin-1-yl)butoxy)quinolin-2(1H)-one

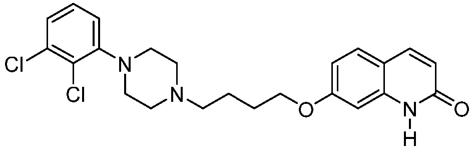

7-(4-Bromobutoxy)quinolin-2(1H)-one (4.8 g, 16.2 mmol), 1-(2,3-dichlorophenyl)piperazine (3.7 g, 16.2 mmol), potassium carbonate (6.7 g, 48.6 mmol), and acetonitrile (100 mL) each were added to a reaction flask, and the mixed solution was heated and reacted at 70 °C for 16 h. After the reaction was completed, the reaction solution was slowly cooled to room temperature and filtered to give a filter cake. The filter cake was washed with water, and then dried to give the target product (3.0 g, 41% yield).

### Preparation of 4-(chloromethyl)-1,2-dimethoxybenzene

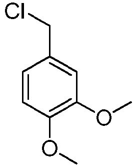

Pyridine (2 mL) and thionyl chloride (3 mL) were added to a solution of (3,4-dimethoxyphenyl)methanol (1.68 g, 10.0 mmol) in diethyl ether (28 mL) at 0 °C. The reaction mixture was stirred at this temperature for 20 min. The reaction solution was then poured into ice water (100 mL) and extracted with ether (100 mL). The organic phase was washed with a saturated aqueous sodium bicarbonate solution (100 mL), ice water (100 mL), and saturated brine (50 mL) sequentially, dried over anhydrous sodium sulfate, filtered, and concentrated to give the target product (1.2 g, 65% yield). The crude product was directly used in the next step without purification.

### Step c: Synthesis of 7-(4-(4-(2,3-dichlorophenyl)piperazin-1-yl)butoxy)-1-(3,4-dimethoxybenzyl)quinolin-2(1H)-one

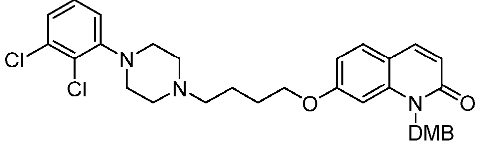

Sodium hydride (2.5 g, 13.4 mmol) was added to a solution of 7-(4-(4-(2,3-dichlorophenyl)piperazin-1-yl)butyl)quinolin-2(1H)-one (1.2 g, 2.7 mmol) in *N,N*-dimethylformamide (20 mL) at room temperature, and then the reaction solution was cooled to 0 °C. After the reaction solution was reacted for 10 min, 4-(chloromethyl)-1,2-dimethoxybenzene (540 mg, 13.4 mmol) was added, and the reaction solution was warmed to room temperature and reacted for 4 h. After the reaction was completed, the reaction solution was poured into a saturated aqueous ammonium chloride solution (100 mL) and extracted with ethyl acetate. The organic phase was washed with water (3 × 400 mL) and saturated brine (100 mL), dried over anhydrous sodium sulfate, filtered, and concentrated. The crude product was purified by column chromatography (dichloromethane:methanol = 20:1) to give the target product (1.24 g, 81% yield).

¹H NMR (400 MHz, *d₆*-DMSO): δ 7.64 (d, *J* = 9.2 Hz, 1H), 7.44 (d, *J* = 8.4 Hz, 1H), 7.26-7.25 (m, 1H), 7.20 (t, *J* = 8.0 Hz, 1H), 7.03 (d, *J* = 8.0 Hz, 1H), 6.83 (d, *J* = 8.4 Hz, 1H), 6.76-6.72 (m, 2H), 6.64 (d, *J* = 9.6 Hz, 1H), 6.52 (d, *J* = 2.4 Hz, 1H), 6.34 (dd, *J* = 8.4, 1.6 Hz, 1H), 5.45 (s, 2H), 3.98 (t, *J* = 5.6 Hz, 2H), 3.95 (s, 3H), 3.75 (s, 3H), 3.62 (br s, 4H), 3.39-3.35 (m, 2H), 3.04 (br s, 4H), 2.15-2.14 (m, 2H), 1.88-1.85 (m, 2H).

### Step d: Synthesis of 5-(4-(4-(2,3-dichlorophenyl)piperazin-1-yl)butoxy)-3-(3,4-dimethoxybenzyl)-3,7b-dihydro-1H-cyclopropa[c]quinolin-2(1aH)-one

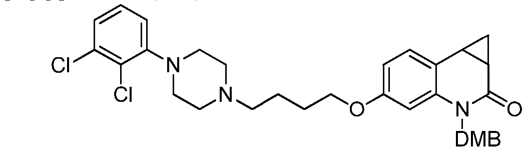

*n*-Butyllithium (20 mL, 2.5 M, 50.0 mmol) was added to a solution of trimethylsulfoxonium iodide (11.05 g, 50.0 mmol) in tetrahydrofuran (200 mL) at 0 °C, and the mixed solution was reacted at this temperature for 30 min. A solution of 7-(4-(4-(2,3-dichlorophenyl)piperazin-1-yl)butoxy)quinofin-2(1H)-one (598 mg, 1.0 mmol) in tetrahydrofuran (3 mL) was then added, and the mixed solution was reacted at 50 °C for 12 h. After the reaction was completed, the reaction solution was poured into a saturated aqueous ammonium chloride solution (100 mL) and extracted with ethyl acetate (300 mL). The organic phase was washed with water (2 × 300 mL) and saturated brine (100 mL), dried over anhydrous sodium sulfate, filtered, and concentrated. The crude product was purified by column chromatography (dichloromethane:methanol = 20:1) to give the target product (460 mg, 76% yield).

MS Found: 610.2[M+H]⁺.

### Step e: Synthesis of 5-(4-(4-(2,3-dichlorophenyl)piperazin-1-yl)butoxy)-3,7b-dihydro-1H-cyclopropa[c]quinolin-2(1aH)-one

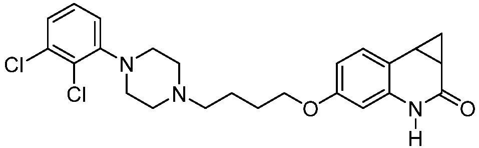

5-(4-(4-(2,3-Dichlorophenyl)piperazin-1-yl)butoxy)-3-(3,4-dimethoxybenzyl)-3,7b-dihydro-1H-cyclopropa[c]quinolin-2(1aH)-one (460 mg, 0.76 mmol) and trifluoroacetic acid (4 mL) each were added to a reaction flask, and the mixed solution was reacted at room temperature for 4 h. The reaction solution was concentrated and then dissolved in dichloromethane (100 mL). The mixed solution was washed with a saturated aqueous sodium bicarbonate solution (50 mL) and aqueous sodium hydroxide solution (10 mL, 1 M), dried over anhydrous sodium sulfate, filtered, and concentrated. The crude product was purified by high performance liquid chromatography (dichloromethane:methanol = 20:1) to give the target product (81 mg, 23% yield).

¹H NMR (400 MHz, *d₆*-DMSO): δ 9.81 (brs, 1H), 7.32-7.27 (m, 2H), 7.23 (d, *J* = 8.4 Hz, 1H), 7.14 (dd, *J* = 6.4, 2.8 Hz, 1H), 6.51 (dd, *J* = 8.4, 2.4 Hz, 1H), 6.43 (d, *J* = 2.4 Hz, 1H), 3.91 (t, *J* = 6.4 Hz, 2H), 2.97 (brs, 4H), 2.58-2.42 (m, 5H), 2.39 (t, *J* = 7.2 Hz, 2H), 1.97-1.89 (m, 1H), 1.76-1.66 (m, 2H), 1.64-1.45 (m, 3H), 0.43 (q, *J* = 4.8 Hz, 1H).

MS Found: 460.1[M+H]⁺.

The compound in Example 1 was resolved by chiral chromatography to give optical enantiomer 1 and optical enantiomer 2. Liquid chromatography: chromatographic column: DAICEL CHIRALCEL OJ column; mobile phase A: supercritical CO₂, mobile phase B: methanol (containing 0.1% dimethylamine); detection wavelength: 214 nm; flow rate: 1.5 mL/min; column temperature: 35 °C; background column pressure: 1800 psi. Mobile phase gradient:

| Time (min) | A (%V/V) | B (%V/V) |
|---|---|---|
| 0.00 | 60 | 40 |
| 10.00 | 60 | 40 |

### Optical enantiomer 1 (Example 1-P1, compound 55):

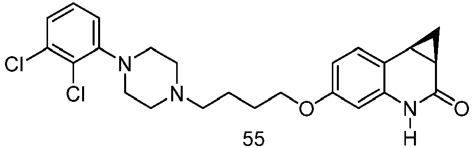

RT: 2.38 min; [α]D²⁰ = -54.6 (c 0.20, MeOH); MS Found: 461.0[M+H]⁺.

¹H NMR (400 MHz, CDCl₃): δ 8.64 (s, 1H), 7.21 (d, *J* = 8.4 Hz, 1H), 7.16-7.11 (m, 2H), 6.95 (dd, *J* = 6.4, 2.8 Hz, 1H), 6.54 (dd, *J* = 8.4, 2.0 Hz, 1H), 6.34 (d, *J* = 1.6 Hz, 1H), 3.95 (t, *J* = 6.4 Hz, 2H), 3.07 (brs, 4H), 2.66 (brs, 4H), 2.50-2.43 (m, 3H), 2.12-2.07 (m, 1H), 1.82-1.77 (m, 2H), 1.74-1.66 (m, 2H), 1.60-1.55 (m, 1H), 0.65 (q, *J* = 4.8 Hz, 1H).

### Optical enantiomer 2 (Example 1-P2, compound 54):

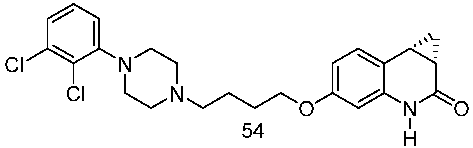

RT: 3.01 min; [α]D²⁰ = +60.9 (c 0.21, MeOH); MS Found: 461.4[M+H]⁺.

¹H NMR (400 MHz, CDCl₃): δ 8.46 (s, 1H), 7.21 (d, *J* = 8.4 Hz, 1H), 7.16-7.11 (m, 2H), 6.95 (dd, *J* = 6.4, 2.8 Hz, 1H), 6.54 (dd, *J* = 8.4, 2.0 Hz, 1H), 6.32 (d, *J* = 2.0 Hz, 1H), 3.96 (t, *J* = 6.0 Hz, 2H), 3.07 (brs, 4H), 2.65 (brs, 4H), 2.50-2.43 (m, 3H), 2.14-2.09 (m, 1H), 1.83-1.77 (m, 2H), 1.74-1.66 (m, 2H), 1.61-1.55 (m, 1H), 0.65 (q, *J* = 4.8 Hz, 1H).

### Examples 2-15

For synthetic routes for compounds 2-15, reference was made to the synthetic route for the compound of Example 1.

### Example 16

### 5-(4-(4-(3-Chloro-2-fluorophenyl)piperazin-1-yl)butoxy)-1,1a,3,7b-tetrahydro-2H-cyclopropa[c]quinolin-2-one

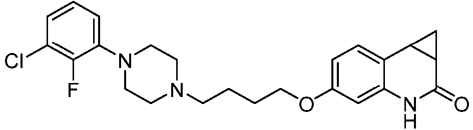

### Synthesis scheme:

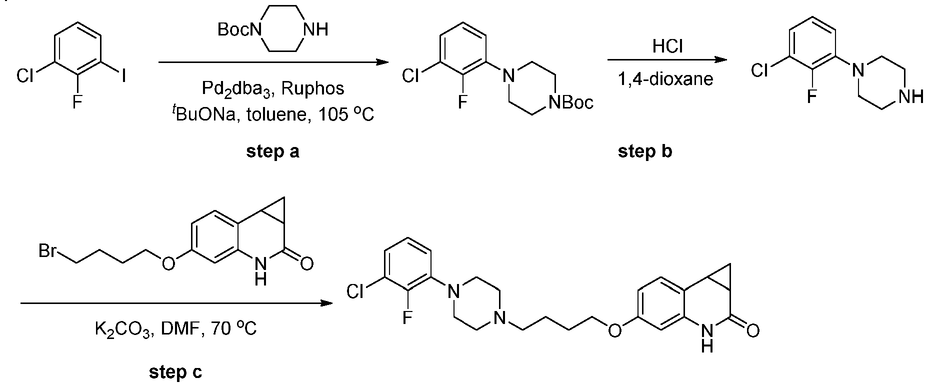

### Step a: Synthesis of tert-butyl 4-(3-chloro-2-fluorophenyl)piperazine-1-carboxylate

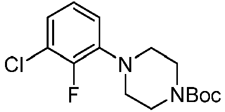

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert-butyl* piperazine-1-carboxylate (838.1 mg, 4.5 mmol) each were added to a dry reaction flask, and then the reaction flask was purged three times with nitrogen in a vacuum pump. Extra-dry toluene (30 mL) and 1-chloro-2-fluoro-3-iodobenzene (767.7 mg, 3 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile: 1‰ trifluoroacetic acid in water = 4:5) to give the target product (300 mg, 32% yield).

### Step b: Synthesis of 1-(3-chloro-2-fluorophenyl)piperazine

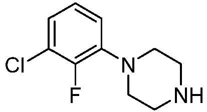

*tert*-Butyl 4-(3-chloro-2-fluorophenyl)piperazine-1-carboxylate (300 mg, 0.96 mmol) and a solution of hydrochloric acid in 1,4-dioxane (0.1 M, 10 mL) each were added to a reaction flask, and the mixed solution was reacted at room temperature for 3 h. After the reaction was completed, the reaction solution was concentrated to give crude 1-(3-chloro-2-fluorophenyl)piperazine hydrochloride, which was directly used in the next step without purification.

### Step c: Synthesis of 5-(4-(4-(3-chloro-2-fluorophenyl)piperazin-1-yl)butoxy)-1,1a,3,7b-tetrahydro-2H-cyclopropa[c]quinolin-2-one

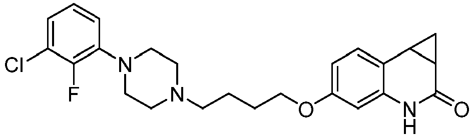

5-(4-Bromobutoxy)-1,1a,3,7b-tetrahydro-2H-cyclopropa[c]quinolin-2-one (231.8 mg, 0.75 mmol), 1-(3-chloro-2-fluorophenyl)piperazine hydrochloride (125.0 mg, 0.5 mmol), potassium carbonate (207.3 mg, 1.5 mmol), and *N,N*-dimethylformamide (5 mL) each were added to a reaction flask, and the mixed solution was reacted at 70 °C for 8 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (40 mg, 18% yield).

¹H NMR (600 MHz, CD₃OD): δ 7.27 (d, *J* = 8.4 Hz, 1H), 7.18-7.15 (m, 1H), 7.12 (td, *J* = 8.4, 1.2 Hz, 1H), 7.05 (td, *J* = 7.8, 1.2 Hz, 1H), 6.61 (dd, *J* = 8.4, 2.4 Hz, 1H), 6.45 (d, *J* = 2.4 Hz, 1H), 4.03 (t, *J* = 6.0 Hz, 2H), 3.70 (d, *J* = 12.0 Hz, 2H), 3.61 (d, *J* = 10.8 Hz, 2H), 3.34-3.29 (m, 4H), 3.16 (t, *J* = 12.6 Hz, 2H), 2.56-2.52 (m, 1H), 2.07-2.03 (m, 1H), 2.02-1.96 (m, 2H), 1.91-1.86 (m, 2H), 1.65-1.61 (m, 1H), 0.49 (q, *J =* 4.8 Hz, 1H).

MS Found: 444.3 [M+H]⁺.

### Example 17

### 5-(4-(4-(2-Chloro-3-fluorophenyl)piperazin-1-yl)butoxy)-1,1a,3,7b-tetrahydro-2H-cyclopropa[c]quinolin-2-one

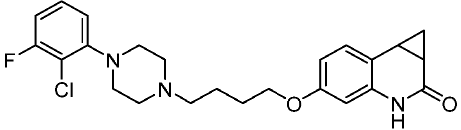

### Synthesis scheme:

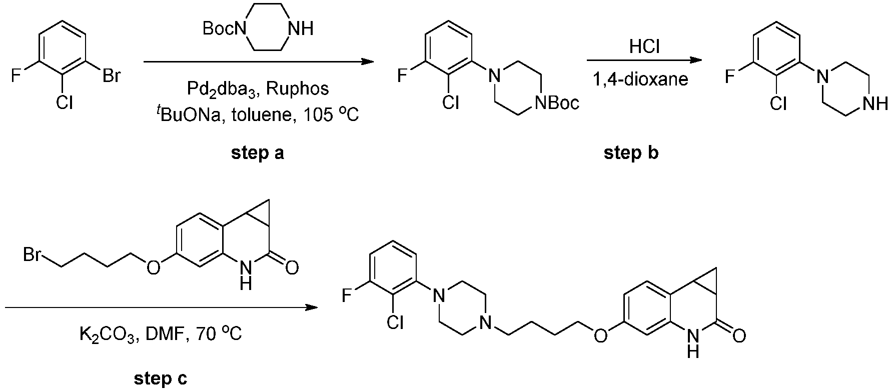

### Step a: Synthesis of tert-butyl 4-(2-chloro-3-fluorophenyl)piperazine-1-carboxylate

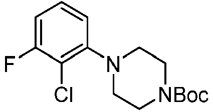

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert-butyl* piperazine-1-carboxylate (838.1 mg, 4.5 mmol) each were added to a dry reaction flask, and then the reaction flask was purged three times with nitrogen in a vacuum pump. Extra-dry toluene (30 mL) and 1-bromo-2-chloro-3-fluorobenzene (623.7 mg, 3 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether: ethyl acetate = 10:1) to give the target product (310 mg, 33% yield).

### Step b: Synthesis of 1-(2-chloro-3-fluorophenyl)piperazine

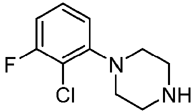

tert-Butyl 4-(2-chloro-3-fluorophenyl)piperazine-1-carboxylate (310 mg, 0.99 mmol) and a solution of hydrochloric acid in 1,4-dioxane (0.1 M, 10 mL) each were added to a reaction flask, and the mixed solution was reacted at room temperature for 3 h. After the reaction was completed, the reaction solution was concentrated to give crude 1-(2-chloro-3-fluorophenyl)piperazine hydrochloride, which was directly used in the next step without purification.

### Step c: Synthesis of 5-(4-(4-(2-chloro-3-fluorophenyl)piperazin-1-yl)butoxy)-1,1a,3,7b-tetrahydro-2H-cyclopropa[c]quinolin-2-one

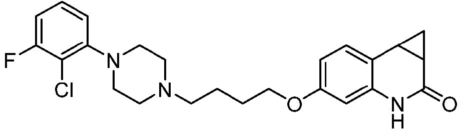

5-(4-Bromobutoxy)-1,1a,3,7b-tetrahydro-2H-cyclopropa[c]quinolin-2-one (231.8 mg, 0.75 mmol), 1-(2-chloro-3-fluorophenyl)piperazine hydrochloride (125.0 mg, 0.5 mmol), potassium carbonate (207.3 mg, 1.5 mmol), and *N,N*-dimethylformamide (5 mL) each were added to a reaction flask, and the mixed solution was reacted at 70 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (63 mg, 28% yield).

¹H NMR (600 MHz, CD₃OD): δ 7.32 (td, *J* = 8.4, 6.0 Hz, 1H), 7.26 (d, *J* = 8.4 Hz, 1H), 7.04-7.01 (m, 2H), 6.60 (dd, *J* = 8.4, 2.4 Hz, 1H), 6.44 (d, *J* = 2.4 Hz, 1H), 4.03 (t, *J* = 6.0 Hz, 2H), 3.71 (d, *J* = 12.0 Hz, 2H), 3.57 (d, *J* = 13.2 Hz, 2H), 3.35-3.31 (m, 4H), 3.14 (t, *J* = 12.0 Hz, 2H), 2.55-2.52 (m, 1H), 2.06-2.02 (m, 1H), 2.01-1.97 (m, 2H), 1.91-1.86 (m, 2H), 1.64-1.61 (m, 1H), 0.49 (q, *J* = 4.8 Hz, 1H).

MS Found: 444.1[M+H]⁺.

### Example 18

### 5-(4-(4-(2,3-Difluorophenyl)piperazin-1-yl)butoxy)-1,1a,3,7b-tetrahydro-2H-cyclopropa[c]quinolin-2-one

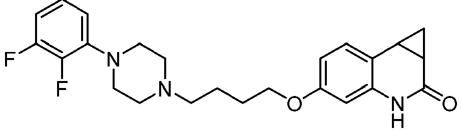

### Synthesis scheme:

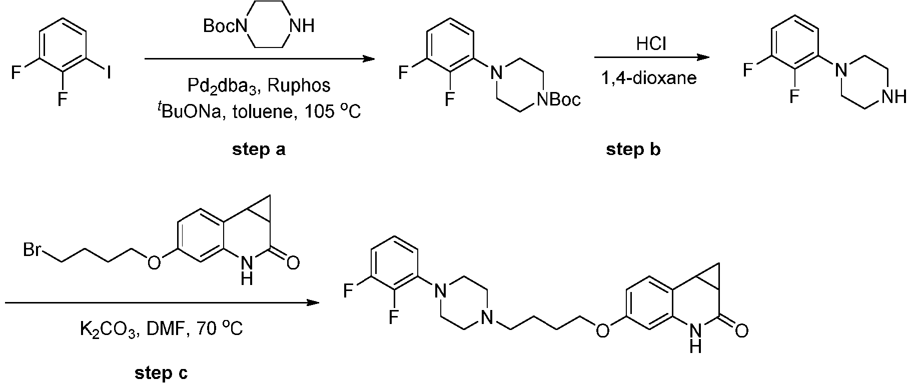

### Step a: Synthesis of tert-butyl 4-(2,3-difluorophenyl)piperazine-1-carboxylate

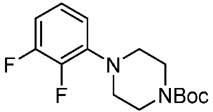

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert-butyl* piperazine-1-carboxylate (838.1 mg, 4.5 mmol) each were added to a dry reaction flask, and then the reaction flask was purged three times with nitrogen in a vacuum pump. Extra-dry toluene (30 mL) and 1,2-difluoro-3-iodobenzene (720 mg, 3 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (750 mg, 84% yield)

### Step b: Synthesis of 1-(2,3-difluorophenyl)piperazine

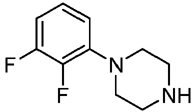

tert-Butyl 4-(2,3-difluorophenyl)piperazine-1-carboxylate (200 mg, 0.67 mmol) and a solution of hydrochloric acid in 1,4-dioxane (0.1 M, 7 mL) each were added to a reaction flask, and the mixed solution was reacted at room temperature for 3 h. After the reaction was completed, the reaction solution was concentrated to give crude 1-(2,3-difluorophenyl)piperazine hydrochloride, which was directly used in the next step without purification.

### Step c: Synthesis of 5-(4-(4-(2,3-difluorophenyl)piperazin-1-yl)butoxy)-1,1a,3,7b-tetrahydro-2H-cyclopropa[c]quinolin-2-one

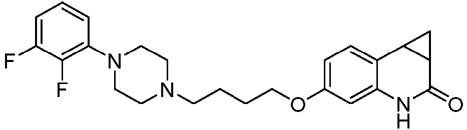

5-(4-Bromobutoxy)-1,1a,3,7b-tetrahydro-2H-cyclopropa[c]quinolin-2-one (231.8 mg, 0.75 mmol), 1-(2,3-difluorophenyl)piperazine hydrochloride (117.0 mg, 0.5 mmol), potassium carbonate (207.3 mg, 1.5 mmol), and *N,N*-dimethylformamide (5 mL) each were added to a reaction flask, and the mixed solution was reacted at 70 °C for 6 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (26 mg, 12% yield).

¹H NMR (600 MHz, CD₃OD): δ 7.26 (d, *J* = 8.4 Hz, 1H), 7.13-7.09 (m, 1H), 6.98-6.94 (m, 1H), 6.89 (t, *J* = 7.8 Hz, 1H), 6.60 (dd, *J* = 8.4, 2.4 Hz, 1H), 6.45 (d, *J* = 2.4 Hz, 1H), 4.02 (t, *J* = 6.0 Hz, 2H), 3.70 (d, *J* = 12.0 Hz, 2H), 3.63 (d, *J* = 13.2 Hz, 2H), 3.34-3.29 (m, 4H), 3.17 (t, *J* = 12.6 Hz, 2H), 2.56-2.52 (m, 1H), 2.06-2.03 (m, 1H), 2.02-1.96 (m, 2H), 1.90-1.86 (m, 2H), 1.65-1.61 (m, 1H), 0.49 (q, *J* = 4.8 Hz, 1H).

MS Found: 428.4[M+H]⁺.

### Example 19

### 5-(4-(4-(3-Chlorophenyl)piperazin-1-yl)butoxy)-1,1a,3,7b-tetrahydro-2H-cyclopropa[c]quinolin-2-one

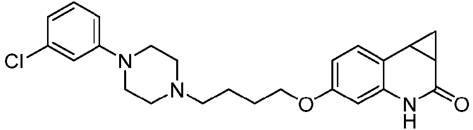

### Synthesis scheme:

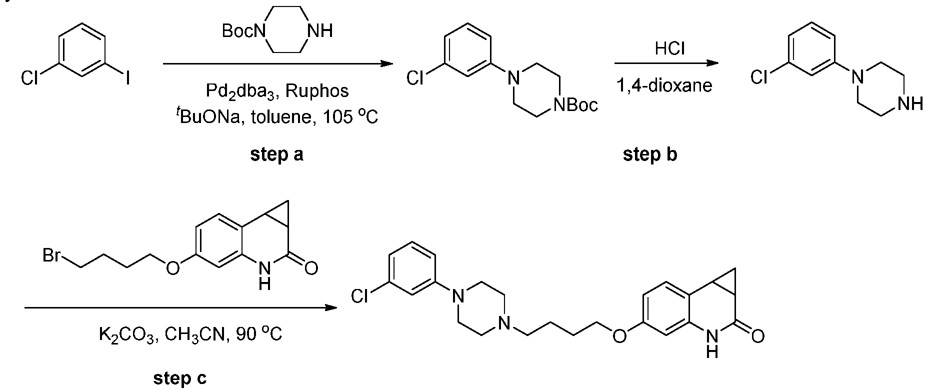

### Step a: Synthesis of tert-butyl 4-(3-chlorophenyl)piperazine-1-carboxylate

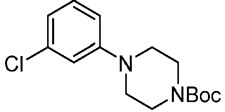

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert-butyl* piperazine-1-carboxylate (838.1 mg, 4.5 mmol) each were added to a dry reaction flask, and then the reaction flask was purged three times with nitrogen in a vacuum pump. Extra-dry toluene (30 mL) and 1-chloro-3-iodobenzene (713.7 mg, 3 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (800 mg, 90% yield)

### Step b: Synthesis of 1-(3-chlorophenyl)piperazine

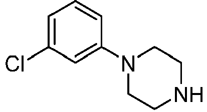

*tert*-Butyl 4-(3-chlorophenyl)piperazine-1-carboxylate (200 mg, 0.68 mmol) and a solution of hydrochloric acid in 1,4-dioxane (0.1 M, 7 mL) each were added to a reaction flask, and the mixed solution was reacted at room temperature for 3 h. After the reaction was completed, the reaction solution was concentrated to give crude 1-(3-chlorophenyl)piperazine hydrochloride, which was directly used in the next step without purification.

### Step c: Synthesis of 5-(4-(4-(3-chlorophenyl)piperazin-1-yl)butoxy)-1,1a,3,7b-tetrahydro-2H-cyclopropa[c]quinolin-2-one

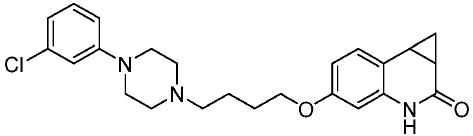

5-(4-Bromobutoxy)-1,1a,3,7b-tetrahydro-2H-cyclopropa[c]quinolin-2-one (67.9 mg, 0.22 mmol), 1-(3-chlorophenyl)piperazine hydrochloride (60.3 mg, 0.26 mmol), potassium carbonate (91.2 mg, 0.66 mmol), and acetonitrile (7 mL) each were added to a reaction flask, and the mixed solution was reacted at 90 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (38 mg, 41% yield).

¹H NMR (600 MHz, CD₃OD): δ 7.27-7.24 (m, 2H), 7.04 (t, *J* = 2.4 Hz, 1H), 6.95 (dd, *J* = 7.8, 2.4 Hz, 1H), 6.92 (dd, *J* = 7.8, 1.2 Hz, 1H), 6.61 (dd, *J* = 8.4, 2.4 Hz, 1H), 6.45 (d, *J* = 2.4 Hz, 1H), 4.03 (t, *J* = 6.0 Hz, 2H), 3.86 (s, 2H), 3.68 (s, 2H), 3.31-3.11 (m, 6H), 2.56-2.52 (m, 1H), 2.07-2.03 (m, 1H), 2.02-1.96 (m, 2H), 1.91-1.86 (m, 2H), 1.65-1.61 (m, 1H), 0.49 (q, *J* = 4.8 Hz, 1H).

MS Found: 426.4[M+H]⁺.

### Example 20

### 5-(4-(4-(2-Chlorophenyl)piperazin-1-yl)butoxy)-1,1a,3,7b-tetrahydro-2H-cyclopropa[c]quinolin-2-one

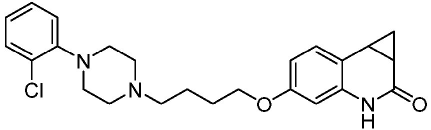

### Synthesis scheme:

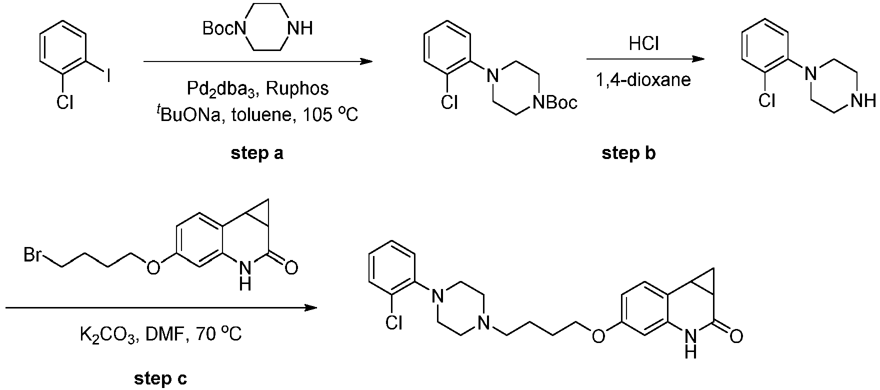

### Step a: Synthesis of tert-butyl 4-(2-chlorophenyl)piperazine-1-carboxylate

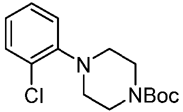

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert-butyl* piperazine-1-carboxylate (838.1 mg, 4.5 mmol) each were added to a dry reaction flask, and then the reaction flask was purged three times with nitrogen in a vacuum pump. Extra-dry toluene (30 mL) and 1-chloro-2-iodobenzene (713.7 mg, 3 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (750 mg, 84% yield)

### Step b: Synthesis of 1-(2-chlorophenyl)piperazine

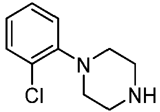

tert-Butyl 4-(2-chlorophenyl)piperazine-1-carboxylate (200 mg, 0.68 mmol) and a solution of hydrochloric acid in 1,4-dioxane (0.1 M, 7 mL) each were added to a reaction flask, and the mixed solution was reacted at room temperature for 3 h. After the reaction was completed, the reaction solution was concentrated to give 1-(2-chlorophenyl)piperazine hydrochloride, which was directly used in the next step without purification.

### Step c: Synthesis of 5-(4-(4-(2-chlorophenyl)piperazin-1-yl)butoxy)-1,1a,3,7b-tetrahydro-2H-cyclopropa[c]quinolin-2-one

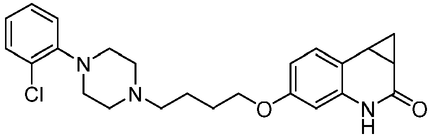

5-(4-Bromobutoxy)-1,1a,3,7b-tetrahydro-2H-cyclopropa[c]quinolin-2-one (336.8 mg, 1.09 mmol), 1-(2-chlorophenyl)piperazine hydrochloride (169.4 mg, 0.73 mmol), potassium carbonate (302.7 mg, 2.19 mmol), and *N,N*-dimethylformamide (7 mL) each were added to a reaction flask and the mixed solution was reacted at 70 °C for 5 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (25 mg, 8% yield).

¹H NMR (600 MHz, CD₃OD): δ 7.41 (dd, *J* = 7.8, 1.8 Hz, 1H), 7.32-7.29 (m, 1H), 7.26 (d, *J* = 8.4 Hz, 1H), 7.18 (dd, *J* = 7.8, 1.2 Hz, 1H), 7.11-7.08 (m, 1H), 6.59 (dd, *J* = 8.4, 2.4 Hz, 1H), 6.43 (d, *J* = 2.4 Hz, 1H), 4.02 (t, *J* = 6.0 Hz, 2H), 3.69 (d, *J* = 12.0 Hz, 2H), 3.53 (d, *J* = 13.2 Hz, 2H), 3.33-3.30 (m, 4H), 3.10 (t, *J* = 11.4 Hz, 2H), 2.55-2.51 (m, 1H), 2.05-2.01 (m, 1H), 2.00-1.96 (m, 2H), 1.92-1.85 (m, 2H), 1.64-1.60 (m, 1H), 0.48 (q, *J* = 4.8 Hz, 1H).

MS Found: 426.3 [M+H]⁺.

### Examples 21-28

For synthetic routes for compounds 21-28, reference was made to the synthetic route for the compound of Example 1.

### Example 29

### 5-(4-(4-(o-Tolyl)piperazin-1-yl)butoxy)-1,1a,3,7b-tetrahydro-2H-cyclopropa[c]quinolin-2-one

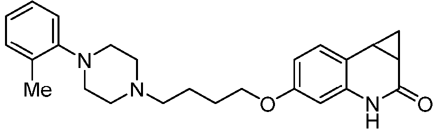

### Synthesis scheme:

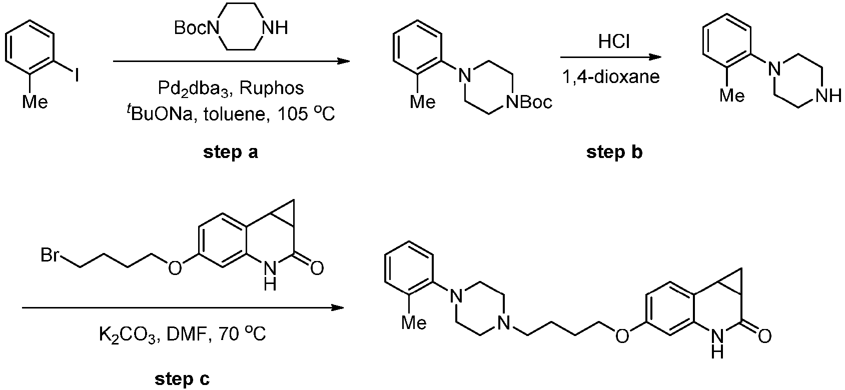

### Step a: Synthesis of tert-butyl 4-(o-tolyl)piperazine-1-carboxylate

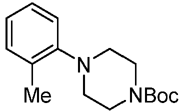

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert-butyl* piperazine-1-carboxylate (838.1 mg, 4.5 mmol) each were added to a dry reaction flask, and then the reaction flask was purged three times with nitrogen in a vacuum pump. Extra-dry toluene (30 mL) and 1-iodo-2-toluene (654.1 mg, 3 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (550 mg, 66% yield)

### Step b: Synthesis of 1-(o-tolyl)piperazine

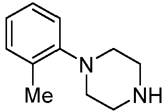

tert-Butyl 4-(o-tolyl)piperazine-1-carboxylate (200 mg, 0.72 mmol) and a solution of hydrochloric acid in 1,4-dioxane (0.1 M, 7 mL) each were added to a reaction flask, and the mixed solution was reacted at room temperature for 3 h. After the reaction was completed, the reaction solution was concentrated to give crude 1-(o-tolyl)piperazine hydrochloride, which was directly used in the next step without purification.

### Step c: Synthesis of 5-(4-(4-(o-tolyl)piperazin-1-yl)butoxy)-1,1a,3,7b-tetrahydro-2H-cyclopropa[c]quinolin-2-one

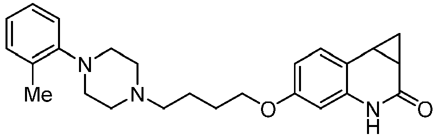

5-(4-Bromobutoxy)-1,1a,3,7b-tetrahydro-2H-cyclopropa[c]quinolin-2-one (278.1 mg, 0.9 mmol), 1-(*o-*tolyl)piperazine hydrochloride (127.3 mg, 0.6 mmol), potassium carbonate (248.8 mg, 1.80 mmol), and *N,N-*dimethylformamide (10 mL) each were added to a reaction flask, and the mixed solution was reacted at 70 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (39 mg, 16% yield).

¹H NMR (600 MHz, CD₃OD): δ 7.26 (d, *J* = 8.4 Hz, 1H), 7.21-7.16 (m, 2H), 7.08 (d, *J* = 7.8 Hz, 1H), 7.03 (t, *J* = 7.2 Hz, 1H), 6.60 (dd, *J* = 8.4, 2.4 Hz, 1H), 6.44 (d, *J* = 2.4 Hz, 1H), 4.02 (t, *J* = 6.0 Hz, 2H), 3.66 (d, *J* = 12.0 Hz, 2H), 3.31-3.25 (m, 6H), 3.08 (t, *J* = 12.0 Hz, 2H), 2.55-2.51 (m, 1H), 2.32 (s, 3H), 2.06-2.02 (m, 1H), 2.01-1.96 (m, 2H), 1.90-1.86 (m, 2H), 1.64-1.60 (m, 1H), 0.48 (q, *J* = 4.2 Hz, 1H).

MS Found: 406.4[M+H]⁺.

### Example 30

### 5-(4-(4-(m-Tolyl)piperazin-1-yl)butoxy)-1,1a,3,7b-tetrahydro-2H-cyclopropa[c]quinolin-2-one

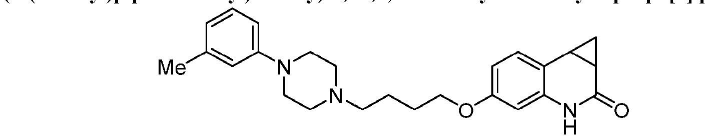

### Synthesis scheme:

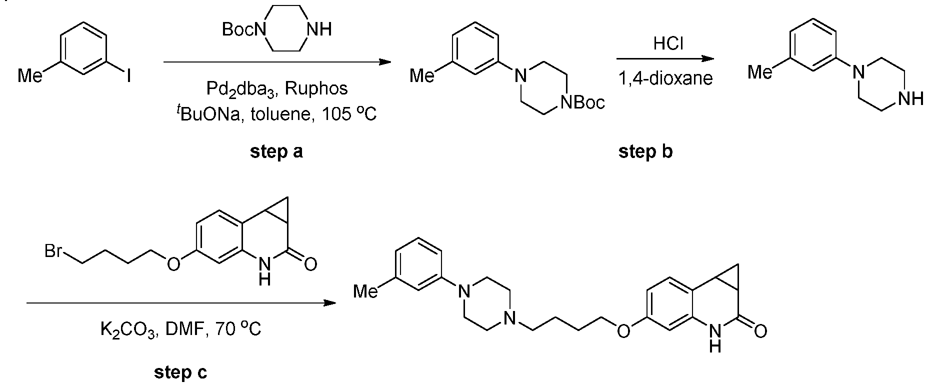

### Step a: Synthesis of tert-butyl 4-(m-tolyl)piperazine-1-carboxylate

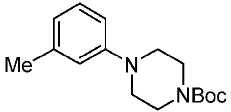

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert-butyl* piperazine-1-carboxylate (838.1 mg, 4.5 mmol) each were added to a dry reaction flask, and then the reaction flask was purged three times with nitrogen in a vacuum pump. Extra-dry toluene (30 mL) and 1-iodo-3-toluene (654.1 mg, 3 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (690 mg, 83% yield)

### Step b: Synthesis of 1-(m-tolyl)piperazine

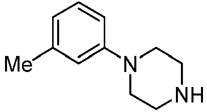

*tert*-Butyl 4-(*m*-tolyl)piperazine-1-carboxylate (200 mg, 0.72 mmol) and a solution of hydrochloric acid in 1,4-dioxane (0.1 M, 7 mL) each were added to a reaction flask, and the mixed solution was reacted at room temperature for 3 h. After the reaction was completed, the reaction solution was concentrated to give crude 1-(m-tolyl)piperazine hydrochloride, which was directly used in the next step without purification.

### Step c: Synthesis of 5-(4-(4-(m-tolyl)piperazin-1-yl)butoxy)-1,1a,3,7b-tetrahydro-2H-cyclopropa[c]quinolin-2-one

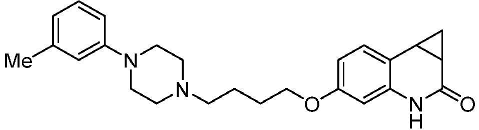

5-(4-Bromobutoxy)-1,1a,3,7b-tetrahydro-2H-cyclopropa[c]quinolin-2-one (278.1 mg, 0.9 mmol), 1-(m-tolyl)piperazine hydrochloride (127.3 mg, 0.6 mmol), potassium carbonate (248.8 mg, 1.8 mmol), and *N,N-*dimethylformamide (5 mL) each were added to a reaction flask, and the mixed solution was reacted at 70 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (36 mg, 15% yield).

¹H NMR (600 MHz, CD₃OD): δ 7.23 (d, *J* = 8.4 Hz, 1H), 7.13 (t, *J* = 7.8 Hz, 1H), 6.82 (s, 1H), 6.78 (dd, *J* = 7.8, 2.4 Hz, 1H), 6.74 (d, *J* = 7.2 Hz, 1H), 6.57 (dd, *J* = 8.4, 2.4 Hz, 1H), 6.42 (d, *J* = 2.4 Hz, 1H), 3.99 (t, *J* = 6.0 Hz, 2H), 3.78 (d, *J* = 12.0 Hz, 2H), 3.65 (d, *J* = 10.2 Hz, 2H), 3.27-3.18 (m, 4H), 3.03 (t, *J* = 11.4 Hz, 2H), 2.52-2.48 (m, 1H), 2.28 (s, 3H), 2.03-1.99 (m, 1H), 1.99-1.93 (m, 2H), 1.87-1.83 (m, 2H), 1.61-1.57 (m, 1H), 0.46 (q, *J* = 4.8 Hz, 1H).

MS Found: 406.1[M+H]⁺.

### Examples 31-32

For synthetic routes for compounds 31-32, reference was made to the synthetic route for the compound of Example 16.

### Example 33

### 5-(4-(4-(2,3-Dimethylphenyl)piperazin-1-yl)butoxy)-1,1a,3,7b-tetrahydro-2H-cyclopropa[c]quinolin-2-one

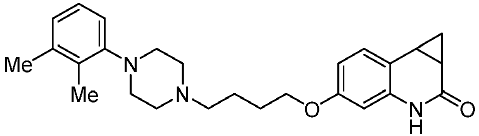

### Synthesis scheme:

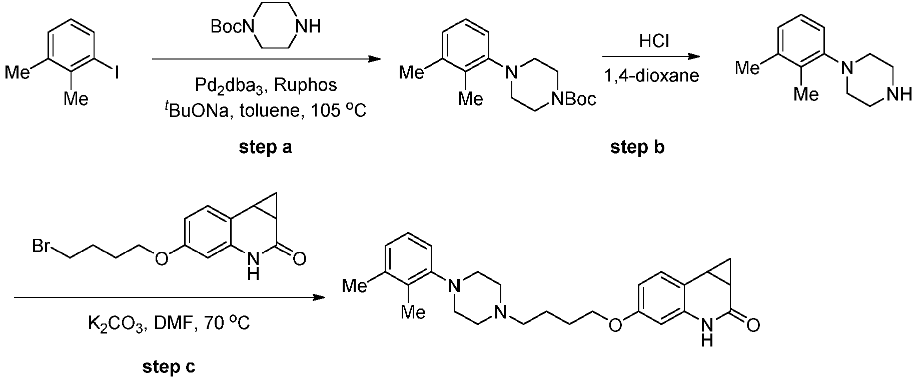

### Step a: Synthesis of tert-butyl 4-(2,3-dimethylphenyl)piperazine-1-carboxylate

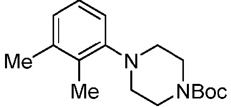

Tris(dibenzylideneacetone)dipalladium(0) (118.5 mg, 0.13 mmol), 2-dicyclohexylphosphonium-2',6'-diisopropoxy-1,1'-biphenyl (247.7 mg, 0.52 mmol), sodium *tert*-butoxide (373.3 mg, 3.89 mmol), and *tert-*butyl piperazine-1-carboxylate (724.5 mg, 3.89 mmol) each were added to a dry reaction flask, and then the reaction flask was purged three times with nitrogen in a vacuum pump. Extra-dry toluene (25 mL) and 1-iodo-2,3-xylene (600 mg, 2.59 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (452 mg, 60% yield)

### Step b: Synthesis of 1-(2,3-dimethylphenyl)piperazine

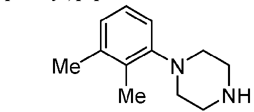

*tert*-Butyl 4-(2,3-dimethylphenyl)piperazine-1-carboxylate (452 mg, 1.56 mmol) and a solution of hydrochloric acid in 1,4-dioxane (0.1 M, 15 mL) each were added to a reaction flask, and the mixed solution was reacted at room temperature for 3 h. After the reaction was completed, the reaction solution was concentrated to give crude 1-(2,3-dimethylphenyl)piperazine hydrochloride, which was directly used in the next step without purification.

### Step c: Synthesis of 5-(4-(4-(2,3-dimethylphenyl)piperazin-1-yl)butoxy)-1,1a,3,7b-tetrahydro-2H-cyclopropa[c]quinolin-2-one

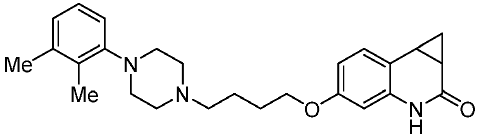

5-(4-Bromobutoxy)-1,1a,3,7b-tetrahydro-2H-cyclopropa[c]quinolin-2-one (275.0 mg, 0.89 mmol), 1-(2,3-dimethylphenyl)piperazine hydrochloride (133.4 mg, 0.59 mmol), potassium carbonate (244.6 mg, 1.77 mmol), and *N,N*-dimethylformamide (5 mL) each were added to a reaction flask, and the mixed solution was reacted at 70 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (44 mg, 18% yield).

¹H NMR (600 MHz, CD₃OD): δ 7.26 (d, *J* = 8.4 Hz, 1H), 7.07 (t, *J* = 7.8 Hz, 1H), 6.95 (d, *J* = 7.8 Hz, 2H), 6.60 (dd, *J* = 8.4, 2.4 Hz, 1H), 6.44 (d, *J* = 2.4 Hz, 1H), 4.02 (t, *J* = 6.0 Hz, 2H), 3.65 (d, *J* = 12.0 Hz, 2H), 3.33-3.28 (m, 4H), 3.21 (d, *J* = 13.2 Hz, 2H), 3.05 (t, *J* = 12.0 Hz, 2H), 2.54-2.51 (m, 1H), 2.26 (s, 3H), 2.24 (s, 3H), 2.05-2.02 (m, 1H), 2.00-1.96 (m, 2H), 1.90-1.86 (m, 2H), 1.64-1.60 (m, 1H), 0.48 (q, *J* = 4.8 Hz, 1H).

MS Found: 420.5[M+H]⁺.

### Example 34

### 2-Chloro-3-(4-(4-((2-oxa-1a,2,3,7b-tetrahydro-1H-cyclopropa[c]quinolin-5-yl)oxy)butyl)piperazin-1-yl)benzonitrile

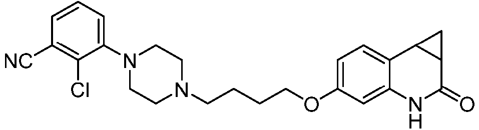

### Synthesis scheme:

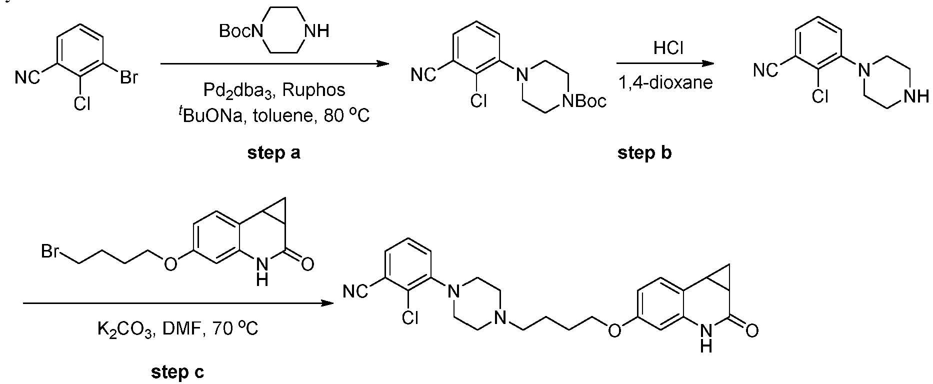

### Step a: Synthesis of tert-butyl 4-(2-chloro-3-cyanophenyl)piperazine-1-carboxylate

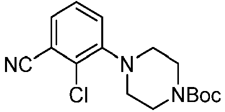

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert*-butyl piperazine-1-carboxylate (838.1 mg, 4.5 mmol) each were added to a dry reaction flask, and then the reaction flask was purged three times with nitrogen in a vacuum pump. Extra-dry toluene (30 mL) and 3-bromo-2-chlorobenzonitrile (644.7 mg, 3 mmol) were added under nitrogen atmosphere, and the mixed solution was reacted at 80 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 3:5) to give the target product (200 mg, 21% yield).

### Step b: Synthesis of 2-chloro-3-(piperazin-1-yl)benzonitrile

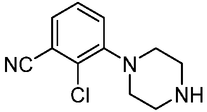

tert-Butyl 4-(2-chloro-3-cyanophenyl)piperazine-1-carboxylate (200 mg, 0.62 mmol) and a solution of hydrochloric acid in 1,4-dioxane (0.1 M, 6 mL) each were added to a reaction flask, and the mixed solution was reacted at room temperature for 3 h. After the reaction was completed, the reaction solution was concentrated to give crude 2-chloro-3-(piperazine-1-yl)benzonitrile hydrochloride, which was directly used in the next step without purification.

### Step c: Synthesis of 2-chloro-3-(4-(4-((2-oxa-1a,2,3,7b-tetrahydro-1H-cyclopropa[c]quinolin-5-yl)oxy)butyl)piperazin-1-yl)benzonitrile

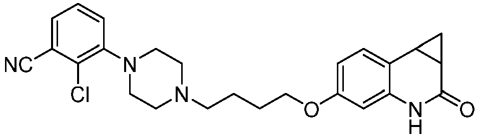

5-(4-Bromobutoxy)-1,1a,3,7b-tetrahydro-2H-cyclopropa[c]quinolin-2-one (222.5 mg, 0.72 mmol), 2-chloro-3-(piperazine-1-yl)benzonitrile hydrochloride (154.2 mg, 0.6 mmol), potassium carbonate (248.8 mg, 1.8 mmol), and *N,N*-dimethylformamide (5 mL) each were added to a reaction flask, and the mixed solution was reacted at 70 °C for 18 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 2:5) to give the target product (70 mg, 26% yield).

¹H NMR (600 MHz, CD₃OD): δ 7.56 (dd, *J* = 7.2, 1.8 Hz, 1H), 7.53-7.48 (m, 2H), 7.26 (d, *J* = 8.4 Hz, 1H), 6.61 (dd, *J* = 8.4, 2.4 Hz, 1H), 6.44 (d, *J* = 2.4 Hz, 1H), 4.03 (t, *J* = 6.0 Hz, 2H), 3.72 (d, *J* = 11.4 Hz, 2H), 3.57 (d, *J* = 12.6 Hz, 2H), 3.37-3.31 (m, 4H), 3.18 (t, *J* = 12.0 Hz, 2H), 2.56-2.52 (m, 1H), 2.06-2.04 (m, 1H), 2.02-1.98 (m, 2H), 1.91-1.87 (m, 2H), 1.65-1.61 (m, 1H), 0.50 (q, *J* = 4.8 Hz, 1H).

MS Found: 451.3[M+H]⁺.

### Example 35

### 2-Chloro-6-(4-(4-((2-oxa-1a,2,3,7b-tetrahydro-1H-cyclopropa[c]quinolin-5-yl)oxy)butyl)piperazin-1-yl)benzonitrile

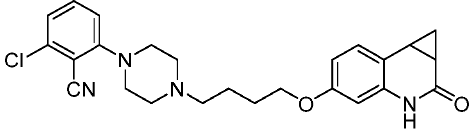

### Synthesis scheme:

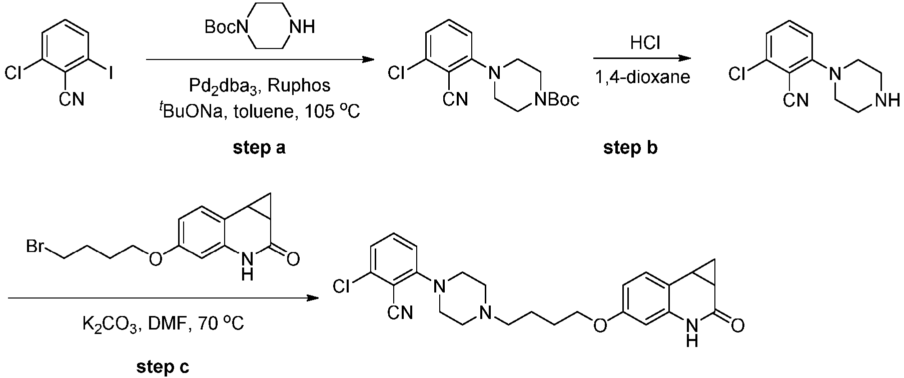

### Step a: Synthesis of tert-butyl 4-(3-chloro-2-cyanophenyl)piperazine-1-carboxylate

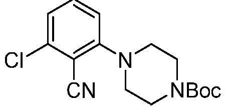

Tris(dibenzylideneacetone)dipalladium(0) (91.5 mg, 0.1 mmol), 2-dicyclohexylphosphonium-2',6'-diisopropoxy-1,1'-biphenyl (186.7 mg, 0.4 mmol), sodium *tert*-butoxide (288.3 mg, 3 mmol), and *tert-butyl* piperazine-1-carboxylate (558.8 mg, 3 mmol) each were added to a dry reaction flask, and then the reaction flask was purged three times with nitrogen in a vacuum pump. Extra-dry toluene (20 mL) and 2-chloro-6-iodobenzonitrile (525.8 mg, 2 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 5:1) to give the target product (416 mg, 65% yield).

### Step b: Synthesis of 2-chloro-6-(piperazin-1-yl)benzonitrile

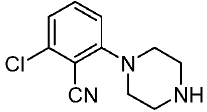

te*r*t-Butyl 4-(3-chloro-2-cyanophenyl)piperazine-1-carboxylate (416 mg, 1.29 mmol) and a solution of hydrochloric acid in 1,4-dioxane (0.1 M, 10 mL) each were added to a reaction flask, and the mixed solution was reacted at room temperature for 3 h. After the reaction was completed, the reaction solution was concentrated to give crude 2-chloro-6-(piperazine-1-yl)benzonitrile hydrochloride, which was directly used in the next step without purification.

### Step c: Synthesis of 2-chloro-6-(4-(4-((2-oxa-1a,2,3,7b-tetrahydro-1H-cyclopropa[c]quinolin-5-yl)oxy)butyl)piperazin-1-yl)benzonitrile

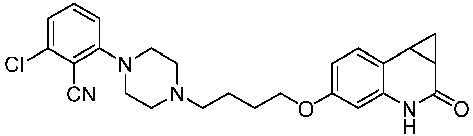

5-(4-Bromobutoxy)-1,1a,3,7b-tetrahydro-2H-cyclopropa[c]quinolin-2-one (231.8 mg, 0.75 mmol), 2-chloro-6-(piperazine-1-yl)benzonitrile hydrochloride (128.5 mg, 0.5 mmol), potassium carbonate (207.3 mg, 1.5 mmol), and *N,N*-dimethylformamide (5 mL) each were added to a reaction flask, and the mixed solution was reacted at 70 °C for 5 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 2:5) to give the target product (71 mg, 32% yield).

¹H NMR (600 MHz, CD₃OD): δ 7.61 (t, *J* = 8.4 Hz, 1H), 7.32 (d, *J* = 7.8 Hz, 1H), 7.27 (d, *J* = 8.4 Hz, 1H), 7.20 (d, *J* = 8.4 Hz, 1H), 6.61 (dd, *J* = 8.4, 2.4 Hz, 1H), 6.45 (d, *J* = 2.4 Hz, 1H), 4.03 (t, *J* = 6.0 Hz, 2H), 3.76 (d, *J* = 10.2 Hz, 4H), 3.35-3.26 (m, 6H), 2.56-2.53 (m, 1H), 2.07-2.03 (m, 1H), 2.01-1.97 (m, 2H), 1.91-1.87 (m, 2H), 1.65-1.62 (m, 1H), 0.50 (q, *J =* 4.8 Hz, 1H).

MS Found: 451.3[M+H]⁺.

### Example 36

### 5-(4-(4-(2-Chloro-3-methylphenyl)piperazin-1-yl)butoxy)-1,1a,3,7b-tetrahydro-2H-cyclopropa[c]quinolin-2-one

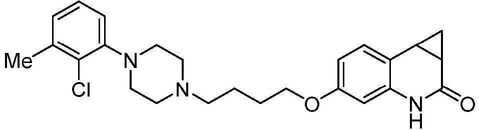

### Synthesis scheme:

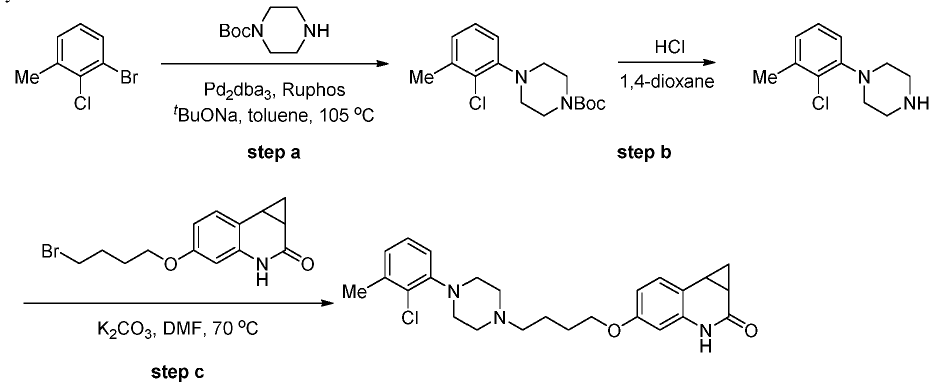

### Step a: Synthesis of tert-butyl 4-(2-chloro-3-methylphenyl)piperazine-1-carboxylate

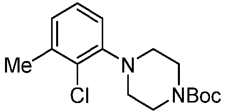

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert-butyl* piperazine-1-carboxylate (838.1 mg, 4.5 mmol) each were added to a dry reaction flask, and then the reaction flask was purged three times with nitrogen in a vacuum pump. Extra-dry toluene (30 mL) and 1-bromo-2-chloro-3-methylbenzene (611.8 mg, 3 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether: ethyl acetate = 10:1) to give the target product (782 mg, 84% yield)

### Step b: Synthesis of 1-(2-chloro-3-methylphenyl)piperazine

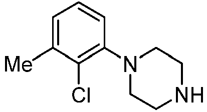

*tert*-Butyl 4-(2-chloro-3-methylphenyl)piperazine-1-carboxylate (782 mg, 2.52 mmol) and a solution of hydrochloric acid in 1,4-dioxane (0.1 M, 20 mL) each were added to a reaction flask, and the mixed solution was reacted at room temperature for 3 h. After the reaction was completed, the reaction solution was concentrated to give crude 1-(2-chloro-3-methylphenyl)piperazine hydrochloride, which was directly used in the next step without purification.

### Step c: Synthesis of 5-(4-(4-(2-chloro-3-methylphenyl)piperazin-1-yl)butoxy)-1,1a,3,7b-tetrahydro-2H-cyclopropa[c]quinolin-2-one

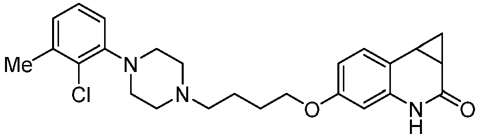

5-(4-Bromobutoxy)-1,1a,3,7b-tetrahydro-2H-cyclopropa[c]quinolin-2-one (231.8 mg, 0.75 mmol), 1-(2-chloro-3-methylphenyl)piperazine hydrochloride (123.0 mg, 0.5 mmol), potassium carbonate (207.3 mg, 1.5 mmol), and *N,N*-dimethylformamide (5 mL) each were added to a reaction flask, and the mixed solution was reacted at 70 °C for 3 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (84 mg, 38% yield).

¹H NMR (600 MHz, CD₃OD): δ 7.28 (d, *J* = 8.4 Hz, 1H), 7.22 (t, *J* = 7.8 Hz, 1H), 7.10 (d, *J* = 7.2 Hz, 1H), 7.06 (d, *J* = 7.8 Hz, 1H), 6.63 (dd, *J* = 8.4, 2.4 Hz, 1H), 6.47 (d, *J* = 2.4 Hz, 1H), 4.05 (t, *J* = 6.0 Hz, 2H), 3.71 (d, *J* = 12.0 Hz, 2H), 3.52 (d, *J =* 13.2 Hz, 2H), 3.36-3.32 (m, 4H), 3.13 (t, *J* = 12.0 Hz, 2H), 2.57-2.54 (m, 1H), 2.40 (s, 3H), 2.08-2.05 (m, 1H), 2.03-1.99 (m, 2H), 1.93-1.88 (m, 2H), 1.67-1.63 (m, 1H), 0.51 (q, *J* = 4.8 Hz, 1H).

MS Found: 440.3[M+H]⁺.

### Example 37

### 5-(4-(4-(3-Chloro-2-methylphenyl)piperazin-1-yl)butoxy)-1,1a,3,7b-tetrahydro-2H-cyclopropa[c]quinolin-2-one

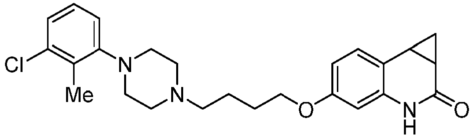

### Synthesis scheme:

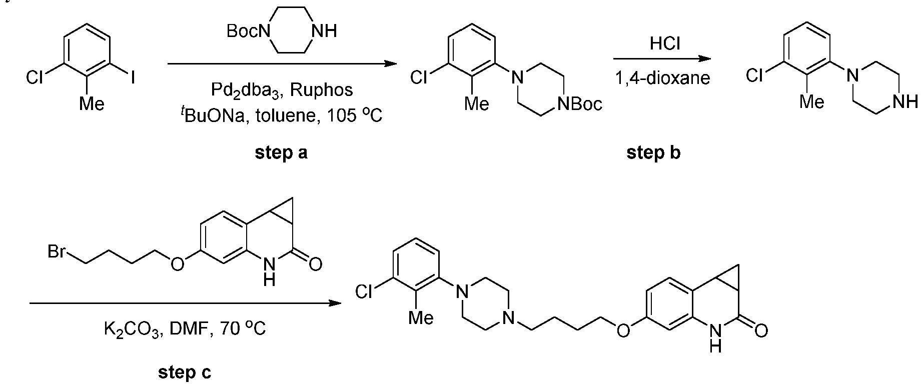

### Step a: Synthesis of tert-butyl 4-(3-chloro-2-methylphenyl)piperazine-1-carboxylate

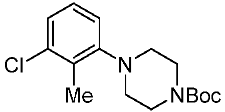

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert-butyl* piperazine-1-carboxylate (838.1 mg, 4.5 mmol) each were added to a dry reaction flask, and then the reaction flask was purged three times with nitrogen in a vacuum pump. Extra-dry toluene (30 mL) and 1-chloro-3-iodo-2-methylbenzene (755.8 mg, 3 mmol) were added under nitrogen atmosphere, and the mixed solution was reacted at 105 °C for 1.5 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether: ethyl acetate = 10: 1) to give the target product (642 mg, 69% yield)

### Step b: Synthesis of 1-(3-chloro-2-methylphenyl)piperazine

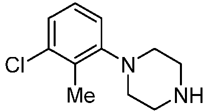

*tert*-Butyl 4-(3-chloro-2-methylphenyl)piperazine-1-carboxylate (642 mg, 2.07 mmol) and a solution of hydrochloric acid in 1,4-dioxane (0.1 M, 20 mL) each were added to a reaction flask, and the mixed solution was reacted at room temperature for 3 h. After the reaction was completed, the reaction solution was concentrated to give crude 1-(3-chloro-2-methylphenyl)piperazine hydrochloride, which was directly used in the next step without purification.

### Step c: Synthesis of 5-(4-(4-(3-chloro-2-methylphenyl)piperazin-1-yl)butoxy)-1,1a,3,7b-tetrahydro-2H-cyclopropalclquinolin-2-one

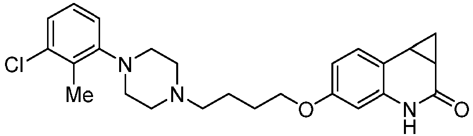

5-(4-Bromobutoxy)-1,1a,3,7b-tetrahydro-2H-cyclopropa[c]quinolin-2-one (185.4 mg, 0.6 mmol), 1-(3-chloro-2-methylphenyl)piperazine hydrochloride (123.0 mg, 0.5 mmol), potassium carbonate (207.3 mg, 1.5 mmol), and *N,N*-dimethylformamide (5 mL) each were added to a reaction flask, and the mixed solution was reacted at 70 °C for 6 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (43 mg, 20% yield).

¹H NMR (600 MHz, CD₃OD): δ 7.26 (d, *J* = 8.4 Hz, 1H), 7.18-7.16 (m, 2H), 7.08-7.05 (m, 1H), 6.60 (dd, *J* = 8.4, 2.4 Hz, 1H), 6.44 (d, *J* = 2.4 Hz, 1H), 4.02 (t, *J* = 6.0 Hz, 2H), 3.67 (d, *J* = 12.0 Hz, 2H), 3.34-3.26 (m, 6H), 3.09 (t, *J* = 12.0 Hz, 2H), 2.55-2.52 (m, 1H), 2.37 (s, 3H), 2.06-2.03 (m, 1H), 2.02-1.96 (m, 2H), 1.90-1.86 (m, 2H), 1.64-1.61 (m, 1H), 0.48 (q, *J* = 4.8 Hz, 1H).

MS Found: 440.3[M+H]⁺.

### Example 38

### 5-(4-(4-(2-Chloro-3-hydroxyphenyl)piperazin-1-yl)butoxy)-1,1a,3,7b-tetrahydro-2H-cyclopropa[c]quinolin-2-one

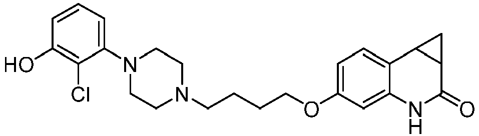

### Synthesis scheme:

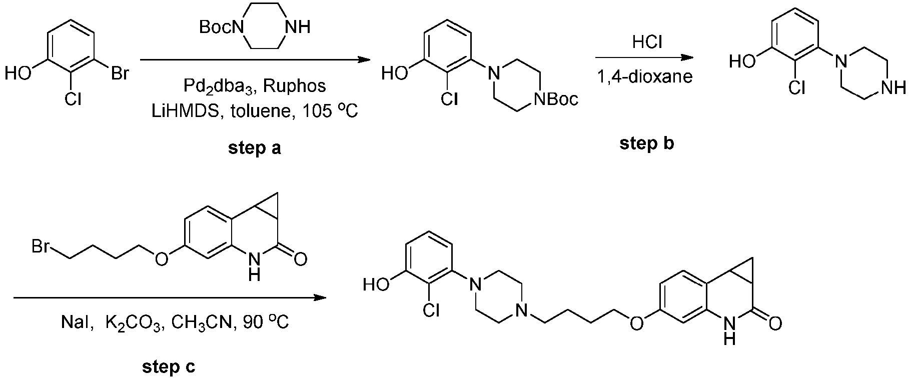

### Step a: Synthesis of tert-butyl 4-(2-chloro-3-hydroxyphenyl)piperazine-1-carboxylate

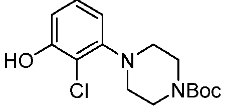

Tris(dibenzylideneacetone)dipalladium(0) (110 mg, 0.12 mmol), 2-dicyclohexylphosphonium-2',6'-diisopropoxy-1,1'-biphenyl (224 mg, 0.48 mmol), and *tert*-butyl piperazine-1-carboxylate (673.3 mg, 3.61 mmol) each were added to a dry reaction flask, and then the reaction flask was purged three times with nitrogen in a vacuum pump. Extra-dry toluene (20 mL), 3-bromo-2-chlorophenol (500 mg, 2.41 mmol), and lithium bis(trimethylsilyl)amide (4.8 mL, 1.0 M in tetrahydrofuran, 4.8 mmol) were added under nitrogen atmosphere, and the mixed solution was reacted at 105 °C for 1 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 5:1) to give the target product (200 mg, 27% yield).

### Step b: Synthesis of 2-chloro-3-(piperazin-1-yl)phenol

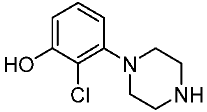

*tert*-Butyl 4-(2-chloro-3-hydroxyphenyl)piperazine-1-carboxylate (200 mg, 0.64 mmol) and a solution of hydrochloric acid in 1,4-dioxane (0.1 M, 6 mL) each were added to a reaction flask, and the mixed solution was reacted at room temperature for 3 h. After the reaction was completed, the reaction solution was concentrated to give crude 2-chloro-3-(piperazine-1-yl)phenol hydrochloride, which was directly used in the next step without purification.

### Step c: Synthesis of 5-(4-(4-(2-chloro-3-hydroxyphenyl)piperazin-1-yl)butoxy)-1,1a,3,7b-tetrahydro-2H-cyclopropa[c]quinolin-2-one

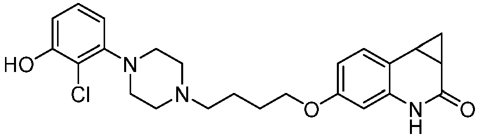

5-(4-Bromobutoxy)-1,1a,3,7b-tetrahydro-2H-cyclopropa[c]quinolin-2-one (86.5 mg, 0.28 mmol), sodium iodide (89.9 mg, 0.6 mmol), and acetonitrile (9 mL) each were added to a reaction flask, and the mixed solution was reacted at 90 °C for 30 min. 2-Chloro-3-(piperazin-1-yl)phenol hydrochloride (104.2 mg, 0.42 mmol) and potassium carbonate (132.7 mg, 0.96 mmol) were then added, and the mixed solution was reacted at 90 °C for another 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (dichloromethane:methanol = 10:1) to give the target product (72 mg, 58% yield).

¹H NMR (600 MHz, CDCl3): δ 8.03 (s, 1H), 7.22 (d, *J* = 8.4 Hz, 1H), 7.11 (t, *J* = 8.4 Hz, 1H), 6.76 (dd, *J* = 7.8, 1.2 Hz, 1H), 6.64 (dd, *J* = 7.8, 1.2 Hz, 1H), 6.54 (dd, *J* = 7.8, 2.4 Hz, 1H), 6.29 (d, *J* = 2.4 Hz, 1H), 3.95 (t, *J* = 6.0 Hz, 2H), 3.12 (brs, 4H), 2.76 (brs, 4H), 2.58 (t, *J* = 8.4 Hz, 2H), 2.48-2.45 (m, 1H), 2.14-2.10 (m, 1H), 1.85-1.72 (m, 4H), 1.60-1.56 (m, 1H), 0.66 (q, *J* = 4.8 Hz, 1H).

MS Found: 442.0[M+H]⁺.

### Example 39

### 5-(4-(4-(3-Chloro-2-hydroxyphenyl)piperazin-1-yl)butoxy)-1,1a,3,7b-tetrahydro-2H-cyclopropa[c]quinolin-2-one

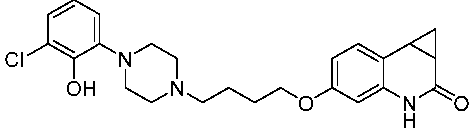

### Synthesis scheme:

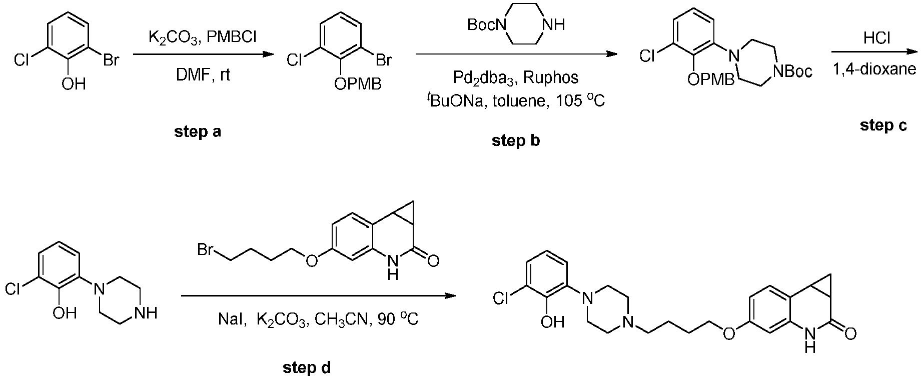

### Step a: Synthesis of 1-bromo-3-chloro-2-((4-methoxybenzyl)oxy)benzene

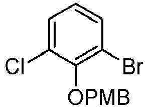

2-Bromo-6-chlorophenol (500 mg, 2.41 mmol), potassium carbonate (449.7 mg, 3.62 mmol), *N,N-*dimethylformamide (20 mL), and 4-methoxybenzyl chloride (0.49 mL, 3.62 mmol) each were added to a reaction flask, and the mixed solution was reacted at room temperature overnight. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 100:1) to give the target product (760 mg, 96% yield)

### Step b: Synthesis of tert-butyl 4-(3-chloro-2-((4-methoxybenzyl)oxy)phenyl)piperazine-1-carboxylate

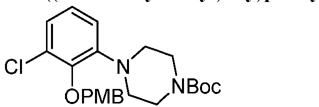

Tris(dibenzylideneacetone)dipalladium(0) (69.9 mg, 0.08 mmol), 2-dicyclohexylphosphonium-2',6'-diisopropoxy-1,1'-biphenyl (142.8 mg, 0.31 mmol), sodium *tert*-butoxide (220.5 mg, 2.30 mmol), *tert*-butyl piperazine-1-carboxylate (428.4 mg, 2.30 mmol), and 1-bromo-3-chloro-2-((4-methoxybenzyl)oxy)benzene (500 mg, 1.53 mmol) each were added to a dry reaction flask, and then the reaction flask was purged three times with nitrogen in a vacuum pump. Extra-dry toluene (30 mL) was added under nitrogen atmosphere, and the mixed solution was reacted at 105 °C for 1 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (360 mg, 54% yield)

### Step c: Synthesis of 2-chloro-6-(piperazin-1-yl)phenol

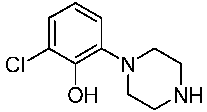

*tert*-Butyl 4-(3-chloro-2-((4-methoxybenzyl)oxy)phenyl)piperazine-1-carboxylate (360 mg, 0.83 mmol) and a solution of hydrochloric acid in 1,4-dioxane (0.1 M, 8 mL) each were added to a reaction flask, and the mixed solution was reacted at room temperature overnight. After the reaction was completed, the reaction solution was concentrated to give crude 2-chloro-6-(piperazine-1-yl)phenol hydrochloride, which was directly used in the next step without purification.

### Step d: Synthesis of 5-(4-(4-(3-chloro-2-hydroxyphenyl)piperazin-1-yl)butoxy)-1,1a,3,7b-tetrahydro-2H-cyclopropa[c]quinolin-2-one

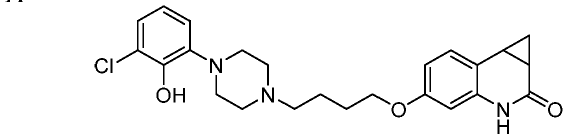

5-(4-Bromobutoxy)-1,1a,3,7b-tetrahydro-2H-cyclopropa[c]quinolin-2-one (80.3 mg, 0.26 mmol), sodium iodide (77.9 mg, 0.52 mmol), and acetonitrile (8 mL) were added to a reaction flask, respectively, and the mixed solution was reacted at 90 °C for 30 min. 2-Chloro-6-(piperazin-1-yl)phenol (94.3 mg, 0.38 mmol) and potassium carbonate (114.7 mg, 0.83 mmol) were then added, and the mixed solution was reacted at 90 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (dichloromethane:methanol = 10:1) to give the target product (114 mg, 99% yield).

¹H NMR (600 MHz, CDCl₃): δ 12.26 (brs, 1H), 8.29 (s, 1H), 7.20 (d, *J* = 8.4 Hz, 1H), 7.14 (d, *J* = 8.4 Hz, 1H), 7.00 (d, *J* = 7.8 Hz, 1H), 6.83 (t, *J* = 7.8 Hz, 1H), 6.50 (dd, *J* = 7.8, 2.4 Hz, 1H), 6.36 (d, *J* = 1.2 Hz, 1H), 3.95 (t, *J* = 6.0 Hz, 2H), 3.75 (d, *J* = 11.4 Hz, 2H), 3.42-3.33 (m, 2H), 3.26-3.16 (m, 6H), 2.46-2.43 (m, 1H), 2.11-2.08 (m, 1H), 2.05-2.00 (m, 2H), 1.87-1.82 (m, 2H), 1.59-1.55 (m, 1H), 0.61 (q, *J* = 4.8 Hz, 1H). MS Found: 442.0[M+H]⁺.

### Example 40

### 5-(4-(4-(2,3-Dichlorophenyl)piperazin-1-yl)butoxy)-4-fluoro-1,1a,3,7b-tetrahydro-2H-cyclopropa[c]quinolin-2-one

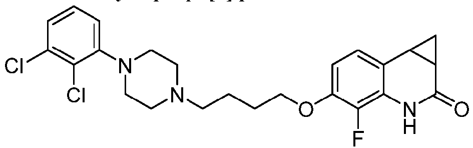

### Synthesis scheme:

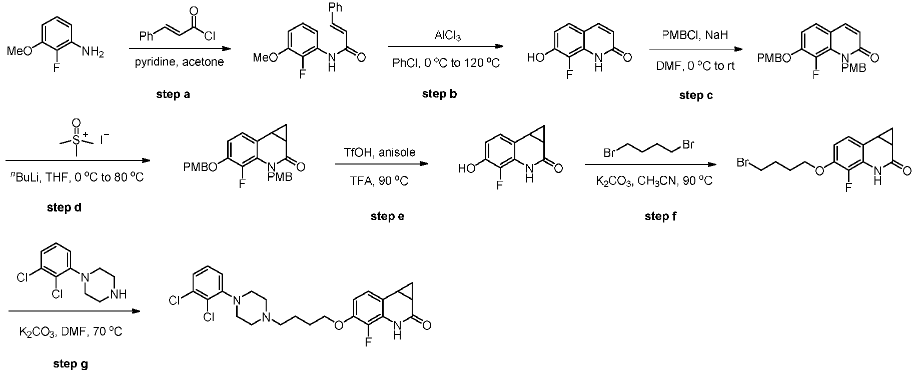

### Step a: Synthesis of N-(2-fluoro-3-methoxyphenyl)cinnamamide

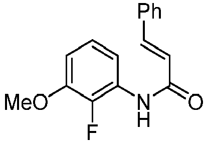

2-Fluoro-3-methoxyaniline (500 mg, 3.54 mmol) and pyridine (0.43 mL, 5.31 mmol) each were added to a reaction flask, and then a solution of cinnamoyl chloride (590 mg, 3.54 mmol) in acetone (15 mL) was slowly added dropwise. The mixed solution was reacted at room temperature for 3 h. After the reaction was completed, the reaction solution was poured into water, and a large amount of solid was precipitated. The mixture was filtered, and the filter cake was washed with water and dried to give the target product (900 mg, 94% yield).

### Step b: Synthesis of 8-fluoro-7-hydroxyquinolin-2(1H)-one

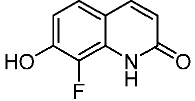

Aluminum trichloride (1.47 g, 11.05 mmol) was added portionwise to a solution of N-(2-fluoro-3-methoxyphenyl)cinnamamide (600 mg, 2.21 mmol) in chlorobenzene (12 mL) at 0 °C, and then the mixed solution was reacted at 120 °C for 3 h. After the reaction was completed, the reaction solution was poured into ice water and extracted with ethyl acetate. The organic phase was washed with saturated brine, dried over anhydrous sodium sulfate, filtered, and concentrated. The crude product was purified by column chromatography (ethyl acetate:petroleum ether = 2:1) to give the target product (100 mg, 25% yield).

### Step c: Synthesis of 8-fluoro-1-(4-methoxybenzyl)-7-((4-methoxybenzyl)oxy)quinolin-2(1H)-one

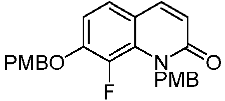

Sodium hydride (34 mg, 1.40 mmol) was added to a solution of 8-fluoro-7-hydroxyquinolin-2(1H)-one (100 mg, 0.56 mmol) in *N,N*-dimethylformamide (6.0 mL) at 0 °C. After the mixed solution was reacted at this temperature for 30 min, 4-methoxybenzyl chloride (0.23 mL, 1.68 mmol) was slowly added dropwise, and then the reaction solution was warmed to room temperature and reacted for 3 h. After the reaction was completed, the reaction was quenched with a saturated aqueous ammonium chloride solution, and the reaction solution was extracted with ethyl acetate. The organic phase was washed with water and saturated brine, dried over anhydrous sodium sulfate, filtered, and concentrated. The crude product was purified by column chromatography (petroleum ether:ethyl acetate = 2:1) to give the target product (135 mg, 57% yield).

### Step d: Synthesis of 4-fluoro-3-(4-methoxybenzyl)-5-((4-methoxybenzyl)oxy)-1,1a,3,7b-tetrahydro-2H-cyclopropa[c]quinolin-2-one

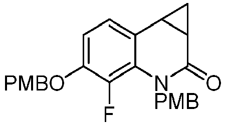

*n*-Butyllithium (0.96 mL, 2.5 M in n-hexane, 2.4 mmol) was slowly added dropwise to a solution of trimethylsulfoxonium iodide (528 mg, 2.4 mmol) in tetrahydrofuran (5 mL) at 0 °C, and the mixed solution was reacted at this temperature for 30 min. A solution of 8-fluoro-1-(4-methoxybenzyl)-7-((4-methoxybenzyl)oxy)quinolin-2(1H)-one (100 mg, 0.24 mmol) in tetrahydrofuran (10 mL) was then added, and the mixed solution was reacted at 80 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 2:1) to give the target product (60 mg, 58% yield).

### Step e: Synthesis of 4-fluoro-5-hydroxy-1,1a,3,7b-tetrahydro-2H-cyclopropa[c]quinolin-2-one

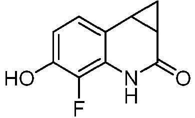

4-Fluoro-3-(4-methoxybenzyl)-5-((4-methoxybenzyl)oxy)-1,1a,3,7b-tetrahydro-2H-cyclopropa[c]quinolin-2-one (59 mg, 0.14 mmol), trifluoroacetic acid (5 mL), anisole (0.5 mL), and trifluoromethanesulfonic acid (0.5 mL) each were added to a reaction flask, and then the mixed solution was reacted at 90 °C for 3 h. After the reaction solution was concentrated, the product was directly purified by column chromatography (petroleum ether:ethyl acetate = 1:2) to give the target product (27 mg, 100% yield).

### Step f: Synthesis of 5-(4-bromobutoxy)-4-fluoro-1,1a,3,7b-tetrahydro-2H-cyclopropa[c]quinolin-2-one

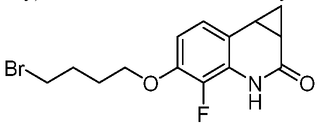

5-Hydroxy-1,1a,3,7b-tetrahydro-2H-cyclopropa[c]quinolin-2-one (135.2 mg, 0.7 mmol), 1,4-dibromobutane (0.25 mL, 2.1 mmol), potassium carbonate (290.2 mg, 2.1 mmol), and acetonitrile (7 mL) each were added to a reaction flask, and the mixed solution was reacted at 90 °C for 3 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 2:1) to give the target product (70 mg, 31% yield).

### Synthesis of tert-butyl 4-(2,3-dichlorophenyl)piperazine-1-carboxylate

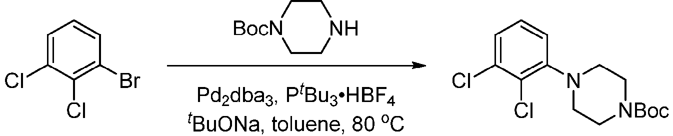

Tris(dibenzylideneacetone)dipalladium(0) (201.3 mg, 0.22 mmol), tri-*tert*-butylphosphine tetrafluoroborate (259.4 mg, 0.89 mmol), sodium *tert*-butoxide (644.8 mg, 6.71 mmol), *tert*-butyl piperazine-1-carboxylate (1.25 g, 6.71 mmol), and 1-bromo-2,3-dichlorobenzene (1 g, 4.47 mmol) each were added to a dry reaction flask, and then the reaction flask was purged three times with nitrogen in a vacuum pump. Extra-dry toluene (45 mL) was added under nitrogen atmosphere, and the mixed solution was reacted at 80 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (500 mg, 34% yield)

### Synthesis of 1-(2,3-dichlorophenyl)piperazine

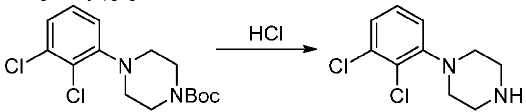

*tert*-Butyl 4-(2,3-dichlorophenyl)piperazine-1-carboxylate (330.1 mg, 1 mmol) and a solution of hydrochloric acid in 1,4-dioxane (0.1 M, 10 mL) each were added to a reaction flask, and the mixed solution was reacted at room temperature for 3 h. After the reaction was completed, the reaction solution was concentrated to give crude 1-(2,3-dichlorophenyl)piperazine hydrochloride, which was directly used in the next step without purification.

### Step g: Synthesis of 5-(4-(4-(2,3-dichlorophenyl)piperazin-1-yl)butoxy)-4-fluoro-1,1a,3,7b-tetrahydro-2H-cyclopropa[c]quinolin-2-one

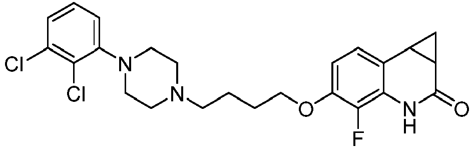

5-(4-Bromobutoxy)-4-fluoro-1,1a,3,7b-tetrahydro-2H-cyclopropa[c]quinolin-2-one (68.9 mg, 0.21 mmol), 1-(2,3-dichlorophenyl)piperazine hydrochloride (50.5 mg, 0.19 mmol), potassium carbonate (87.1 mg, 0.63 mmol), and *N,N-*dimethylformamide (3 mL) were added to a reaction flask, respectively, and the mixed solution was reacted at 70 °C for 6 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 2:5) to give the target product (15 mg, 17% yield).

¹H NMR (600 MHz, CD₃OD): δ 7.32-7.28 (m, 2H), 7.15 (dd, *J* = 7.8, 2.4 Hz, 1H), 7.10 (dd, *J* = 8.4, 1.8 Hz, 1H), 6.75 (t, *J* = 7.8 Hz, 1H), 4.12 (t, *J* = 6.0 Hz, 2H), 3.70 (d, *J* = 12.6 Hz, 2H), 3.54 (d, *J* = 13.2 Hz, 2H), 3.35-3.33 (m, 4H), 3.11 (t*, J* = 12.6 Hz, 2H), 2.60-2.57 (m, 1H), 2.09-2.06 (m, 1H), 2.04-1.99 (m, 2H), 1.94-1.89 (m, 2H), 1.69-1.65 (m, 1H), 0.58 (q, *J* = 4.8 Hz, 1H).

MS Found: 478.0[M+H]⁺.

### Example 41

### 5-(4-(4-(2,2-Difluorobenzo[d][1,3]dioxolan-4-yl)piperazin-1-yl)butoxy)-1,1a,3,7b-tetrahydro-2H-cyclopropa[c]quinolin-2-one

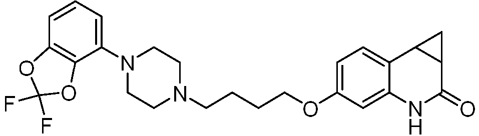

### Synthesis scheme:

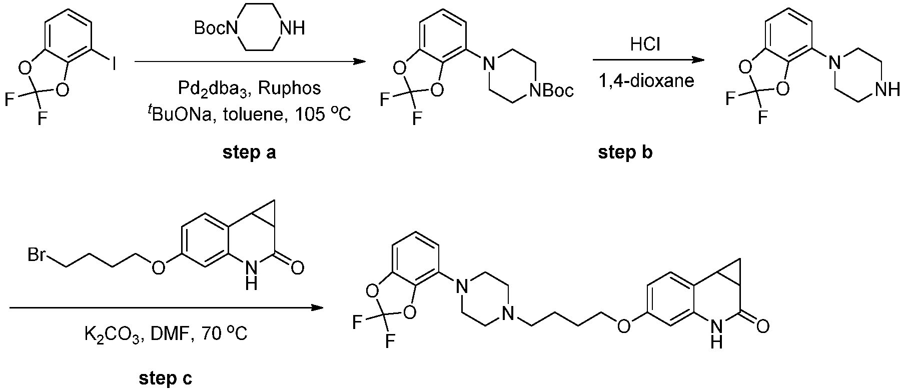

### Step a: Synthesis of tert-butyl 4-(2,2-difluorobenzo[d][1,3]dioxolan-4-yl)piperazine-1-carboxylate

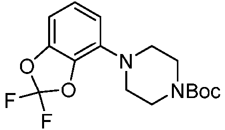

Tris(dibenzylideneacetone)dipalladium(0) (68.6 mg, 0.075 mmol), 2-dicyclohexylphosphonium-2',6'-diisopropoxy-1,1'-biphenyl (139.9 mg, 0.3 mmol), sodium tert-butoxide (216.2 mg, 2.25 mmol), and *tert-butyl* piperazine-1-carboxylate (419.1 mg, 2.25 mmol) each were added to a dry reaction flask, and then the reaction flask was purged three times with nitrogen in a vacuum pump. Extra-dry toluene (15 mL) and 2,2-difluoro-4-iodobenzo[d][1,3]dioxolane (425.9 mg, 1.5 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (462 mg, 90% yield)

### Step b: Synthesis of 1-(2,2-difluorobenzo[d][1,3]dioxolan-4-yl)piperazine

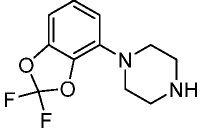

*tert*-Butyl 4-(2,2-difluorobenzo[d][1,3]dioxolan-4-yl)piperazine-1-carboxylate (200 mg, 0.72 mmol) and a solution of hydrochloric acid in 1,4-dioxane (0.1 M, 7 mL) were added to a reaction flask, respectively, and the mixed solution was reacted at room temperature for 3 h. After the reaction was completed, the reaction solution was concentrated to give crude 1-(2,2-difluorobenzo[d][1,3]dioxolan-4-yl)piperazine hydrochloride, which was directly used in the next step without purification.

### Step c: Synthesis of 5-(4-(4-(2,2-difluorobenzo[d][1,3]dioxolan-4-yl)piperazin-1-yl)butoxy)-1,1a,3,7b-tetrahydro-2H-cyclopropa[c]quinolin-2-one

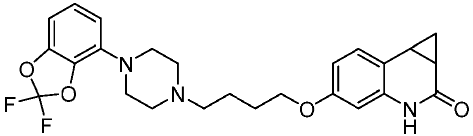

5-(4-Bromobutoxy)-1,1a,3,7b-tetrahydro-2H-cyclopropa[c]quinolin-2-one (268.9 mg, 0.87 mmol), 1-(2,2-difluorobenzo[d][1,3]dioxolan-4-yl)piperazine hydrochloride (161.3 mg, 0.58 mmol), potassium carbonate (240.5 mg, 1.74 mmol), and *N,N*-dimethylformamide (6 mL) each were added to a reaction flask, and the mixed solution was reacted at 70 °C for 8 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (71 mg, 26% yield).

¹H NMR (600 MHz, CD₃OD): δ 7.25 (d, *J* = 8.4 Hz, 1H), 7.11 (t, *J* = 8.4 Hz, 1H), 6.85 (d, *J* = 7.8 Hz, 1H), 6.81 (d, *J* = 8.4 Hz, 1H), 6.60 (dd, *J* = 8.4, 2.4 Hz, 1H), 6.44 (d, *J* = 2.4 Hz, 1H), 4.02 (t, *J* = 6.0 Hz, 2H), 3.85 (d, *J* = 12.0 Hz, 2H), 3.71 (d, *J* = 10.8 Hz, 2H), 3.32-3.29 (m, 4H), 3.21 (t, *J* = 12.6 Hz, 2H), 2.55-2.51 (m, 1H), 2.06-2.02 (m, 1H), 2.01-1.97 (m, 2H), 1.90-1.86 (m, 2H), 1.64-1.60 (m, 1H), 0.49 (q, *J* = 4.8 Hz, 1H).

MS Found: 472.4[M+H]⁺.

The compound in Example 41 was resolved by chiral chromatography to give optical enantiomer 1 and optical enantiomer 2. Liquid chromatography: chromatographic column: DAICEL CHIRALPAK AS column; mobile phase A: supercritical CO₂, mobile phase B: methanol (containing 0.1% dimethylamine); detection wavelength: 214 nm; flow rate: 1.5 mL/min; column temperature: 35 °C; background column pressure: 1800 psi. Mobile phase gradient:

| Time (min) | A (%V/V) | B (%V/V) |
|---|---|---|
| 0.00 | 60 | 40 |
| 8.00 | 60 | 40 |

### Optical enantiomer 1 (Example 41-P1):

RT: 0.80 min; [α]D²⁰ = +60.7 (c 0.102, MeOH); MS Found: 472.6[M+H]⁺.

¹H NMR (400 MHz, CDCl₃): δ 7.96 (s, 1H), 7.22 (d, *J* = 8.4 Hz, 1H), 6.96 (t, *J* = 8.4 Hz, 1H), 6.63 (d, *J* = 7.6 Hz, 1H), 6.57 (d, *J* = 8.4 Hz, 1H), 6.55 (dd, *J* = 8.4, 2.4 Hz, 1H), 6.27 (d, *J* = 2.4 Hz, 1H), 3.96 (t, *J* = 6.0 Hz, 2H), 3.25 (t, *J* = 4.8 Hz, 4H), 2.62 (t, *J* = 4.8 Hz, 4H), 2.49-2.44 (m, 3H), 2.14-2.09 (m, 1H), 1.85-1.78 (m, 2H), 1.73-1.67 (m, 2H), 1.61-1.56 (m, 1H), 0.66 (q, *J* = 4.8 Hz, 1H).

### Optical enantiomer 2 (Example 41-P2):

RT: 1.86 min; [α]D²⁰ = -60.6 (c 0.101, MeOH); MS Found: 472.5[M+H]⁺.

¹H NMR (400 MHz, CDCl₃): δ 8.31 (s, 1H), 7.22 (d, *J* = 8.4 Hz, 1H), 6.96 (t, *J* = 8.4 Hz, 1H), 6.63 (dd, *J* = 8.0, 0.8 Hz, 1H), 6.57 (d, *J* = 8.8 Hz, 1H), 6.55 (dd, *J* = 8.4, 2.4 Hz, 1H), 6.31 (d, *J* = 2.4 Hz, 1H), 3.96 (t, *J* = 6.0 Hz, 2H), 3.25 (t*, J* = 4.8 Hz, 4H), 2.62 (t, *J* = 4.8 Hz, 4H), 2.49-2.44 (m, 3H), 2.15-2.09 (m, 1H), 1.85-1.75 (m, 2H), 1.71-1.66 (m, 2H), 1.61-1.56 (m, 1H), 0.66 (q, *J* = 4.8 Hz, 1H).

### Example 42

### 5-(4-(4-(Benzo[b]thiophen-4-yl)piperazin-1-yl)butoxy)-1,1a,3,7b-tetrahydro-2H-cyclopropa[c]quinolin-2-one

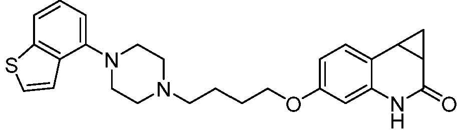

### Synthesis scheme:

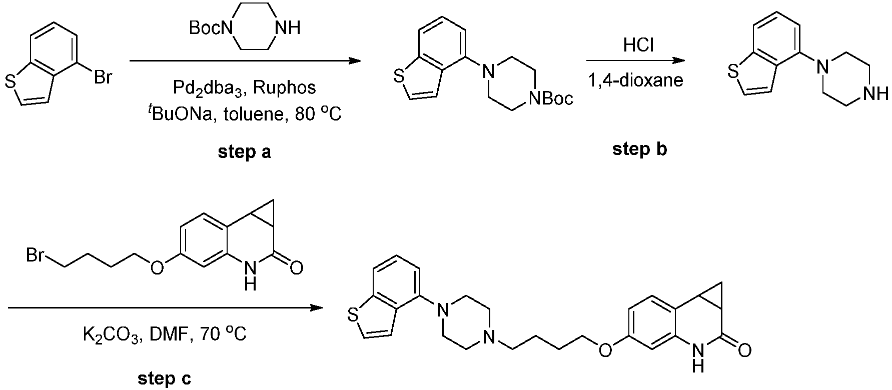

### Step a: Synthesis of tert-butyl 4-(benzo[b]thiophen-4-yl)piperazine-1-carboxylate

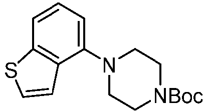

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert-butyl* piperazine-1-carboxylate (838.1 mg, 4.5 mmol) each were added to a dry reaction flask, and then the reaction flask was purged three times with nitrogen in a vacuum pump. Extra-dry toluene (30 mL) and 4-bromobenzo[b]thiophene (635.8 mg, 3 mmol) were added under nitrogen atmosphere, and the mixed solution was reacted at 80 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether: ethyl acetate = 10:1) to give the target product (850 mg, 89% yield)

### Step b: Synthesis of 1-(benzo[b]thiophen-4-yl)piperazine

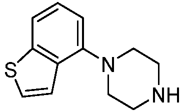

*tert*-Butyl 4-(benzo[b]thiophen-4-yl)piperazine-1-carboxylate (400 mg, 1.26 mmol) and a solution of hydrochloric acid in 1,4-dioxane (0.1 M, 10 mL) each were added to a reaction flask, and the mixed solution was reacted at room temperature for 3 h. After the reaction was completed, the reaction solution was concentrated to give crude 1-(benzo [b]thiophen-4-yl)piperazine hydrochloride, which was directly used in the next step without purification.

### Step c: Synthesis of 5-(4-(4-(benzo[b]thiophen-4-yl)piperazin-1-yl)butoxy)-1,1a,3,7b-tetrahydro-2H-cyclopropa[c]quinolin-2-one

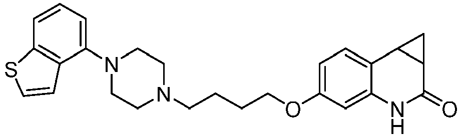

5-(4-Bromobutoxy)-1,1a,3,7b-tetrahydro-2H-cyclopropa[c]quinolin-2-one (231.8 mg, 0.75 mmol), 1-(benzo[b]thiophen-4-yl)piperazine hydrochloride (127.0 mg, 0.5 mmol), potassium carbonate (207.3 mg, 1.5 mmol), and *N,N*-dimethylformamide (5 mL) each were added to a reaction flask, and the mixed solution was reacted at 70 °C for 5 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by reversed-phase column chromatography (acetonitrile:1‰ trifluoroacetic acid in water = 1:3) to give the target product (98 mg, 44% yield).

¹H NMR (600 MHz, CD₃OD): δ 7.61 (d, *J* = 8.4 Hz, 1H), 7.55 (d, *J* = 5.4 Hz, 1H), 7.45 (d, *J* = 6.0 Hz, 1H), 7.28 (t, *J* = 7.8 Hz, 1H), 7.23 (d, *J* = 8.4 Hz, 1H), 6.97 (d, *J* = 7.8 Hz, 1H), 6.58 (dd, *J* = 8.4, 2.4 Hz, 1H), 6.42 (d, *J* = 2.4 Hz, 1H), 4.00 (t, *J* = 6.0 Hz, 2H), 3.69 (d, *J* = 12.0 Hz, 2H), 3.60 (d, *J* = 13.2 Hz, 2H), 3.38 (t, *J* = 11.4 Hz, 2H), 3.31-3.28 (m, 2H), 3.14 (t, *J* = 12.6 Hz, 2H), 2.51-2.47 (m, 1H), 2.02-1.95 (m, 3H), 1.89-1.84 (m, 2H), 1.60-1.56 (m, 1H), 0.45 (q, *J* = 4.8 Hz, 1H).

MS Found: 448.0[M+H]⁺.

The compound in Example 42 was resolved by chiral chromatography to give optical enantiomer 1 and optical enantiomer 2. Liquid chromatography: chromatographic column: DAICEL CHIRALPAK AS column; mobile phase A: supercritical CO₂, mobile phase B: methanol (containing 0.1% dimethylamine); detection wavelength: 214 nm; flow rate: 1.5 mL/min; column temperature: 35 °C; background column pressure: 1800 psi. Mobile phase gradient:

| Time (min) | A (%V/V) | B (%V/V) |
|---|---|---|
| 0.00 | 60 | 40 |
| 8.00 | 60 | 40 |

### Optical enantiomer 1 (Example 42-P1):

RT: 1.64 min; [α]D²⁰ = +60.8 (c 0.102, MeOH); MS Found: 448.5[M+H]⁺.

¹H NMR (400 MHz, CDCl₃): δ 8.08 (s, 1H), 7.55 (d, *J* = 8.0 Hz, 1H), 7.41 (d, *J* = 5.6 Hz, 1H), 7.38 (d, *J* = 5.6 Hz, 1H), 7.27 (t, *J* = 7.6 Hz, 1H), 7.22 (d, *J* = 8.4 Hz, 1H), 6.89 (d, *J* = 7.6 Hz, 1H), 6.56 (dd, *J* = 8.4, 2.4 Hz, 1H), 6.29 (d, *J* = 2.4 Hz, 1H), 3.97 (t, *J* = 6.0 Hz, 2H), 3.19 (brs, 4H), 2.71 (brs, 4H), 2.52 (t, *J* = 7.6 Hz, 2H), 2.50-2.44 (m, 1H), 2.15-2.09 (m, 1H), 1.87-1.80 (m, 2H), 1.76-1.71 (m, 2H), 1.61-1.55 (m, 1H), 0.66 (q, *J* = 4.8 Hz, 1H).

### Optical enantiomer 2 (Example 42-P2):

RT: 4.62 min; [α]D²⁰ = -60.8 (c 0.106, MeOH); MS Found: 448.5[M+H]⁺.

¹H NMR (400 MHz, CDCl₃): δ 8.23 (s, 1H), 7.55 (d, *J* = 8.0 Hz, 1H), 7.41 (d, *J* = 5.6 Hz, 1H), 7.38 (d, *J* = 5.6 Hz, 1H), 7.27 (t, *J* = 8.0 Hz, 1H), 7.22 (d, *J* = 8.4 Hz, 1H), 6.90 (d, *J* = 7.6 Hz, 1H), 6.56 (dd, *J* = 8.4, 2.4 Hz, 1H), 6.31 (d, *J* = 2.4 Hz, 1H), 3.97 (t, *J* = 6.0 Hz, 2H), 3.20 (brs, 4H), 2.73 (brs, 4H), 2.52 (t, *J* = 7.6 Hz, 2H), 2.49-2.43 (m, 1H), 2.14-2.09 (m, 1H), 1.87-1.80 (m, 2H), 1.77-1.70 (m, 2H), 1.61-1.55 (m, 1H), 0.66 (q, *J* = 4.8 Hz, 1H).

### Example 43

### 5-(4-(4-(2,3-Dihydro-1H-inden-4-yl)piperazin-1-yl)butoxy)-1,1a,3,7b-tetrahydro-2H-cyclopropa[c]qiiinolin-2-one

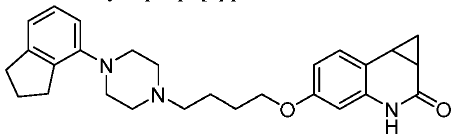

### Synthesis scheme:

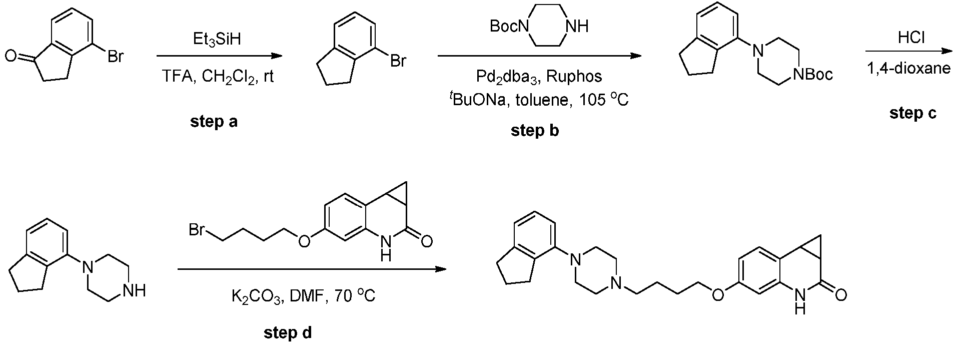

### Step a: Synthesis of 4-bromo-2,3-dihydro-1H-indene

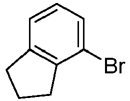

Trifluoroacetic acid (10.8 g, 94.8 mmol) was slowly added dropwise to a solution of 4-bromo-2,3-dihydro-1H-inden-1-one (5 g, 23.7 mmol) in dichloromethane (10 mL), and the reaction solution was cooled to 0 °C. A solution of triethylsilane (8.3 g, 71.1 mmol) in dichloromethane (10 mL) was slowly added dropwise, and the reaction solution returned to room temperature and was reacted at this temperature for 72 h. After the reaction was completed, the reaction solution was concentrated to dryness by rotary evaporation, washed with a saturated aqueous sodium bicarbonate solution, and extracted with ethyl acetate. The organic phase was washed with saturated brine, dried over anhydrous sodium sulfate, filtered, and concentrated. The crude product was purified by column chromatography (petroleum ether) to give the target product (1 g, 15% yield).

### Step b: Synthesis of tert-butyl 4-(2,3-dihydro-1H-inden-4-yl)piperazine-1-carboxylate

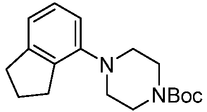

Tris(dibenzylideneacetone)dipalladium(0) (301.9 mg, 0.33 mmol), 2-dicyclohexylphosphonium-2',6'-diisopropoxy-1,1'-biphenyl (615.9 mg, 1.32 mmol), sodium *tert*-butoxide (951.4 mg, 9.9 mmol), and *tert-butyl* piperazine-1-carboxylate (1.84 g, 9.9 mmol) each were added to a dry reaction flask, and then the reaction flask was purged three times with nitrogen in a vacuum pump. Extra-dry toluene (50 mL) and 4-bromo-2,3-dihydro-1H-indene (1.3 g, 6.6 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (1.7 g, 85% yield).

### Step c: Synthesis of 1-(2,3-dihydro-1H-inden-4-yl)piperazine

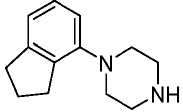

*tert-*Butyl 4-(2,3-dihydro-1H-inden-4-yl)piperazine-1-carboxylate (1.7 g, 5.6 mmol) and a solution of hydrochloric acid in 1,4-dioxane (0.1 M, 30 mL) each were added to a reaction flask, and the mixed solution was reacted at room temperature for 3 h. After the reaction was completed, the reaction solution was concentrated to give crude 1-(2,3-dihydro-1H-inden-4-yl)piperazine hydrochloride, which was directly used in the next step without purification.

### Step d: Synthesis of 5-(4-(4-(2,3-Dihydro-1H-inden-4-yl)piperazin-1-yl)butoxy)-1,1a,3,7b-tetrahydro-2H-cyclopropa[c]quinolin-2-one

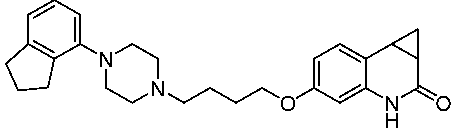

5-(4-Bromobutoxy)-1,1a,3,7b-tetrahydro-2H-cyclopropa[c]quinolin-2-one (1.85 g, 6 mmol), 1-(2,3-dihydro-1H-inden-4-yl)piperazine hydrochloride (1.19 g, 5 mmol), potassium carbonate (2.07 g, 15 mmol), and *N,N-*dimethylformamide (70 mL) each were added to a reaction flask, and the mixed solution was reacted at 70 °C for 5 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 1:3) to give the target product (1.2 g, 55% yield)

¹H NMR (600 MHz, CDCl₃): δ 8.87 (s, 1H), 7.21 (d, *J* = 8.4 Hz, 1H), 7.11 (t*, J* = 7.2 Hz, 1H), 6.92 (d, *J* =7.2 Hz, 1H), 6.73 (d, *J* = 7.8 Hz, 1H), 6.55 (dd, *J* = 8.4, 2.4 Hz, 1H), 6.37 (d, *J* = 2.4 Hz, 1H), 3.96 (t, *J* = 6.0 Hz, 2H), 3.06 (s, 4H), 2.89 (t, *J* = 7.2 Hz, 2H), 2.83 (t, *J* = 7.2 Hz, 2H), 2.65 (s, 4H), 2.50 (t, *J* = 7.8 Hz, 2H), 2.47-2.45 (m, 1H), 2.14-2.10 (m, 1H), 2.07-2.03 (m, 2H), 1.83-1.79 (m, 2H), 1.75-1.70 (m, 2H), 1.60-1.56 (m, 1H), 0.65 (q, *J* = 4.8 Hz, 1H).

MS Found: 432.5[M+H]⁺.

The compound in Example 43 was resolved by chiral chromatography to give optical enantiomer 1 and optical enantiomer 2. Liquid chromatography: chromatographic column: DAICEL CHIRALPAK AS column; mobile phase A: supercritical CO₂, mobile phase B: methanol (containing 0.1% dimethylamine); detection wavelength: 214 nm; flow rate: 1.5 mL/min; column temperature: 35 °C; background column pressure: 1800 psi. Mobile phase gradient:

| Time (min) | A (%V/V) | B (%V/V) |
|---|---|---|
| 0.00 | 60 | 40 |
| 8.00 | 60 | 40 |

### Optical enantiomer 1 (Example 43-P1):

RT: 1.05 min; [α]D²⁰ = +65.5 (c 0.103, MeOH); MS Found: 432.6[M+H]⁺.

¹H NMR (400 MHz, CDCl₃): δ 8.67 (s, 1H), 7.21 (d, *J* = 8.0 Hz, 1H), 7.11 (t, *J* = 7.6 Hz, 1H), 6.91 (d, *J* =7.2 Hz, 1H), 6.73 (d, *J* = 8.0 Hz, 1H), 6.55 (dd, *J* = 8.4, 2.0 Hz, 1H), 6.34 (d, *J* = 1.6 Hz, 1H), 3.96 (t, *J* = 6.0 Hz, 2H), 3.04 (s, 4H), 2.89 (t, *J* = 7.2 Hz, 2H), 2.84 (t, *J* = 7.2 Hz, 2H), 2.62 (s, 4H), 2.49-2.44 (m, 3H), 2.15-2.11 (m, 1H), 2.09-2.01 (m, 2H), 1.85-1.78 (m, 2H), 1.74-1.67 (m, 2H), 1.61-1.55 (m, 1H), 0.66 (q, *J* = 4.8 Hz, 1H).

### Optical enantiomer 2 (Example 43-P2):

RT: 2.72 min; [α]D²⁰ = -63.1 (c 0.110, MeOH); MS Found: 432.6[M+H]⁺.

¹HNMR (400 MHz, CDCl₃): δ 8.56 (s, 1H), 7.21 (d, *J* = 8.4 Hz, 1H), 7.10 (t, *J* = 7.6 Hz, 1H), 6.92 (d, *J* =7.2 Hz, 1H), 6.73 (d, *J* = 8.0 Hz, 1H), 6.55 (dd, *J* = 8.0, 2.4 Hz, 1H), 6.33 (d, *J* = 2.0 Hz, 1H), 3.96 (t, *J* = 6.0 Hz, 2H), 3.06 (t, *J* = 4.0 Hz, 4H), 2.89 (t, *J* = 7.2 Hz, 2H), 2.83 (t, *J* = 7.2 Hz, 2H), 2.65 (s, 4H), 2.52-2.44 (m, 3H), 2.14-2.10 (m, 1H), 2.09-2.01 (m, 2H), 1.84-1.78 (m, 2H), 1.76-1.69 (m, 2H), 1.61-1.55 (m, 1H), 0.66 (q, *J* = 4.8 Hz, 1H).

### Example 44

### 5-(4-(4-(2,3-Dihydrobenzofuran-7-yl)piperazin-1-yl)butoxy)-1,1a,3,7b-tetrahydro-2H-cyclopropa[c]quinolin-2-one

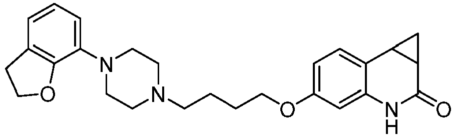

### Synthesis scheme:

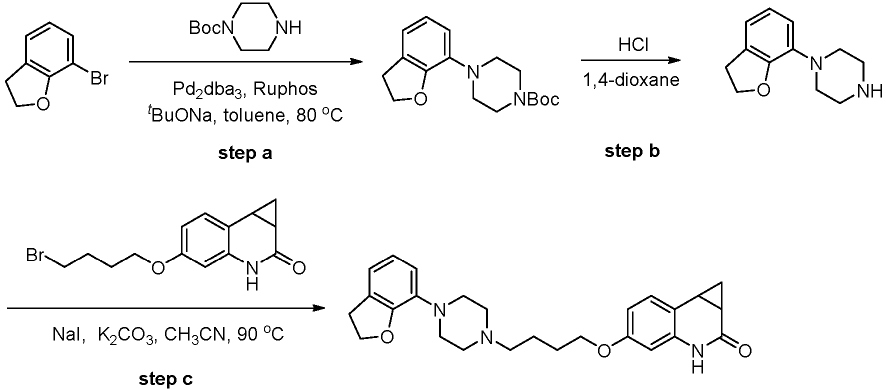

### Step a: Synthesis of tert-butyl 4-(2,3-dihydrobenzofuran-7-yl)piperazine-1-carboxylate

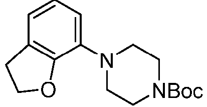

Tris(dibenzylideneacetone)dipalladium(0) (345 mg, 0.38 mmol), 2-dicyclohexylphosphonium-2',6'-diisopropoxy-1,1'-biphenyl (704.6 mg, 1.51 mmol), sodium *tert*-butoxide (1.09 g, 11.31 mmol), and *tert*-butyl piperazine-1-carboxylate (2.11 g, 11.31 mmol) each were added to a dry reaction flask, and then the reaction flask was purged three times with nitrogen in a vacuum pump. Extra-dry toluene (50 mL) and 7-bromo-2,3-dihydrobenzofuran (1.5 g, 7.54 mmol) were added under nitrogen atmosphere, and the mixed solution was reacted at 80 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (2.3 g, 100% yield)

### Step b: Synthesis of 1-(2,3-dihydrobenzofuran-7-yl)piperazine

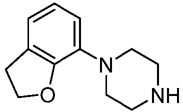

*tert*-Butyl 4-(2,3-dihydrobenzofuran-7-yl)piperazine-1-carboxylate (1.7 g, 5.6 mmol) and a solution of hydrochloric acid in 1,4-dioxane (0.1 M, 30 mL) each were added to a reaction flask, and the mixed solution was reacted at room temperature for 3 h. After the reaction was completed, the reaction solution was concentrated to give crude 1-(2,3-dihydrobenzofuran-7-yl)piperazine hydrochloride, which was directly used in the next step without purification.

### Step c: Synthesis of 5-(4-(4-(2,3-dihydrobenzofuran-7-yl)piperazin-1-yl)butoxy)-1,1a,3,7b-tetrahydro-2H-cyclopropa[c]quinolin-2-one

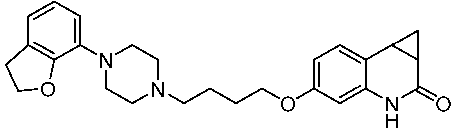

5-(4-Bromobutoxy)-1,1a,3,7b-tetrahydro-2H-cyclopropa[c]quinolin-2-one (1.10 g, 3.55 mmol), sodium iodide (1.06 g, 7.10 mmol), and acetonitrile (30 mL) each were added to a reaction flask, and the mixed solution was reacted at 90 °C for 30 min. 1-(2,3-Dihydrobenzofuran-7-yl)piperazine hydrochloride (938.9 mg, 3.91 mmol) and potassium carbonate (1.08 g, 7.81 mmol) were then added, and the mixed solution was reacted at 90 °C for another 4.5 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (dichloromethane:methanol = 20:1) to give the target product (1.17 g, 76% yield).

¹H NMR (600 MHz, CDCl₃): δ 8.38 (s, 1H), 7.21 (d, *J* = 8.4 Hz, 1H), 6.86 (d, *J* = 7.2 Hz, 1H), 6.80 (t, *J* = 7.2 Hz, 1H), 6.70 (d, *J* = 7.8 Hz, 1H), 6.54 (dd, *J* = 8.4, 2.4 Hz, 1H), 6.32 (d, *J* = 1.8 Hz, 1H), 4.59 (t, *J* = 8.4 Hz, 2H), 3.95 (t, *J* = 6.0 Hz, 2H), 3.19 (t, *J* = 8.4 Hz, 6H), 2.70 (s, 4H), 2.51 (t, *J* = 7.2 Hz, 2H), 2.48-2.44 (m, 1H), 2.13-2.09 (m, 1H), 1.83-1.78 (m, 2H), 1.76-1.71 (m, 2H), 1.60-1.56 (m, 1H), 0.65 (q, *J* = 4.8 Hz, 1H).

MS Found: 434.5[M+H]⁺.

The compound in Example 44 was resolved by chiral chromatography to give optical enantiomer 1 and optical enantiomer 2. Liquid chromatography: chromatographic column: DAICEL CHIRALPAK AS column; mobile phase A: supercritical CO₂, mobile phase B: methanol (containing 0.1% dimethylamine); detection wavelength: 214 nm; flow rate: 1.5 mL/min; column temperature: 35 °C; background column pressure: 1800 psi. Mobile phase gradient:

| Time (min) | A (%V/V) | B (%V/V) |
|---|---|---|
| 0.00 | 60 | 40 |
| 8.00 | 60 | 40 |

### Optical enantiomer 1 (Example 44-P1):

RT: 1.75 min; [α]D²⁰ = +74.0 (c 0.088, CHCl₃); MS Found: 434.6[M+H]⁺.

¹H NMR (400 MHz, CDCl₃): δ 7.98 (s, 1H), 7.21 (d, *J* = 8.4 Hz, 1H), 6.86 (d, *J* = 7.2 Hz, 1H), 6.80 (t, *J* = 7.6 Hz, 1H), 6.70 (d, *J* = 7.6 Hz, 1H), 6.54 (dd, *J* = 8.0, 2.0 Hz, 1H), 6.27 (d, *J* = 2.4 Hz, 1H), 4.59 (t, *J* = 8.8 Hz, 2H), 3.95 (t*, J* = 6.0 Hz, 2H), 3.20 (t, *J =* 8.8 Hz, 2H), 3.17 (s, 4H), 2.67 (s, 4H), 2.51-2.44 (m, 3H), 2.14-2.08 (m, 1H), 1.84-1.78 (m, 2H), 1.75-1.68 (m, 2H), 1.61-1.55 (m, 1H), 0.66 (q, *J* = 4.8 Hz, 1H).

### Optical enantiomer 2 (Example 44-P2):

RT: 5.22 min; [α]D²⁰ = -72.3 (c 0.086, CHCl₃); MS Found: 434.6[M+H]⁺.

¹H NMR (400 MHz, CDCl₃): δ 7.73 (s, 1H), 7.22 (d, *J* = 8.4 Hz, 1H), 6.86 (d, *J* = 7.2 Hz, 1H), 6.80 (t, *J* = 7.2 Hz, 1H), 6.70 (d, *J* = 7.6 Hz, 1H), 6.55 (dd, *J* = 8.0, 2.0 Hz, 1H), 6.25 (d, *J* = 2.0 Hz, 1H), 4.59 (t, *J* = 8.8 Hz, 2H), 3.95 (t, *J* = 6.0 Hz, 2H), 3.20 (t, *J* = 8.8 Hz, 2H), 3.16 (s, 4H), 2.64 (s, 4H), 2.49-2.44 (m, 3H), 2.14-2.09 (m, 1H), 1.82-1.77 (m, 2H), 1.73-1.66 (m, 2H), 1.61-1.55 (m, 1H), 0.66 (q, *J* = 4.8 Hz, 1H).

### Example 45

### 5-(4-(4-(2,3-Dihydrobenzofuran-4-yl)piperazin-1-yl)butoxy)-1,1a,3,7b-tetrahydro-2H-cyclopropa[c]quinolin-2-one

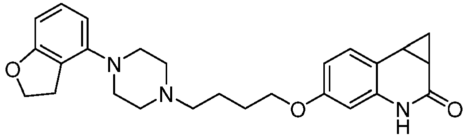

### Synthesis scheme:

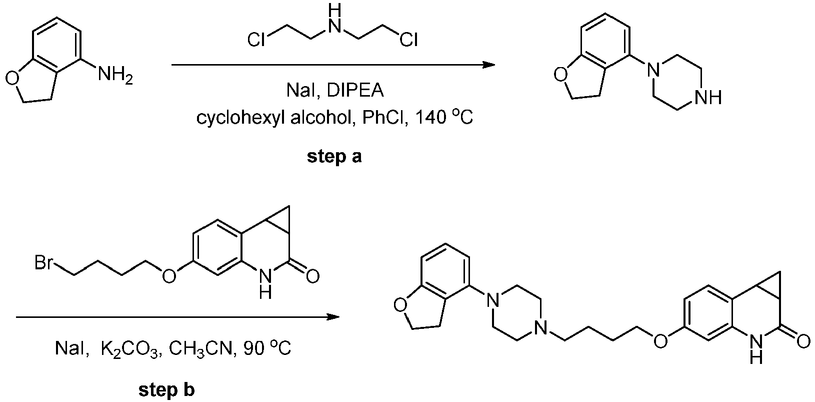

### Step a: Synthesis of 1-(2,3-dihydrobenzofuran-4-yl)piperazine

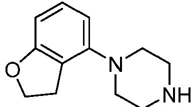

2,3-Dihydrobenzofuran-4-amine (1 g, 7.40 mmol), chlorobenzene (18 mL), cyclohexyl alcohol (0.8 mL), and *N,N*-diisopropylethylamine (690 mg, 5.33 mmol) were added to a reaction flask, and the reaction solution was reacted at room temperature for 10 min. Bis(2-chloroethyl)amine hydrochloride (1.31 g, 7.40 mmol) and sodium iodide (352 mg, 2.35 mmol) were then added, and the mixed solution was reacted at 140 °C for 9 h. After the reaction was completed, the reaction solution was concentrated to dryness by rotary evaporation, the crude product was diluted with dichloromethane, and a large amount of solid was precipitated. The mixture was filtered, and the filter cake was washed with dichloromethane and petroleum ether and dried to give the target product (1.3 g, 73% yield).

### Step b: Synthesis of 5-(4-(4-(2,3-dihydrobenzofuran-4-yl)piperazin-1-yl)butoxy)-1,1a,3,7b-tetrahydro-2H-cylopropa[c]quinolin-2-one

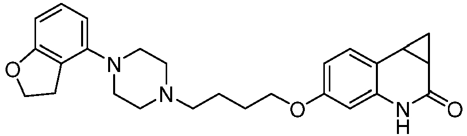

5-(4-Bromobutoxy)-1,1a,3,7b-tetrahydro-2H-cyclopropa[c]quinolin-2-one (1 g, 3.21 mmol), sodium iodide (969.8 mg, 6.47 mmol), and acetonitrile (50 mL) were added to a reaction flask, respectively, and the mixed solution was reacted at 90 °C for 30 min. 1-(2,3-Dihydrobenzofuran-4-yl)piperazine hydrochloride (1.19 g, 4.94 mmol) and potassium carbonate (982.7 mg, 7.11 mmol) were then added, and the mixed solution was reacted at 90 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (dichloromethane:methanol = 20:1) to give the target product (1.08 g, 78% yield).

¹H NMR (600 MHz, CDCl₃): δ 8.41 (s, 1H), 7.22 (d, *J* = 8.4 Hz, 1H), 7.06 (t, *J* = 8.4 Hz, 1H), 6.55 (dd, *J* = 8.4, 1.2 Hz, 1H), 6.50 (d, *J* = 7.8 Hz, 1H), 6.45 (d, *J* = 7.8 Hz, 1H), 6.32 (d, *J* = 1.2 Hz, 1H), 4.54 (t, *J* = 8.4 Hz, 2H), 3.96 (t, *J* = 6.0 Hz, 2H), 3.13 (t, *J* = 8.4 Hz, 2H), 3.09 (t, *J* = 4.8 Hz, 4H), 2.62 (s, 4H), 2.50-2.45 (m, 3H), 2.14-2.10 (m, 1H), 1.84-1.79 (m, 2H), 1.74-1.69 (m, 2H), 1.60-1.56 (m, 1H), 0.66 (q, *J* = 4.8 Hz, 1H).

MS Found: 434.5[M+H]⁺.

The compound in Example 45 was resolved by chiral chromatography to give optical enantiomer 1 and optical enantiomer 2. Liquid chromatography: chromatographic column: DAICEL CHIRALPAK AS column; mobile phase A: supercritical CO₂, mobile phase B: methanol (containing 0.1% dimethylamine); detection wavelength: 214 nm; flow rate: 1.5 mL/min; column temperature: 35 °C; background column pressure: 1800 psi. Mobile phase gradient:

| Time (min) | A (%V/V) | B (%V/V) |
|---|---|---|
| 0.00 | 60 | 40 |
| 10.00 | 60 | 40 |

### Optical enantiomer 1 (Example 45-P1):

RT: 2.25 min; [α]D²⁰ = +67.2 (c 0.097, MeOH); MS Found: 434.6[M+H]⁺.

¹H NMR (400 MHz, CDCl₃): δ 8.08 (s, 1H), 7.22 (d, *J* = 8.4 Hz, 1H), 7.06 (t, *J* = 8.0 Hz, 1H), 6.55 (dd, *J* = 8.4, 2.4 Hz, 1H), 6.49 (d, *J* = 7.6 Hz, 1H), 6.45 (d, *J* = 8.0 Hz, 1H), 6.28 (d, *J* = 2.4 Hz, 1H), 4.54 (t, *J* = 8.4 Hz, 2H), 3.95 (t, *J* = 6.0 Hz, 2H), 3.13 (t, *J* = 8.4 Hz, 2H), 3.07 (t, *J* = 4.8 Hz, 4H), 2.60 (t, *J* = 4.4 Hz, 4H), 2.49-2.44 (m, 3H), 2.14-2.09 (m, 1H), 1.84-1.78 (m, 2H), 1.73-1.66 (m, 2H), 1.61-1.56 (m, 1H), 0.66 (q, *J* = 4.8 Hz, 1H).

### Optical enantiomer 2 (Example 45-P2):

RT: 7.94 min; [α]D²⁰ = -65.7 (c 0.102, MeOH); MS Found: 434.6[M+H]⁺.

¹H NMR (400 MHz, CDCl₃): δ 8.00 (s, 1H), 7.22 (d, *J* = 8.4 Hz, 1H), 7.06 (t, *J* = 8.0 Hz, 1H), 6.55 (dd, *J =* 8.4, 2.4 Hz, 1H), 6.50 (d, *J* = 7.6 Hz, 1H), 6.45 (d, *J* = 8.0 Hz, 1H), 6.28 (d, *J* = 2.4 Hz, 1H), 4.54 (t, *J* = 8.4 Hz, 2H), 3.96 (t, *J* = 6.0 Hz, 2H), 3.13 (t, *J* = 8.8 Hz, 2H), 3.10 (t, *J* = 4.8 Hz, 4H), 2.64 (t, *J* = 4.4 Hz, 4H), 2.52-2.44 (m, 3H), 2.14-2.09 (m, 1H), 1.83-1.78 (m, 2H), 1.76-1.69 (m, 2H), 1.61-1.56 (m, 1H), 0.66 (q, *J* = 4.8 Hz, 1H).

### Example 46

### 5-(4-(4-(2,2-Difluoro-2,3-dihydro-1H-inden-4-yl)piperazin-1-yl)butoxy)-1,1a,3,7b-tetrahydro-2H-cyclopropa[c]quinolin-2-one

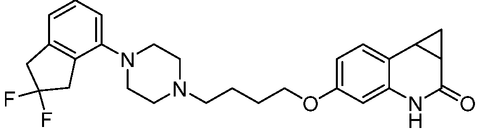

### Synthesis scheme:

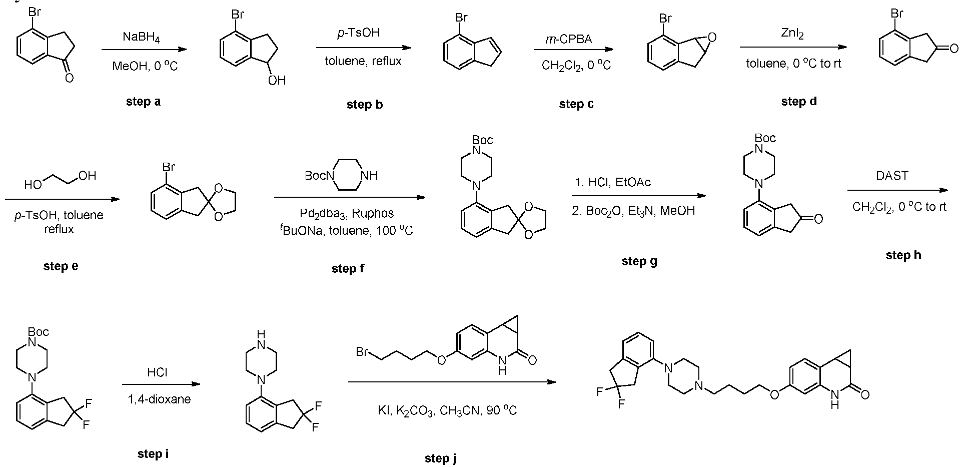

### Step a: Synthesis of 4-bromo-2,3-dihydro-1H-inden-1-ol

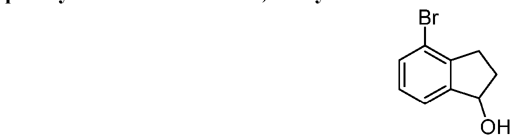

4-Bromo-1-indanone (8.0 g, 37.9 mmol) was added to methanol (80 mL), and the mixed solution was cooled to 0 °C under nitrogen atmosphere. Sodium borohydride (1.4 g, 37.9 mmol) was then added portionwise, and the mixed solution was stirred for 1 h. The reaction solution was slowly poured into ice diluted hydrochloric acid, and a solid was precipitated. The mixture was filtered, and the resulting filter cake was washed with water and dried to give the target product (7.4 g, 92% yield).

### Step b: Synthesis of 4-bromo-1H-indene

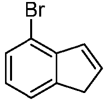

4-Bromo-2,3-dihydro-1H-inden-1-ol (7.4 g, 34.7 mmol) was added to toluene (50 mL),p-toluenesulfonic acid (1.3 g, 6.9 mmol) was added, and the mixed solution was stirred at reflux for 2 h. The reaction solution was poured into a saturated aqueous sodium bicarbonate solution and extracted with ethyl acetate. The organic phase was washed with saturated brine, dried over anhydrous sodium sulfate, filtered, and concentrated. The ethyl acetate layer was dried, filtered, and concentrated. The crude product was purified by column chromatography (petroleum ether) to give the target product (5.8 g, 86% yield).

### Step c: Synthesis of 2-bromo-1a,6a-dihydro-6H-indeno[1,2-b]oxirane

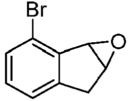

4-Bromo-1H-indene (5.8 g, 29.7 mmol) and sodium bicarbonate (7.7 g, 44.6 mmol) were added sequentially to dichloromethane (60 mL), and the mixed solution was cooled to 0 °C under nitrogen atmosphere. m-Chloroperoxybenzoic acid (3.8 g, 44.6 mmol) was added portionwise, and the mixed solution was stirred for 12 h. A saturated aqueous sodium thiosulfate solution was added to the reaction solution, and the mixed solution was stirred for half an hour. Liquid separation was performed to give a dichloromethane layer. The dichloromethane layer was dried and concentrated to give a crude product, which was directly used in the next step without purification.

### Step d: Synthesis of 4-bromo-1,3-dihydro-2H-inden-2-one

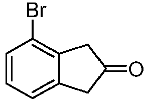

Zinc iodide (8.7 g, 27.4 mmol) was added to anhydrous toluene (100 mL), and the mixed solution was cooled to 0 °C under nitrogen atmosphere. A solution of 2-bromo-1a,6a-dihydro-6H-indeno[1,2-b]oxirane (4.3 g, crude) in toluene was added slowly and the mixed solution was stirred for 12 h. The reaction solution was poured into a saturated aqueous sodium bicarbonate solution and extracted with ethyl acetate. The organic phase was washed with saturated brine, dried over anhydrous sodium sulfate, filtered, and concentrated. The crude product was purified by column chromatography (petroleum ether:ethyl acetate = 20:1) to give the target product (2.2 g, 28% yield).

### Step e: Synthesis of 4-bromo-1,3-dihydrospiro[indene-2,2'-[1,3]dioxolane]

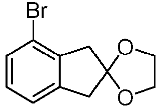

4-Bromo-1,3-dihydro-2H-inden-2-one (2.2 g, 10.4 mmol), ethylene glycol (1.3 g, 20.8 mmol), and *p-*toluenesulfonic acid (0.2 g, 1.0 mmol) were added sequentially to anhydrous toluene (20 mL). The mixed solution was stirred at reflux for 2 h, and water was removed with a water separator. The reaction solution was poured into a saturated aqueous sodium bicarbonate solution and extracted with ethyl acetate. The organic phase was washed with saturated brine, dried over anhydrous sodium sulfate, filtered, and concentrated. The crude product obtained after the organic phase was concentrated to dryness by rotary evaporation was directly used in the next step without purification.

### Step f: Synthesis of tert-butyl 4-(1,3-dihydrospiro[indene-2,2'-[1,3]dioxolan]-4-yl)piperazine-1-carboxylate

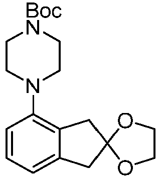

4-Bromo-1,3-dihydrospiro[indene-2,2'-[1,3]dioxolane] (2.3 g, crude) was dissolved in toluene (20 mL), *tert-*butyl piperazine-1-carboxylate (2.3 g, 12.5 mmol), tris(dibenzylideneacetone)dipalladium(0) (0.96 g, 1.0 mmol), 2-dicyclohexylphosphonium-2',6'-diisopropoxy-1,1'-biphenyl (0.97 g, 2.1 mmol), and sodium *tert-*butoxide (1.9 g, 20.0 mmol) were added sequentially, and the mixed solution was stirred at 100 °C for 4 h under nitrogen atmosphere. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether: ethyl acetate = 10:1) to give the target product (3.4 g, 91% yield)

### Step g: Synthesis of tert-butyl 4-(2-one-2,3-dihydro-1H-inden-4-yl)piperazine-1-carboxylate

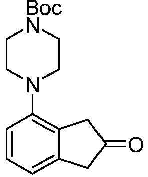

*tert*-Butyl 4-(1,3-dihydrospiro[indene-2,2'-[1,3]dioxolan]-4-yl)piperazine-1-carboxylate (3.4 g, 9.4 mmol) was added to a solution of ethyl acetate in hydrochloric acid (0.1 M, 20 mL), and the mixed solution was stirred for 2 h and concentrated to give a solid that was dissolved in methanol (20 mL). Di-tert-butyl dicarbonate (2.5 g, 11.3 mmol) was added, and triethylamine (1.9 g, 18.9 mmol) was slowly added under an ice bath. The mixed solution was stirred for half an hour. The reaction solution was concentrated, and the resulting crude product was purified by column chromatography (petroleum ether:ethyl acetate = 5:1) to give the target product (2.6 g, 87% yield).

### Step h: Synthesis of tert-butyl 4-(2,2-difluoro-2,3-dihydro-1H-inden-4-yl)piperazine-1-carboxylate

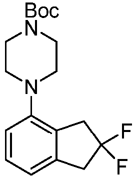

*tert*-Butyl 4-(2-one-2,3-dihydro-1H-inden-4-yl)piperazine-1-carboxylate (2.6 g, 8.2 mmol) was added to dichloromethane (40 mL) and cooled to 0 °C under nitrogen atmosphere. Diethylaminosulfur trifluoride (6.6 g, 41.1 mmol) was slowly added dropwise, and the mixed solution was stirred for 12 h. The reaction solution was slowly poured into a saturated aqueous sodium bicarbonate solution and extracted with dichloromethane. The organic phase was washed with saturated brine, dried over anhydrous sodium sulfate, filtered, and concentrated. The crude product was purified by column chromatography (petroleum ether:ethyl acetate = 10:1) to give the target product (630 mg, 23% yield).

### Step i: Synthesis of 4-(2,2-difluoro-2,3-dihydro-1H-inden-4-yl)piperazine hydrochloride

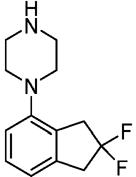

*tert*-Butyl 4-(2,2-difluoro-2,3-dihydro-1H-inden-4-yl)piperazine-1-carboxylate (1.1 g, 3.2 mmol) was added to a solution of hydrochloric acid in 1,4-dioxane (0.1 M, 15 mL), and the mixed solution was stirred for 2 h and concentrated to give crude 4-(2,2-difluoro-2,3-dihydro-1H-inden-4-yl)piperazine hydrochloride, which was directly used in the next step without purification.

### Step j: Synthesis of 5-(4-(4-(2,2-difluoro-2,3-dihydro-1H-inden-4-yl)piperazin-1-yl)butoxy)-1,1a,3,7b-tetrahydro-2H-cyclopropa[c]quinolin-2-one

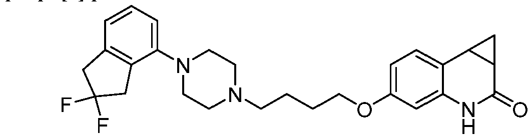

5-(4-Bromobutoxy)-1,1a,3,7b-tetrahydro-2H-cyclopropa[c]quinolin-2-one (1.1 g, 3.5 mmol) was dissolved in anhydrous acetonitrile (15 mL), potassium iodide (524.6 mg, 3.5 mmol) was added, and the mixed solution was stirred at 90 °C for half an hour. Potassium carbonate (981.3 mg, 7.1 mmol) and 4-(2,2-difluoro-2,3-dihydro-1H-inden-4-yl)piperazine hydrochloride (780 mg, 3.4 mmol) each were then added to the reaction system, and the reaction system was stirred at 90 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (dichloromethane:methanol = 20:1) to give the target product (1.1 g, 66% yield).

¹H NMR (600 MHz, *d₆*-DMSO): δ 9.82 (s, 1H), 7.23 (d, *J* = 10.2 Hz, 1H), 7.18 (t, *J* = 9.6 Hz, 1H), 6.90 (d, *J* = 8.4 Hz, 1H), 6.81 (d, *J* = 9.6 Hz, 1H), 6.51 (dd, *J* = 9.6, 2.4 Hz, 1H), 6.43 (d, *J* = 2.4 Hz, 1H), 3.91 (t*, J* = 7.8 Hz, 2H), 3.40 (t, *J* = 18.0 Hz, 4H), 3.34 (t, *J* = 17.4 Hz, 4H), 2.91 (brs, 4H), 2.48-2.43 (m, 1H), 2.43-2.37 (m, 2H), 1.96-1.92 (m, 1H), 1.75-1.69 (m, 2H), 1.59 (t, *J* = 8.4 Hz, 2H), 1.53-1.48 (m, 1H), 0.43 (q, *J* = 5.4 Hz, 1H).

MS Found: 468.3[M+H]⁺.

The compound in Example 46 was resolved by chiral chromatography to give optical enantiomer 1 and optical enantiomer 2. Liquid chromatography: chromatographic column: DAICEL CHIRALPAK AS column; mobile phase A: supercritical CO₂, mobile phase B: methanol (containing 0.1% dimethylamine); detection wavelength: 214 nm; flow rate: 1.5 mL/min; column temperature: 35 °C; background column pressure: 1800 psi. Mobile phase gradient:

| Time (min) | A (%V/V) | B (%V/V) |
|---|---|---|
| 0.00 | 60 | 40 |
| 10.00 | 60 | 40 |

### Optical enantiomer 1 (Example 46-P1):

RT: 1.24 min; [α]D²⁰ = +58.4 (c 0.106, MeOH); MS Found: 468.6[M+H]⁺.

¹H NMR (400 MHz, CDCl₃): δ 8.05 (s, 1H), 7.22 (d, *J* = 8.4 Hz, 1H), 7.20 (t, *J* = 8.0 Hz, 1H), 6.88 (d, *J* = 7.6 Hz, 1H), 6.83 (d, *J* = 8.0 Hz, 1H), 6.55 (dd, *J* = 8.4, 2.4 Hz, 1H), 6.29 (d, *J* = 2.0 Hz, 1H), 3.96 (t, *J* = 6.4 Hz, 2H), 3.37 (dt, *J* = 22.0, 14.4 Hz, 4H), 3.00 (t, *J* = 4.8 Hz, 4H), 2.60 (brs, 4H), 2.49-2.44 (m, 3H), 2.14-2.09 (m, 1H), 1.85-1.78 (m, 2H), 1.73-1.66 (m, 2H), 1.61-1.56 (m, 1H), 0.43 (q, *J* = 4.8 Hz, 1H).

### Optical enantiomer 2 (Example 46-P2):

RT: 4.02 min; [α]D²⁰ = -60.1 (c 0.101, MeOH); MS Found: 468.6[M+H]⁺.

¹H NMR (400 MHz, CDCl₃): δ 8.25 (s, 1H), 7.22 (d, *J* = 8.0 Hz, 1H), 7.19 (t, *J* = 7.6 Hz, 1H), 6.88 (d, *J* = 7.2 Hz, 1H), 6.82 (d, *J* = 8.0 Hz, 1H), 6.55 (dd, *J* = 8.4, 2.0 Hz, 1H), 6.30 (d, *J* = 2.0 Hz, 1H), 3.96 (t, *J* = 6.4 Hz, 2H), 3.37 (dt, *J* = 22.0, 14.4 Hz, 4H), 3.00 (t, *J* = 4.8 Hz, 4H), 2.60 (brs, 4H), 2.49-2.44 (m, 3H), 2.14-2.09 (m, 1H), 1.85-1.78 (m, 2H), 1.74-1.66 (m, 2H), 1.61-1.56 (m, 1H), 0.66 (q, *J* = 4.8 Hz, 1H).

### Example 47

### Synthesis of 5-(4-(4-(2,2-difluoro-1-one-2,3-dihydro-1H-inden-4-yl)piperazin-1-yl)butoxy)-1,1a,3,7b-tetrahydro-2H-cyclopropa[c]quinolin-2-one

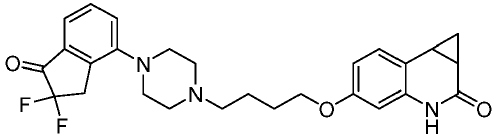

### Synthesis scheme:

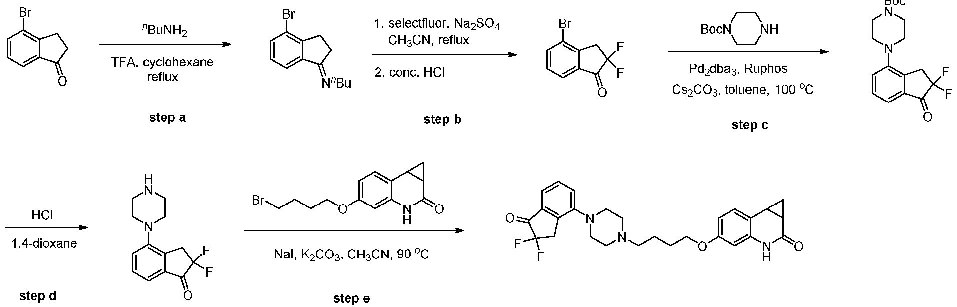

### Step a: Synthesis of 4-bromo-N-butyl-2,3-dihydro-1H-inden-1-imine

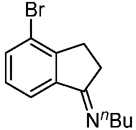

4-Bromo-1-indanone (5.0 g, 23.7 mmol) and n-butylamine (3.5 g, 47.4 mmol) were dissolved in cyclohexane (50 mL), 5 drops of trifluoroacetic acid was added, and then the reaction solution was refluxed for 12 h. Water was removed with a water separator. After the reaction was completed, the reaction solution was cooled to room temperature and concentrated to give the target crude product (6.8 g), which was directly used in the next step without purification.

### Step b: Synthesis of 4-bromo-2,2-difluoro-2,3-dihydro-1H-inden-1-one

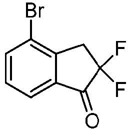

4-Bromo-N-butyl-2,3-dihydro-1H-inden-1-imine (6.8 g) was dissolved in extra-dry acetonitrile (50 mL), and anhydrous sodium sulfate (4.0 g, 28.4 mmol) and 1-chloromethyl-4-fluoro-1,4-diazabicyclo[2.2.2]octane bis(tetrafluoroborate) (18.4 g, 52.1 mmol) were added sequentially. The mixed solution was refluxed for 4 h. After the mixed solution was cooled to room temperature, concentrated hydrochloric acid (1.5 mL) was added, and the mixed solution was stirred for 1 h. After concentration, the crude product was purified by column chromatography (petroleum ether:ethyl acetate = 20:1) to give the target product (3.2 g, 55% yield).

### Step c: Synthesis of tert-butyl 4-(2,2-difluoro-1-oxo-2,3-dihydro-1H-inden-4-yl)piperazine-1-carboxylate

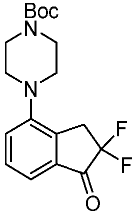

4-Bromo-2,2-difluoro-2,3-dihydro-1H-inden-1-one (600 mg, 2.4 mmol) was dissolved in toluene (15 mL), *tert*-butyl piperazine-1-carboxylate (542 mg, 2.9 mmol), tris(dibenzylideneacetone)dipalladium(0) (222 mg, 0.24 mmol), 2-dicyclohexylphosphonium-2',6'-diisopropoxy-1,1'-biphenyl (226 mg, 0.48 mmol), and anhydrous cesium carbonate (1.5 g, 4.8 mmol) were added sequentially, and the mixed solution was stirred at 100 °C for 4 h under nitrogen atmosphere. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (620 mg, 73% yield)

### Step d: Synthesis of 2,2-difluoro-4-(piperazin-1-yl)-2,3-dihydro-1H-inden-1-one hydrochloride

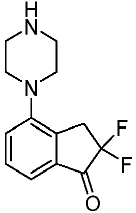

*tert*-Butyl 4-(2,2-difluoro-1-oxo-2,3-dihydro-1H-inden-4-yl)piperazine-1-carboxylate (200 mg, 0.58 mmol) was added to a solution of hydrochloric acid in 1,4-dioxane (0.1 M, 10 mL), and the mixed solution was stirred at room temperature for 2 h. The crude product obtained after concentration was directly used in the next step without purification.

### Step e: Synthesis of 5-(4-(4-(2,2-difluoro-1-one-2,3-dihydro-1H-inden-4-yl)piperazin-1-yl)butoxy)-1,1a,3,7b-tetrahydro-2H-cyclopropa[c]quinolin-2-one

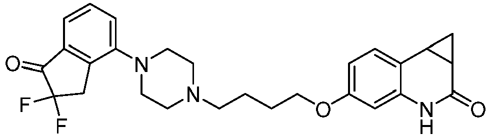

5-(4-Bromobutoxy)-1,1a,3,7b-tetrahydro-2H-cyclopropa[c]quinolin-2-one (148.3 mg, 0.48 mmol) was dissolved in anhydrous acetonitrile (15 mL), sodium iodide (71.9 mg, 0.48 mmol) was added, and the mixed solution was stirred at 90 °C for half an hour. Potassium carbonate (132.7 mg, 0.96 mmol) and 2,2-difluoro-4-(piperazin-1-yl)-2,3-dihydro-1H-inden-1-one hydrochloride (138.3 mg, 0.48 mmol) were then added to the reaction system, and the reaction system was stirred at 90 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (dichloromethane:methanol = 10:1) to give the target product (60 mg, 26% yield).

¹H NMR (600 MHz, *d₆*-DMSO): δ 9.83 (s, 1H), 7.51 (t, *J* = 7.8 Hz, 1H), 7.43 (d, *J* = 7.2 Hz, 1H), 7.40 (d, *J* = 7.8 Hz, 1H), 7.24 (d, *J* = 8.4 Hz, 1H), 6.51 (dd, *J* = 8.4, 2.4 Hz, 1H), 6.43 (d, *J* = 2.4 Hz, 1H), 3.91 (t, *J* = 6.0 Hz, 2H), 3.60 (t, *J* = 13.2 Hz, 2H), 3.00 (t, *J* = 4.2 Hz, 4H), 2.53 (brs, 4H), 2.47-2.44 (m, 1H), 2.39 (t, *J* = 7.2 Hz, 2H), 1.95-1.92 (m, 1H), 1.75-1.70 (m, 2H), 1.61-1.56 (m, 2H), 1.52-1.49 (m, 1H), 0.43 (q, *J* = 4.8 Hz, 1H).

MS Found: 482.6[M+H]⁺.

### Example 48

### 5-(4-(4-(Benzo[b]thiophen-7-yl)piperazin-1-yl)butoxy)-1,1a,3,7b-tetrahydro-2H-cyclopropa[c]quinolin-2-one

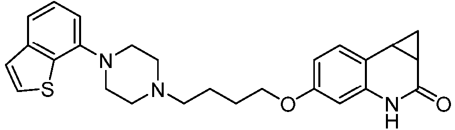

### Synthesis scheme:

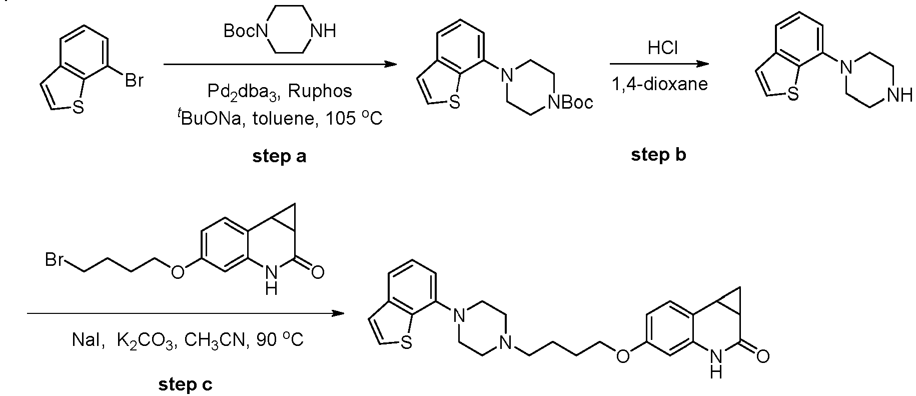

### Step a: Synthesis of tert-butyl 4-(benzo[b]thiophen-7-yl)piperazine-1-carboxylate

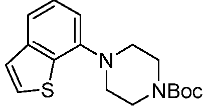

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert*-butoxide (432.5 mg, 4.5 mmol), and *tert-butyl* piperazine-1-carboxylate (838.1 mg, 4.5 mmol) each were added to a dry reaction flask, and then the reaction flask was purged three times with nitrogen in a vacuum pump. Extra-dry toluene (30 mL) and 7-bromobenzo[b]thiophene (635.8 mg, 3 mmol) were added under nitrogen atmosphere, and the mixed solution was reacted at 105 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether: ethyl acetate = 10: 1) to give the target product (900 mg, 94% yield).

### Step b: Synthesis of 1-(benzo[b]thiophen-7-yl)piperazine

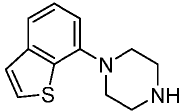

*tert-*Butyl 4-(benzo[b]thiophen-7-yl)piperazine-1-carboxylate (250 mg, 0.79 mmol) and a solution of hydrochloric acid in 1,4-dioxane (0.1 M, 10 mL) each were added to a reaction flask, and the mixed solution was reacted at room temperature for 3 h. After the reaction was completed, the reaction solution was concentrated to give crude 1-(benzo[b]thiophen-7-yl)piperazine hydrochloride, which was directly used in the next step without purification.

### Step c: Synthesis of 5-(4-(4-(benzo[b]thiophen-7-yl)piperazin-1-yl)butoxy)-1,1a,3,7b-tetrahydro-2H-cyclopropa[c]quinolin-2-one

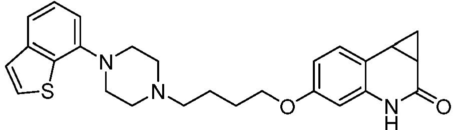

5-(4-Bromobutoxy)-1,1a,3,7b-tetrahydro-2H-cyclopropa[c]quinolin-2-one (98.9 mg, 0.32 mmol), sodium iodide (97.4 mg, 0.65 mmol), and acetonitrile (5 mL) were added to a reaction flask, respectively, and the mixed solution was reacted at 90 °C for 30 min. 1-(Benzo[b]thiophen-7-yl)piperazine hydrochloride (116.8 mg, 0.46 mmol) and potassium carbonate (98.1 mg, 0.71 mmol) were then added, and the mixed solution was reacted at 90 °C for another 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (dichloromethane:methanol = 20:1) to give the target product (100 mg, 70% yield).

¹H NMR (600 MHz, CDCl₃): δ 8.02 (s, 1H), 7.52 (d, *J* = 7.8 Hz, 1H), 7.41 (d, *J* = 5.4 Hz, 1H), 7.34-7.31 (m, 2H), 7.22 (d, *J* = 7.8 Hz, 1H), 6.94 (d, *J* = 7.8 Hz, 1H), 6.56 (dd, *J* = 8.4, 2.4 Hz, 1H), 6.29 (d, *J* = 2.4 Hz, 1H), 3.97 (t, *J* = 6.0 Hz, 2H), 3.29 (brs, 4H), 2.75 (brs, 4H), 2.55 *(t, J* = 7.2 Hz, 2H), 2.48-2.45 (m, 1H), 2.14-2.10 (m, 1H), 1.86-1.81 (m, 2H), 1.78-1.74 (m, 2H), 1.60-1.56 (m, 1H), 0.66 (q, *J* = 4.8 Hz, 1H).

MS Found: 448.4[M+H]⁺.

### Example 49

### 5-(4-(4-(2-Chlorobenzo[b]thiophen-4-yl)piperazin-1-yl)butoxy)-1,1a,3,7b-tetrahydro-2H-cyclopropa[c]quinolin-2-one

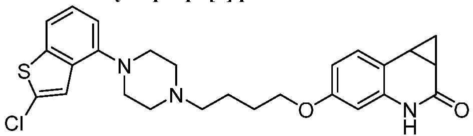

### Synthesis scheme:

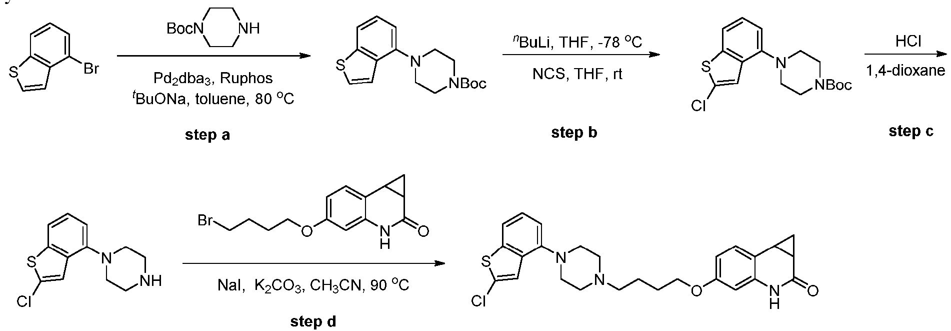

### Step a: Synthesis of tert-butyl 4-(benzo[b]thiophen-4-yl)piperazine-1-carboxylate

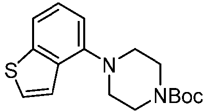

Tris(dibenzylideneacetone)dipalladium(0) (137.3 mg, 0.15 mmol), 2-dicyclohexylphosphonium-2',6'-diisopropoxy-1,1'-biphenyl (279.9 mg, 0.6 mmol), sodium *tert-*butoxide (432.5 mg, 4.5 mmol), and *tert-*butyl piperazine-1-carboxylate (838.1 mg, 4.5 mmol) each were added to a dry reaction flask, and then the reaction flask was purged three times with nitrogen in a vacuum pump. Extra-dry toluene (30 mL) and 4-bromobenzo[b]thiophene (635.8 mg, 3 mmol) were added under nitrogen atmosphere, and the mixed solution was reacted at 80 °C for 2 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether: ethyl acetate = 10:1) to give the target product (850 mg, 89% yield).

### Step b: Synthesis of tert-butyl 4-(2-chlorobenzo[b]thiophen-4-yl)piperazine-1-carboxylate

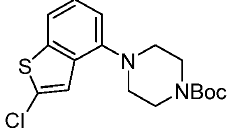

*n*-Butyllithium (0.38 mL, 2.5 M in *n*-hexane, 0.95 mmol) was slowly added dropwise to a solution of *tert-*butyl 4-(benzo[b]thiophen-4-yl)piperazine-1-carboxylate (200.4 mg, 0.63 mmol) in tetrahydrofuran (5 mL) at -78 °C, and the mixed solution was reacted at this temperature for 3 h. A solution of N-chlorosuccinimide (168.2 mg, 1.26 mmol) in tetrahydrofuran (5 mL) was then added, and the reaction solution was warmed to room temperature and stirred overnight. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (210 mg, 95% yield)

### Step c: Synthesis of 1-(2-chlorobenzo[b]thiophen-4-yl)piperazine

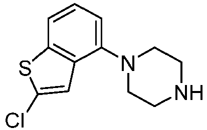

*tert-*Butyl 4-(2-chlorobenzo[b]thiophen-4-yl)piperazine-1-carboxylate (210 mg, 0.60 mmol) and a solution of hydrochloric acid in 1,4-dioxane (0.1 M, 6 mL) each were added to a reaction flask, and the mixed solution was reacted at room temperature for 3 h. After the reaction was completed, the reaction solution was concentrated to give crude 1-(2-chlorobenzo[b]thiophen-4-yl)piperazine hydrochloride, which was directly used in the next step without purification.

### Step d: Synthesis of 5-(4-(4-(2-chlorobenzo[b]thiophen-4-yl)piperazin-1-yl)butoxy)-1,1a,3,7b-tetrahydro-2H-cyclopropa[c]quinolin-2-one

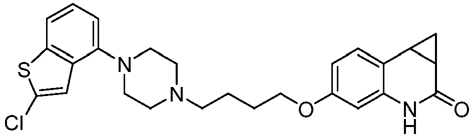

5-(4-Bromobutoxy)-1,1a,3,7b-tetrahydro-2H-cyclopropa[c]quinolin-2-one (98.9 mg, 0.32 mmol), sodium iodide (97.4 mg, 0.65 mmol), and acetonitrile (5 mL) were added to a reaction flask, respectively, and the mixed solution was reacted at 90 °C for 30 min. 1-(2-Chlorobenzo[b]thiophen-4-yl)piperazine hydrochloride (128.7 mg, 0.45 mmol) and potassium carbonate (98.1 mg, 0.71 mmol) were then added, and the mixed solution was reacted at 90 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (ethyl acetate:petroleum ether = 2:3) to give the target product (127 mg, 83% yield).

¹H NMR (600 MHz, CDCl₃): δ 7.98 (s, 1H), 7.36 (d, *J* = 8.4 Hz, 1H), 7.26-7.22 (m, 3H), 6.89 (d, *J* = 7.2 Hz, 1H), 6.56 (dd, *J* = 8.4, 1.2 Hz, 1H), 6.28 (d, *J* = 2.4 Hz, 1H), 3.97 (t, *J* = 6.0 Hz, 2H), 3.14 (brs, 4H), 2.70 (brs, 4H), 2.52 (t, *J* = 7.2 Hz, 2H), 2.48-2.45 (m, 1H), 2.14-2.10 (m, 1H), 1.85-1.81 (m, 2H), 1.75-1.70 (m, 2H), 1.60-1.57 (m, 1H), 0.66 (q, *J* = 4.8 Hz, 1H).

MS Found: 482.3 [M+H]⁺.

### Example 50

### 5-(4-(4-(5-Chlorothiazol-2-yl)piperazin-1-yl)butoxy)-1,1a,3,7b-tetrahydro-2H-epoxy[c]quinolin-2-one

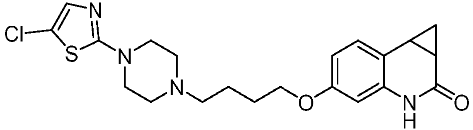

### Synthesis scheme:

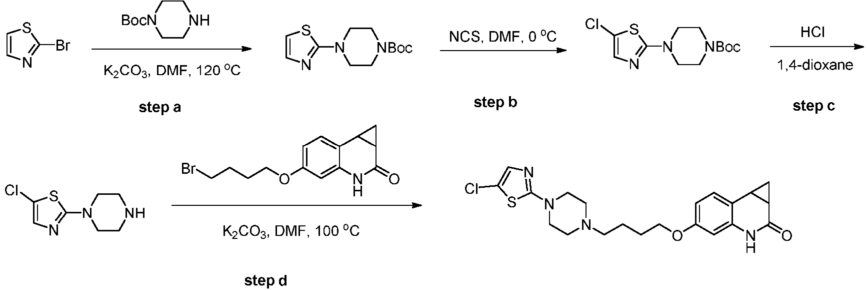

### Step a: Synthesis of tert-butyl 4-(thiazol-2-yl)piperazine-1-carboxylate

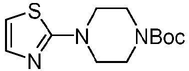

2-Bromothiazole (2.0 g, 12.2 mmol) was dissolved in *N,N*-dimethylformamide (20 mL), and *tert*-butyl piperazine-1-carboxylate (2.7 g, 14.6 mmol) and potassium carbonate (3.4 g, 24.4 mmol) were added sequentially. The mixed solution was stirred at 120 °C for 4 h under nitrogen atmosphere. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (0.7 g, 21% yield)

### Step b: Synthesis of tert-butyl 4-(5-chlorothiazol-2-yl)piperazine-1-carboxylate

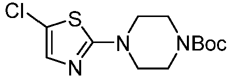

*tert*-Butyl 4-(thiazol-2-yl)piperazine-1-carboxylate (500 mg, 1.9 mmol) was dissolved in *N,N-*dimethylformamide (20 mL) and cooled to 0 °C under nitrogen atmosphere. N-Chlorosuccinimide (247 mg, 1.9 mmol) was added slowly, and the mixed solution was stirred for 3 h. The reaction solution was poured into saturated brine and extracted with ethyl acetate. The organic phase was washed with saturated brine, dried over anhydrous sodium sulfate, filtered, and concentrated. The crude product was purified by column chromatography (petroleum ether:ethyl acetate = 4:1) to give the target product (240 mg, 43% yield).

### Step c: Synthesis of 5-chloro-2-(piperazin-1-yl)thiazole hydrochloride

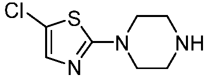

*tert*-Butyl 4-(5-chlorothiazol-2-yl)piperazine-1-carboxylate (240 mg, 0.8 mmol) was dissolved in a solution of hydrochloric acid in 1,4-dioxane (0.1 M, 5 mL), and the mixed solution was stirred for 2 h. The reaction solution was concentrated to give crude 5-chloro-2-(piperazine-1-yl)thiazole hydrochloride, which was directly used in the next step without purification.

### Step d: Synthesis of 5-(4-(4-(5-chlorothiazol-2-yl)piperazin-1-yl)butoxy)-1,1a,3,7b-tetrahydro-2H-epoxy[c]quinolin-2-one

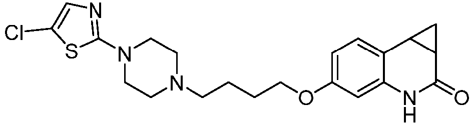

5-(4-Bromobutoxy)-1,1a,3,7b-tetrahydro-2H-cyclopropa[c]quinolin-2-one (154.5 mg, 0.5 mmol) was dissolved in *N,N*-dimethylformamide (5 mL), potassium carbonate (179.9 mg, 1.3 mmol) and 5-chloro-2-(piperazin-1-yl)thiazole hydrochloride (182.4 mg, 0.6 mmol) were added sequentially, and the reaction solution was stirred at 100 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (dichloromethane:methanol = 20:1) to give the target product (40 mg, 29% yield).

¹H NMR (600 MHz, *d₆*-DMSO): δ 9.83 (s, 1H), 7.23 (d, *J* = 8.4 Hz, 1H), 7.15 (s, 1H), 6.50 (dd, *J* = 8.4, 2.4 Hz, 1H), 6.42 (d, *J* = 2.4 Hz, 1H), 3.89 (t, *J* = 6.0 Hz, 2H), 3.32 (brs, 4H), 2.47-2.44 (m, 5H), 2.36 (t, *J* = 7.2 Hz, 2H), 1.95-1.91 (m, 1H), 1.72-1.68 (m, 2H), 1.59-1.54 (m, 2H), 1.52-1.48 (m, 1H), 0.43 (q, *J* = 4.8 Hz, 1H).

MS Found: 433.2[M+H]⁺.

### Example 51

### 5-(4-(4-(5-Chlorothiophen-2-yl)piperazin-1-yl)butoxy)-1,1a,3,7b-tetrahydro-2H-epoxy[c]quinolin-2-one

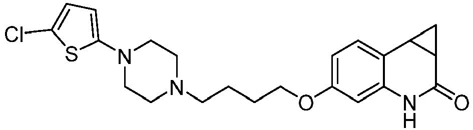

### Synthesis scheme:

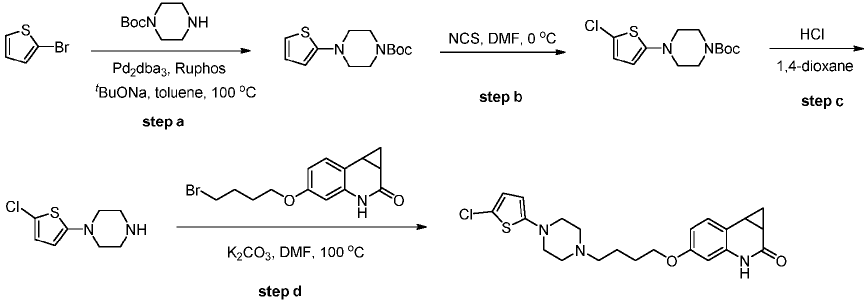

### Step a: Synthesis of tert-butyl 4-(thiophen-2-yl)piperazine-1-carboxylate

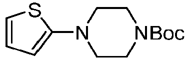

2-Bromothiophene (2.0 g, 12.3 mmol) was dissolved in toluene (20 mL), *tert*-butyl piperazine-1-carboxylate (2.7 g, 14.7 mmol), tris(dibenzylideneacetone)dipalladium(0) (1.1 g, 1.2 mmol), 2-dicyclohexylphosphonium-2',6'-diisopropoxy-1,1'-biphenyl (1.2 g, 2.5 mmol), and sodium *tert*-butoxide (2.7 g, 24.5 mmol) were added sequentially, and the mixed solution was stirred at 100 °C for 4 h under nitrogen atmosphere. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (400 mg, 12% yield)

### Step b: Synthesis of tert-butyl 4-(5-chlorothiophen-2-yl)piperazine-1-carboxylate

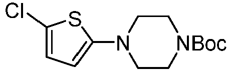

*tert*-Butyl 4-(thiophen-2-yl)piperazine-1-carboxylate (400 mg, 1.5 mmol) was dissolved in *N,N-*dimethylformamide (15 mL) and cooled to 0 °C under nitrogen atmosphere. *N*-Chlorosuccinimide (198 mg, 1.5 mmol) was added slowly, and the mixed solution was stirred for 1 h. The reaction solution was poured into a saturated aqueous sodium bicarbonate solution and extracted with ethyl acetate. The organic phase was washed with saturated brine, dried over anhydrous sodium sulfate, filtered, and concentrated. The crude product was purified by column chromatography (petroleum ether:ethyl acetate = 10:1) to give the target product (190 mg, 42% yield).

### Step c: Synthesis of 1-(5-chlorothiophen-2-yl)piperazine hydrochloride

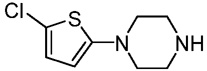

*tert*-Butyl 4-(5-chlorothiophen-2-yl)piperazine-1-carboxylate (90 mg, 0.6 mmol) was dissolved in a solution of hydrochloric acid in 1,4-dioxane (0.1 M, 5 mL), and the mixed solution was stirred for 2 h. The reaction solution was concentrated to give crude 1-(5-chlorothiophen-2-yl)piperazine hydrochloride, which was directly used in the next step without purification.

### Step d: Synthesis of 5-(4-(4-(5-chlorothiophen-2-yl)piperazin-1-yl)butoxy)-1,1a,3,7b-tetrahydro-2H-epoxy[c]quinolin-2-one

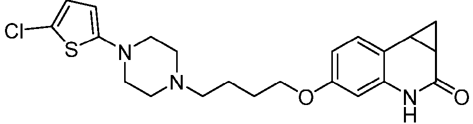

5-(4-Bromobutoxy)-1,1a,3,7b-tetrahydro-2H-cyclopropa[c]quinolin-2-one (123.6 mg, 0.4 mmol) was dissolved in *N,N*-dimethylformamide (5 mL), potassium carbonate (179.7 mg, 1.3 mmol) and 1-(5-chlorothiophen-2-yl)piperazine hydrochloride (165 mg, 0.5 mmol) were added sequentially, and the reaction solution was stirred at 100 °C for 12 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (dichloromethane:methanol = 20:1) to give the target product (30 mg, 24% yield).

¹HNMR (600 MHz, CDCl₃): δ 7.53 (s, 1H), 7.23 (d, *J* = 8.4 Hz, 1H), 6.83 (d, *J* = 6.0 Hz, 1H), 6.79 (d, *J* = 5.4 Hz, 1H), 6.55 (dd, *J* = 8.4, 2.4 Hz, 1H), 6.23 (d, *J* = 2.4 Hz, 1H), 3.95 (t, *J* = 6.6 Hz, 2H), 3.10 (brs, 4H), 2.66 (brs, 4H), 2.48-2.45 (m, 3H), 2.14-2.10 (m, 1H), 1.82-1.79 (m, 2H), 1.73-1.71 (m, 2H), 1.61-1.57 (m, 1H), 0.67 (q, *J* = 4.8 Hz, 1H).

MS Found: 432.2[M+H]⁺.

### Example 52

### 5-(4-(4-(1H-Indol-4-yl)piperazin-1-yl)butoxy)-1,1a,3,7b-tetrahydro-2H-cyclopropa[c]quinolin-2-one

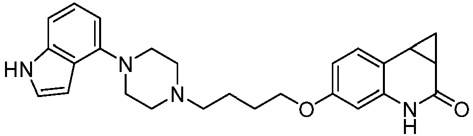

### Synthesis scheme:

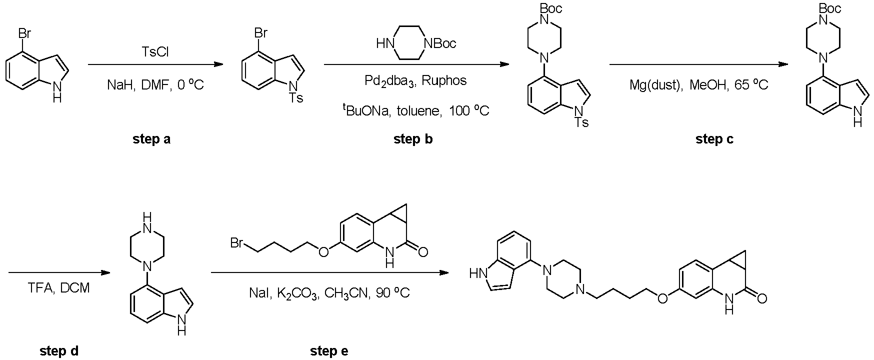

### Step a: Synthesis of 4-bromo-tosyl-1H-indole

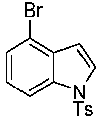

Sodium hydride (2.0 g, 51.0 mmol) was added portionwise to a solution of 4-bromo-1-indanone (5.0 g, 25.5 mmol) in *N,N*-dimethylformamide (50 mL) at 0 °C. After the mixed solution was reacted at this temperature for 30 min, *p*-toluenesulfonyl chloride (5.3 g, 28.1 mmol) was added portionwise, and then the reaction solution was warmed to room temperature and stirred for 30 min. After the reaction was completed, the reaction solution was poured into ice water and extracted with ethyl acetate. The organic phase was washed with saturated brine, dried over anhydrous sodium sulfate, filtered, and concentrated. The crude product was purified by column chromatography (petroleum ether: ethyl acetate = 10:1) to give the target product (7.0 g, 78% yield).

### Step b: Synthesis of tert-butyl 4-(1-tosyl-1H-indol-4-yl)piperazine-1-carboxylate

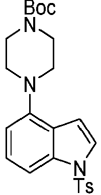

*tert*-Butyl piperazine-1-carboxylate (4.5 g, 24.0 mmol), tris(dibenzylideneacetone)dipalladium(0) (916 mg, 1.0 mmol), 2-dicyclohexylphosphonium-2',6'-diisopropoxy-1,1'-biphenyl (932 mg, 2.0 mmol), and sodium *tert*-butoxide (3.8 g, 40.0 mmol) each were added to a solution of 4-bromo-tosyl-1H-indole (7.0 g, 20.0 mmol) in toluene (50 mL),, and the mixed solution was stirred at 100 °C for 4 h under nitrogen atmosphere. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether: ethyl acetate = 5:1) to give the target product (4.5 g, 49% yield).

### Step c: Synthesis of tert-butyl 4-(1H-indol-4-yl)piperazine-1-carboxylate

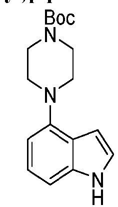

Magnesium chips (1.2 g, 49.4 mmol) were added to a solution of *tert*-butyl 4-(1-tosyl-1H-indol-4-yl)piperazine-1-carboxylate (4.5 g, 9.9 mmol) in methanol (50 mL), and the mixed solution was refluxed for 30 min. After the reaction was completed, the reaction solution was cooled to room temperature, filtered, and concentrated. The crude product was purified by column chromatography (petroleum ether:ethyl acetate = 1:1) to give the target product (2.7 g, 93% yield).

### Step d: Synthesis of 4-(piperazin-1-yl)-1H-indole trifluoroacetate

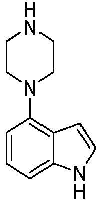

*tert*-Butyl 4-(1H-indol-4-yl)piperazine-1-carboxylate (200 mg, 0.49 mmol), dichloromethane (10 mL), and trifluoroacetic acid (2 mL) each were added to a reaction flask" and the mixed solution was stirred at room temperature for 2 h. The reaction solution was concentrated to give a crude product, which was directly used in the next step without purification.

### Step e: Synthesis of 5-(4-(4-(1H-indol-4-yl)piperazin-1-yl)butoxy)-1,1a,3,7b-tetrahydro-2H-cyclopropa[c]quinolin-2-one

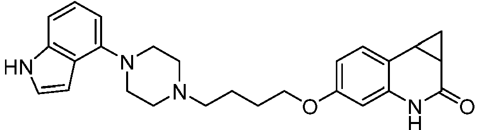

5-(4-Bromobutoxy)-1,1a,3,7b-tetrahydro-2H-cyclopropa[c]quinolin-2-one (206.0 mg, 0.66 mmol), sodium iodide (99.6 mg, 0.66 mmol), and acetonitrile (15 mL) were added to a reaction flask, respectively, and the mixed solution was reacted at 90 °C for 30 min. Potassium carbonate (275.1 mg, 1.99 mmol) and 4-(piperazin-1-yl)-1H-indole trifluoroacetate (133.6 mg, 0.66 mmol) were then added, and the mixed solution was reacted at 90 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (dichloromethane:methanol =10:1) to give the target product (30 mg, 10% yield).

¹H NMR (600 MHz, *d₆*-DMSO): δ 11.00 (s, 1H), 9.83 (s, 1H), 7.24 (d, *J* = 8.4 Hz, 1H), 7.22 (t, *J* = 3.0 Hz, 1H), 7.01 (d, *J* = 8.4 Hz, 1H), 6.95 (t, *J* = 7.8 Hz, 1H), 6.52 (dd, *J* = 8.4, 2.4 Hz, 1H), 6.44-6.42 (m, 2H), 6.35 (t, *J* = 2.4 Hz, 1H), 3.92 (t, *J* = 6.6 Hz, 2H), 3.10 (brs, 4H), 2.59 (brs, 4H), 2.47-2.44 (m, 1H), 2.41 (t, *J* = 7.2 Hz, 2H), 1.95-1.92 (m, 1H), 1.75-1.71 (m, 2H), 1.63-1.58 (m, 2H), 1.52-1.48 (m, 1H), 0.44 (q, *J* = 4.8 Hz, 1H).

MS Found: 431.6[M+H]⁺.

### Example 53

### Synthesis of 5-(4-(4-(1H-indol-7-yl)piperazin-1-yl)butoxy)-1,1a,3,7b-tetrahydro-2H-cyclopropa[c]quinolin-2-one

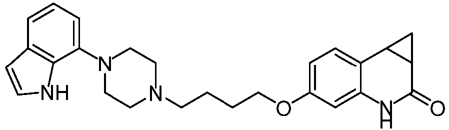

### Synthesis scheme:

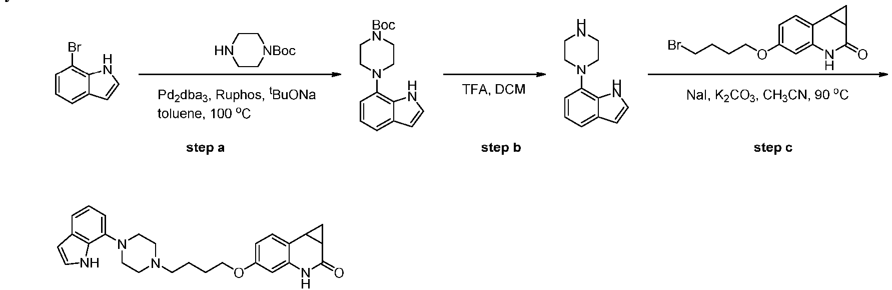

### Step a: Synthesis of tert-butyl 4-(1H-indol-7-yl)piperazine-1-carboxylate

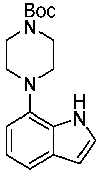

Tris(dibenzylideneacetone)dipalladium(0) (701.0 mg, 0.76 mmol), 2-dicyclohexylphosphonium-2',6'-diisopropoxy-1,1'-biphenyl (713.2 mg, 1.53 mmol), sodium *tert-*butoxide (1.5 g, 15.3 mmol), and *tert-butyl* piperazine-1-carboxylate (1.7 g, 9.2 mmol) each were added to a dry reaction flask" and then the reaction flask was purged three times with nitrogen in a vacuum pump. Extra-dry toluene (20 mL) and 7-bromo-1H-indole (1.5 g, 25.5 mmol) were added under nitrogen atmosphere, and the mixed solution was reacted at 100 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (petroleum ether:ethyl acetate = 5:1) to give the target product (420 mg, 18% yield).

### Step b: Synthesis of 7-(piperazin-1-yl)-1H-indole trifluoroacetate

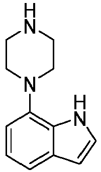

*tert*-Butyl 4-(1H-indol-7-yl)piperazine-1-carboxylate (200 mg, 0.66 mmol), dichloromethane (10 mL), and trifluoroacetic acid (2 mL) each were added to a reaction flask" and the mixed solution was stirred at room temperature for 2 h. The reaction solution was concentrated to give a crude product, which was directly used in the next step without purification.

### Step c: Synthesis of 5-(4-(4-(1H-indol-7-yl)piperazin-1-yl)butoxy)-1,1a,3,7b-tetrahydro-2H-cyclopropa[c]quinolin-2-one

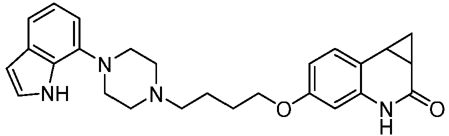

5-(4-Bromobutoxy)-1,1a,3,7b-tetrahydro-2H-cyclopropa[c]quinolin-2-one (206.0 mg, 0.66 mmol), sodium iodide (99.6 mg, 0.66 mmol), and acetonitrile (15 mL) were added to a reaction flask, respectively, and the mixed solution was reacted at 90 °C for 30 min. Potassium carbonate (275.1 mg, 1.99 mmol) and 7-(piperazin-1-yl)-1H-indole trifluoroacetate (133.6 mg, 0.66 mmol) were then added, and the mixed solution was reacted at 90 °C for 4 h. After the reaction was completed, the reaction was quenched with a saturated aqueous ammonium chloride solution, and the reaction solution was extracted with ethyl acetate. The organic phase was washed with saturated brine, dried over anhydrous sodium sulfate, filtered, and concentrated. The crude product was purified by column chromatography (dichloromethane:methanol =10:1) to give the target product (35 mg, 12% yield).

¹HNMR (600 MHz, *d₆*-DMSO): δ 11.00 (s, 1H), 9.83 (s, 1H), 7.24 (d, *J* = 8.4 Hz, 1H), 7.22 (t, *J* = 3.0 Hz, 1H), 7.01 (d, *J* = 8.4 Hz, 1H), 6.95 (t, *J* = 7.8 Hz, 1H), 6.52 (dd, *J* = 8.4, 2.4 Hz, 1H), 6.44-6.42 (m, 2H), 6.35 (t, *J* = 2.4 Hz, 1H), 3.92 (t, *J* = 6.6 Hz, 2H), 3.10 (brs, 4H), 2.59 (brs, 4H), 2.47-2.44 (m, 1H), 2.41 (t, *J* = 7.2 Hz, 2H), 1.95-1.92 (m, 1H), 1.75-1.71 (m, 2H), 1.63-1.58 (m, 2H), 1.52-1.48 (m, 1H), 0.44 (q, *J* = 4.8 Hz, 1H).

MS Found: 431.6[M+H]⁺.

### Biological Test Evaluation

### Test Example 1. Test Method for D_{2S} Receptor cAMP Agonist

### 1.1 Experimental materials:

cAMP detection kit was purchased from Cisbio; an HEK293 cell strain stably expressing D_{2S} receptor was constructed by Shanghai Shujing Biopharma Co., Ltd.; dopamine, forskolin, and IBMX were purchased from Sigma; ProxiPlate-384 well plates were purchased from PerkinElmer; HBSS was purchased from Thermo Fisher Scientific. PerkinElmer Envision 2105 multifunctional microplate reader, Tecan D300e picoliter micro-dosing system, Agilent Bravo liquid workstation, and Countstar BioTech cell counter.

### 1.2 Experimental methods:

(1) The cultured cells were digested with trypsin, and the cell suspension was washed with HBSS buffer after the digestion was terminated. The medium was removed by centrifugation at 200× g. The pellet was resuspended in an appropriate amount of experimental buffer, and 20 µL of cell suspension was counted using a cell counter and diluted to 0.6 × 10⁶ cells/mL.
(2) 5 µL of cell suspension was added to a ProxiPlate-384 well plate (3 × 10³ cells/well).
(3) Test compounds were serially diluted in an experimental buffer, and 5 µL of the mixed solution was transferred to the reaction plate with Bravo. 5 µL of experimental buffer was transferred to negative control wells, and 5 µL of dopamine (with a final concentration of 1 × 10⁻⁷ M) was transferred to positive control wells.
(4) The plate was incubated at room temperature for 15 min.
(5) 25 nL of forskolin (with a final concentration of 5 × 10⁻⁷ M) was added to the reaction plate using Tecan D300e picoliter micro-dosing system.
(6) The plate was incubated at room temperature for 45 min.
(7) 10 µL of detection reagent was added to the reaction plate, and the plate was incubated at room temperature for 1 h in the dark.
(8) Detection was performed using Envision 2105 multifunctional microplate reader. Excitation light at 340 nm, emission light at 620 nm and 665 nm. The ratio of 665 nm/620 nm for each test well was calculated.Activation rate (Activity%) = (negative control ratio - compound ratio) / (negative control ratio - positive control ratio) × 100%

### 1.3 Experimental results: The results are shown in Table 1.

**Table 1. Agonistic activity results of compounds of the present invention on D_{2S} receptor**

| **Example No.** | **D_{2S}** | |
|---|---|---|
| | **EC₅₀ (nM)** | **Max Act%** |
| Example 1 | 0.97 | 114.9 |
| Example 1-P1 | 0.94 | 111.9 |
| Example 1-P2 | 1.49 | 113.8 |
| Example 38 | 0.88 | 92.4 |
| Example 41 | 0.92 | 115.0 |
| Example 41-P1 | 0.93 | 114.2 |
| Example 41-P2 | 0.96 | 113.4 |
| Example 42 | 0.95 | 106.0 |
| Example 42-P1 | 1.00 | 102.0 |
| Example 42-P2 | 0.93 | 99.2 |
| Example 43 | 0.92 | 134.9 |
| Example 43-P1 | 1.30 | 123.1 |
| Example 43-P2 | 0.92 | 125.3 |
| Example 44 | 0.93 | 91.0 |
| Example 44-P1 | 0.98 | 96.8 |
| Example 44-P2 | 0.92 | 89.1 |
| Example 45 | 0.93 | 105.6 |
| Example 45-P1 | 1.01 | 106.1 |
| Example 45-P2 | 0.89 | 111.5 |
| Example 46 | 0.93 | 114.3 |
| Example 46-P1 | 0.96 | 113.3 |
| Example 46-P2 | 0.93 | 110.2 |
| Example 47 | 0.97 | 102.9 |
| Example 48 | 0.89 | 114.8 |
| Example 49 | 0.99 | 112.6 |
| Example 51 | 0.76 | 111.1 |
| Aripiprazole | 0.92 | 108.8 |
| RP5063 | 1.81 | 91.6 |

### 1.4 Experimental conclusion:

As can be seen from the results in Table 1 described above, the compounds of the present invention have an agonistic effect on the dopamine D_{2S}.

### Test Example 2. Test Method for 5-HT_{1A} Receptor cAMP Agonist

### 2.1 Experimental materials:

cAMP detection kit was purchased from Cisbio; an HEK293 cell strain stably expressing 5-HT_{1A} receptor was constructed by Shanghai Shujing Biopharma Co., Ltd.; serotonin, forskolin, and IBMX were purchased from Sigma; ProxiPlate-384 well plates were purchased from PerkinElmer; HBSS was purchased from Thermo Fisher Scientific. PerkinElmer Envision 2105 multifunctional microplate reader, Tecan D300e picoliter micro-dosing system, Agilent Bravo liquid workstation, and Countstar BioTech cell counter.

### 2.2 Experimental methods:

(1) The cultured cells were digested with trypsin, and the cell suspension was washed with HBSS buffer after the digestion was terminated. The medium was removed by centrifugation at 200× g. The pellet was resuspended in an appropriate amount of experimental buffer, and 20 µL of cell suspension was counted using a cell counter and diluted to 0.4 × 10⁶ cells/mL.
(2) 5 µL of cell suspension was added to a ProxiPlate-384 well plate (2 × 10³ cells/well).
(3) Test compounds were serially diluted in an experimental buffer, and 5 µL of the mixed solution was transferred to the reaction plate with Bravo. 5 µL of experimental buffer was transferred to negative control wells, and 5 µL of serotonin (with a final concentration of 10⁻⁶ M) was transferred to positive control wells.
(4) The plate was incubated at room temperature for 15 min.
(5) 25 nL of forskolin (with a final concentration of 2.5 × 10⁻⁷ M) was added to the reaction plate using Tecan D300e picoliter micro-dosing system.
(6) The plate was incubated at room temperature for 45 min.
(7) 10 µL of detection reagent was added to the reaction plate, and the plate was incubated at room temperature for 1 h in the dark.
(9) Detection was performed using Envision 2105 multifunctional microplate reader. Excitation light at 340 nm, emission light at 620 nm and 665 nm. The ratio of 665 nm/620 nm for each test well was calculated.Activation rate (Activity%) = (negative control ratio - compound ratio) / (negative control ratio - positive control ratio) × 100

### 2.3 Experimental results: The results are shown in Table 2.

**Table 2. Agonistic activity results of compounds of the present invention on 5-HT_{1A} receptor**

| **Example No.** | **5-HT_{1A}** | |
|---|---|---|
| | **EC₅₀ (nM)** | **Max Act%** |
| Example 1 | 1005 | 50.2 |
| Example 1-P1 | 1796 | 33.9 |
| Example 1-P2 | 1213 | 45.2 |
| Example 41 | 50.84 | 92.7 |
| Example 41-P1 | 36.49 | 99.0 |
| Example 41-P2 | 18.64 | 96.2 |
| Example 42 | 313.1 | 55.7 |
| Example 42-P1 | 143.1 | 62.9 |
| Example 42-P2 | 692.7 | 45.5 |
| Example 43 | 157.6 | 67.7 |
| Example 43-P1 | 194.1 | 68.3 |
| Example 43-P2 | 92.41 | 60.6 |
| Example 44 | 11.24 | 58.9 |
| Example 44-P1 | 12.73 | 49.5 |
| Example 44-P2 | 7.05 | 48.0 |
| Example 45 | 253.0 | 54.7 |
| Example 45-P1 | 243.0 | 56.4 |
| Example 45-P2 | 199.6 | 52.8 |
| Example 46 | 353.5 | 62.6 |
| Example 46-P1 | 344.7 | 67.7 |
| Example 46-P2 | 258.4 | 59.3 |
| Example 47 | 206.5 | 75.8 |
| Example 48 | 366.2 | 55.2 |
| Example 49 | 128.1 | 80.7 |
| Example 51 | 73.40 | 79.4 |
| Aripiprazole | 2417 | 43.8 |
| RP5063 | 1881 | 31.6 |

### 2.4 Experimental conclusion:

As can be seen from the results in Table 2 described above, the compounds of the present invention have an agonistic effect on the 5-HT_{1A} receptor.

### Test Example 3. Calcium Flux Test Method for 5-HT_{2A} Receptor

### 3.1 Experimental materials:

Fluo-4 calcium flux detection kit was purchased from Invitrogen; a CHO cell strain stably expressing 5-HT_{2A} receptor was purchased from PerkinElmer; serotonin was purchased from Sigma; ketanserin was purchased from Targetmol; polylysine-coated 384-well plates and 384-well compound plates were purchased from Greiner Bio-One. Molecular Device FLIPR, Agilent Bravo liquid workstation, and Countstar BioTech cell counter.

### 3.2 Experimental methods:

The first day: cell plating
(1) The cryopreserved cells were rapidly thawed in a water bath at 37 °C, and the cells were resuspended in a plating medium. The cryopreservation solution was removed by centrifugation at 200× g. The pellet was resuspended in an appropriate amount of plating medium, 20 µL of cell suspension was counted using a cell counter, and the cell density was diluted to 1 × 10⁶ cells/mL.
(2) 20 µL of cell suspension was added to a polylysine-coated 384-well plate (2 × 10⁴ cells/well).
(3) The cell plate was incubated in a 37 °C/5% CO₂ incubator for 16-20 h.

The next day: test compound detection
(1) The cell plate was taken out from the incubator and centrifuged at 100× g to remove the medium. 20 µL of experimental buffer was added, and then 20 µL of 2× fluorescent probe solution was added. The plate was incubated at 37 °C for 50 min and equilibrated at room temperature for 10 min.
(2) EC₈₀ test for an agonist positive compound: the agonist positive compound serotonin was serially diluted in an experimental buffer. The cell plate and agonist positive compound plate were placed into the FLIPR instrument, the detection program was run, 10 µL of the diluted compound was transferred from the agonist compound plate to the cell plate, and the fluorescence signal was read. ECso for the agonist positive compound was calculated using Screenworks software, and 6× ECso concentration was prepared for later use.
(3) Agonistic and antagonistic activity test for test compounds: the test compounds were serially diluted in an experimental buffer using Bravo. The cell plate and test compound plate were placed in the FLIPR instrument, the detection program was run, and 10 µL of the diluted compound was transferred from the test compound plate to the cell plate. 10 µL of experimental buffer was used as a negative control, 10 µL of serotonin (with a final concentration of 10⁻⁵ M) was used as a positive control, and the fluorescence signal was read. The test compound plate was then taken out, and the agonist positive compound plate with 6× ECso concentration was placed. 10 µL of solution was transferred from the agonist positive compound plate with 6× ECso concentration to the cell plate, 10 µL of experimental buffer was used as the negative control, 10 µL of ketanserin (with a final concentration of 10⁻⁵ M) was used as the positive control, and the fluorescent signal was read.Inhibition rate (Inhibitiony%) = (negative control reading value - compound reading value) / (negative control reading value - positive control reading value) × 100%

### 3.3 Experimental results: The results are shown in Table 3.

**Table 3. Regulatory activity results of compounds of the present invention on 5-HT_{2A} receptor**

| **Example No.** | **5-HT_{2A}** | |
|---|---|---|
| | **IC₅₀ (nM)** | **Max Inh%** |
| Example 1 | 730.9 | 98.4 |
| Example 1-P1 | 745.4 | 100.3 |
| Example 1-P2 | 736.9 | 100.3 |
| Example 38 | 4847 | 98.1 |
| Example 39 | 2931 | 94.2 |
| Example 41 | 3347 | 80.4 |
| Example 41-P1 | 2912 | 88.2 |
| Example 41-P2 | 3452 | 88.2 |
| Example 42 | 1687 | 100.6 |
| Example 42-P1 | 1955 | 100.5 |
| Example 42-P2 | 497.3 | 97.3 |
| Example 43 | 971.2 | 100.3 |
| Example 43-P1 | 853.0 | 100.7 |
| Example 43-P2 | 1128 | 96.6 |
| Example 44 | 260.2 | 99.7 |
| Example 44-P1 | 192.2 | 99.6 |
| Example 44-P2 | 289.5 | 99.7 |
| Example 45 | 460.0 | 100.1 |
| Example 45-P1 | 397.9 | 99.8 |
| Example 45-P2 | 411.5 | 100.0 |
| Example 46 | 2342 | 91.6 |
| Example 46-P1 | 910.1 | 96.4 |
| Example 46-P2 | 535.1 | 90.9 |
| Example 47 | 268.2 | 96.0 |
| Example 48 | 2406 | 100.5 |
| Example 49 | 7402 | 90.4 |
| Example 51 | 2526 | 99.8 |
| Example 52 | 1585 | 99.9 |
| Aripiprazole | 8543 | 86.0 |
| RP5063 | 9126 | 67.6 |

### 3.4 Experimental conclusion:

As can be seen from the results in Table 3 described above, the compounds of the present invention have an antagonistic effect on the 5-HT_{2A} receptor.

### Test Example 4. Calcium Flux Test Method for 5-HT_{2B} Receptor

### 4.1 Experimental materials:

Fluo-4 calcium flux detection kit was purchased from Invitrogen; an HEK293 cell strain stably expressing 5-HT_{2B} receptor was purchased from GenScript; serotonin was purchased from Sigma; methiothepin was purchased from Targetmol; polylysine-coated 384-well plates and 384-well compound plates were purchased from Greiner Bio-One. Molecular Device FLIPR, Agilent Bravo liquid workstation, and Countstar BioTech cell counter.

### 4.2 Experimental methods:

The first day: cell plating
(1) The cultured cells were digested with trypsin, and the cell suspension was taken after the digestion was terminated. The cryopreserved cells were rapidly thawed in a water bath at 37 °C, and the cells were resuspended in a plating medium. The medium and cryopreservation solution were removed by centrifugation at 200× g. The pellet was resuspended in an appropriate amount of plating medium, 20 µL of cell suspension was counted using a cell counter, and the cell density was diluted to 1 × 10⁶ cells/mL.
(2) 20 µL of cell suspension was added to a polylysine-coated 384-well plate (2 × 10⁴ cells/well).
(3) The cell plate was incubated in a 37 °C/5% CO₂ incubator for 16-20 h.

The next day: test compound detection
(1) The cell plate was taken out from the incubator and centrifuged at 100× g to remove the medium. 20 µL of experimental buffer was added, and then 20 µL of 2× fluorescent probe solution was added. The plate was incubated at 37 °C for 50 min and equilibrated at room temperature for 10 min.
(2) EC₈₀ test for an agonist positive compound: the agonist positive compound serotonin was serially diluted in an experimental buffer. The cell plate and agonist positive compound plate were placed into the FLIPR instrument, the detection program was run, 10 µL of the diluted compound was transferred from the agonist compound plate to the cell plate, and the fluorescence signal was read. ECso for the agonist positive compound was calculated using Screenworks software, and 6× ECso concentration was prepared for later use.
(3) Antagonistic activity test for test compounds: the test compounds were serially diluted in an experimental buffer. The cell plate and test compound plate were placed in the FLIPR instrument, the detection program was run, and 10 µL of the diluted compound was transferred from the test compound plate to the cell plate. 10 µL of solution was then transferred from the agonist positive compound plate with 6× ECso concentration to the cell plate. 10 µL of experimental buffer was used as the negative control, 10 µL of methiothepin (with a final concentration of 10⁻⁵ M) was used as the positive control, and the fluorescence signal was read.Inhibition rate (Inhibitiony%) = (negative control reading value - compound reading value) / (negative control reading value - positive control reading value) × 100%

### 4.3 Experimental results: The results are shown in Table 4.

**Table 4. Regulatory activity results of compounds of the present invention on 5-HT_{2B} receptor**

| **Example No.** | **5-HT_{2B}** | |
|---|---|---|
| | **IC₅₀ (nM)** | **Max Inh%** |
| Example 1 | 658.1 | 105.8 |

### 4.4 Experimental conclusion:

As can be seen from the results in Table 4 described above, the compounds of the present invention have an antagonistic effect on the 5-HT_{2B} receptor.

### Test Example 5. Test Method for D_{2L} Receptor cAMP Agonist

### 5.1 Experimental materials:

cAMP detection kit was purchased from Cisbio; an HEK293 cell strain stably expressing D_{2L} receptor was constructed by Shanghai Shujing Biopharma Co., Ltd.; dopamine, forskolin, and IBMX were purchased from Sigma; ProxiPlate-384 well plates were purchased from PerkinElmer; HBSS was purchased from Thermo Fisher Scientific. PerkinElmer Envision 2105 multifunctional microplate reader, Tecan D300e picoliter micro-dosing system, Agilent Bravo liquid workstation, and Countstar BioTech cell counter.

### 5.2 Experimental methods:

(1) The cryopreserved cells were thawed in a water bath at 37 °C, the cell suspension was resuspended in HBSS buffer, and the mixed solution was centrifuged at 750 rpm for 5 min to remove the medium. The pellet was resuspended in an appropriate amount of experimental buffer, and an appropriate amount of cell suspension was diluted to 0.4 × 10⁶ cells/mL.
(2) 5 µL of cell suspension was added to a ProxiPlate-384 well plate (2 × 10³ cells/well), and the plate was centrifuged at 1000 rpm for 1 min.
(3) Test compounds were serially diluted in an experimental buffer, and 5 µL of the mixed solution was transferred to the reaction plate with Bravo. 5 µL of experimental buffer was transferred to negative control wells, and 5 µL of dopamine (with a final concentration of 1 µM) was transferred to positive control wells.
(4) The plate was incubated at room temperature for 15 min.
(5) 25.1 nL of forskolin (with a final concentration of 0.5 µM) was added to the reaction plate using Tecan D300e picoliter micro-dosing system.
(6) The plate was incubated at room temperature for 45 min in the dark.
(7) 10 µL of detection reagent was added to the reaction plate, and the plate was incubated at room temperature for 1 h in the dark.
(8) Detection was performed using Envision 2105 multifunctional microplate reader. Excitation light at 340 nm, emission light at 620 nm and 665 nm. The ratio of 665 nm/620 nm for each test well was calculated.Activation rate (Activity%) = (negative control ratio - compound ratio) / (negative control ratio - positive control ratio) × 100%

### 5.3 Experimental results: The results are shown in Table 5.

**Table 5. Agonistic activity results of compounds of the present invention on D_{2L} receptor**

| **Example No.** | **D_{2L}** | |
|---|---|---|
| | **EC₅₀ (nM)** | **Max Act%** |
| Example 1 | 0.62 | 54.4 |
| Example 1-P1 | 1.07 | 47.7 |
| Example 1-P2 | 0.61 | 33.1 |
| Example 38 | 1.39 | 32.3 |
| Example 41 | 0.97 | 42.4 |
| Example 41-P1 | 0.78 | 46.7 |
| Example 41-P2 | 0.36 | 58.6 |
| Example 42 | 0.47 | 32.7 |
| Example 42-P1 | 0.99 | 29.6 |
| Example 42-P2 | 0.43 | 32.6 |
| Example 43 | 0.30 | 42.8 |
| Example 43-P1 | 0.90 | 37.9 |
| Example 43-P2 | 0.25 | 35.7 |
| Example 44 | 0.37 | 44.7 |
| Example 44-P1 | 0.26 | 45.9 |
| Example 44-P2 | 0.22 | 53.9 |
| Example 45 | 0.44 | 41.3 |
| Example 45-P1 | 2.12 | 28.0 |
| Example 45-P2 | 0.62 | 41.5 |
| Example 46 | 0.40 | 43.4 |
| Example 46-P1 | 0.49 | 42.2 |
| Example 46-P2 | 0.38 | 43.6 |
| Example 47 | 0.84 | 35.9 |
| Example 48 | 0.49 | 49.0 |
| Example 49 | 0.91 | 56.3 |
| Example 51 | 1.53 | 82.6 |
| Example 52 | 0.31 | 65.3 |
| Aripiprazole | 1.06 | 57.7 |
| RP5063 | 3.40 | 40.6 |

5.4 Experimental conclusion:

As can be seen from the results in Table 5 described above, the compounds of the present invention have an agonistic effect on the dopamine D_{2L}.

### Test Example 6. Pharmacokinetic Test of the Compounds of the Present Invention in Mice

### 6.1 Objective:

Male ICR mice were administered orally intragastrically, the plasma concentrations of the compounds of the present invention were measured in the mice, the PK parameters were calculated, and the pharmacokinetic evaluation was performed on the compounds of the present invention.

### 6.2 Test materials:

(1) Test samples: the compounds of the examples of the present invention, self-made.
(2) Test animals: ICR mice, SPF grade, male, Xuzhou Medical University.
(3) Main test instruments:

| **Name** | **Model** | **Manufacturer** |
|---|---|---|
| AB Sciex triple quadrupole liquid chromatography-mass spectrometry instrument | Triple Quad 5500+ QTRAP | SCIEX |
| Sartorius dual-range balance | SECURA225D-ICN | Sartorius Scientific Instruments Co., Ltd. |
| Benchtop high-speed refrigerated centrifuge | Thermo 75009915 | Thermo Scientific |
| RAININ micropipette | 20/200/1000 µL | RAININ^{®}, USA |
| Microplate shaker | 88882006 | Thermo Scientific |
| Benchtop ultrasonic cleaner | KQ5200DE | Kunshan Ultrasonic Instruments Co., Ltd. |
| Vortex oscillator | Vortex genie 2 | Scientific Industries, USA |
| Water purification instrument | Milli-Q IQ7000 | Merck, Germany |
| Precision electronic balance | XSE105DU | METTLER TOLEDO |

### 6.3 Test protocol:

### (1) Administration information:

Drug preparation: according to the weight of the weighed drug, the preparation volume was calculated. 5% of DMSO was added firstly. After the drug was fully dissolved, 95% of 20% aqueous β-cyclodextrin solution was then added, and the mixture was mixed well for later use.

Administration route: oral intragastric administration. Frequency and duration of administration: single administration.

### (2) Test method:

ICR mice were stratified according to body weight and then randomly divided into groups of 9 mice each, and the mice were fasted overnight prior to the test. The mice were separately administered orally intragastrically. 50 µL of blood was collected from the fundus vein of the mouse at 0 h, 0.083 h, 0.25 h, 0.5 h, 1 h, 2 h, 4 h, 8 h, 12 h, and 24 h (3 time points for each mouse) to a sample tube containing heparin sodium as an anticoagulant, and the tube was placed in wet ice and centrifuged at 4000 r·min⁻¹ for 10 min. The plasma was separated, and cryopreserved in a refrigerator at -80 °C for testing.

### 6.4 Test results and analysis:

The concentrations of plasma samples were measured by high performance liquid chromatography-tandem mass spectrometry (LC-MS/MS). The retention times of the compounds and internal standard, chromatogram acquisitions, and integrals of chromatograms were processed using the software Analyst (AB SCIEX), and the data statistics were processed using the software Watson LIMS (Thermo Fisher Scientific) or Analyst (AB SCIEX). The concentration of plasma was processed using a non-compartmental model of the pharmacokinetic software WinNonlin^{™} Version 6.3 (Pharsight, Mountain View, CA), and pharmacokinetic parameters were calculated by a linear log-trapezoidal method. The specific results are shown in Table 6.

**Table 6. Pharmacokinetic experiment results in mice**

| **Example No.** | **Dose (mg/kg)** | **t_{1/2} (h)** | **Cₘₐₓ (ng/mL)** | **AUC_{(0-inf)} (ng•h/mL)** |
|---|---|---|---|---|
| Example 1 | 5 | 10.8 | 510.7 | 7162.9 |
| Example 1-P1 | 5 | 34.3 | 341.3 | 13142.6 |
| Example 1-P2 | 5 | 10.8 | 429.7 | 7096.5 |
| Example 41 | 5 | 8.6 | 971.3 | 9974.8 |
| Example 41-P1 | 5 | 6.4 | 1593.3 | 12950.5 |
| Example 41-P2 | 5 | 15.9 | 701.3 | 13958.8 |
| Example 42 | 5 | 13.0 | 687.0 | 11254.7 |
| Example 42-P1 | 5 | 4.1 | 1180.0 | 8000.6 |
| Example 42-P2 | 5 | 5.8 | 1296.7 | 11157.8 |
| Example 45 | 5 | 6.1 | 1160.0 | 9654.5 |
| Example 45-P1 | 5 | 3.2 | 773.7 | 4769.0 |
| Example 45-P2 | 5 | 3.0 | 660.0 | 4083.7 |
| Example 46 | 5 | 15.2 | 1197.7 | 17727.1 |
| Example 46-P1 | 5 | 2.9 | 1240.7 | 6287.5 |
| Example 46-P2 | 5 | 5.3 | 837.7 | 6094.3 |
| Example 49 | 3 | 10.5 | 420.0 | 4775.6 |
| Aripiprazole | 5 | 3.5 | 180.3 | 1037.8 |
| RP5063 | 5 | 3.8 | 195.0 | 1557.5 |

### 6.5 Test conclusion:

As can be seen from the pharmacokinetic experiment results in mice in the table, the compounds of the present invention can be absorbed rapidly after being administrated, showing good metabolic properties, and showing good exposures AUC and maximum plasma concentrations Cₘₐₓ.

## Claims

1. A compound of general formula (Ia), a stereoisomer thereof, or a pharmaceutically acceptable salt thereof: 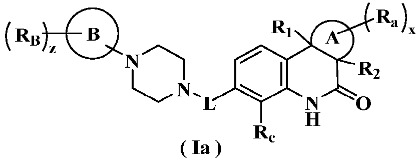 wherein:
L is -(CH₂)ₙ₁-, -(CH₂)ₙ₁O(CH₂)ₙ₂-, -(CH₂)ₙ₁S(CH₂)ₙ₂-, -(CH₂)ₙ₁NH(CH₂)ₙ₂-, -(CH₂)ₙ₁C(O)(CH₂)ₙ₂-, - (CH₂)ₙ₁S(O)₂(CH₂)ₙ₂-, -(CH₂)ₙ₁C(O)O(CH₂)ₙ₂-, -(CH₂)n₁OC(O)(CH₂)ₙ₂-, -(CH₂)ₙ₁C(O)S(CH₂)ₙ₂-, - (CH₂)ₙ₁SC(O)(CH₂)ₙ₂-, -(CH₂)ₙ₁C(O)NH(CH₂)ₙ₂-, or -(CH₂)ₙ₁NHC(O)(CH₂)ₙ₂-;
ring A is C₃₋₆ cycloalkyl or 3- to 6-membered heterocyclyl;
Rₐ is hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb};
R₁ and R₂ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb};
ring B is C₆₋₁₂ aryl, 5- to 12-membered heteroaryl, C₆₋₁₂ arylo C₃₋₈ cycloalkyl, C₆₋₁₂ arylo 4- to 8-membered heterocyclyl, or C₆₋₁₂ arylo 5- to 10-membered heteroaryl;
R_{B} is hydrogen, deuterium, halogen, hydroxy, cyano, amino, C₁₋₆ alkoxy, C₁₋₆ alkyl, C₁₋₆ deuterated alkyl, C₁₋₆ haloalkyl, oxo, -C(O)ORₐₐ, -NRₐₐR_{bb}, or -C(O)NRₐₐR_{bb};
R_{c} is hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb};
Rₐₐ and R_{bb} are each independently hydrogen, deuterium, hydroxy, cyano, amino, C₁₋₆ alkoxy, C₁₋₆ alkyl, C₁₋₆ deuterated alkyl, or C₁₋₆ haloalkyl;
x is an integer from 0 to 4;
z is an integer from 0 to 5; and
n1 and n2 are each independently an integer from 0 to 6.

2. The compound of general formula (Ia), the stereoisomer thereof, or the pharmaceutically acceptable salt thereof according to claim 1, wherein one or more of the following conditions are met:
(1) L is -(CH₂)ₙ₁O-, -(CH₂)ₙ₁OCH₂-, -(CH₂)₂O(CH₂)ₙ₂-, -(CH₂)ₙ₁S-, -(CH₂)ₙ₁SCH₂-, -(CH₂)₂S(CH₂)ₙ₂-, - (CH₂)ₙ₁C(O)NH-, -(CH₂)ₙ₁C(O)NHCH₂-, -(CH₂)ₙ₁NHC(O)CH₂-, or -(CH₂)₂NHC(O)(CH₂)ₙ₂-, preferably - (CH₂)ₙ₁O-, -(CH₂)ₙ₁S-, or -(CH₂)ₙ₁C(O)NH-, more preferably -(CH₂)ₙ₁O-, and further preferably -(CH₂)₃O-, - (CH₂)₄O-, or -(CH₂)₅O-;
(2) ring A is C₃₋₆ cycloalkyl, preferably 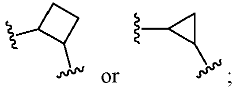
(3) Rₐ is hydrogen, deuterium, halogen, or C₁₋₆ alkyl, preferably hydrogen, deuterium, fluorine, chlorine, bromine, or C₁₋₃ alkyl, more preferably hydrogen, deuterium, fluorine, or methyl, and further preferably hydrogen, deuterium, or fluorine;
(4) R₁ and R₂ are each independently hydrogen, deuterium, halogen, cyano, or C₁₋₆ alkyl, preferably hydrogen, deuterium, fluorine, chlorine, bromine, cyano, or C₁₋₃ alkyl, and more preferably hydrogen, deuterium, fluorine, cyano, or methyl;
(5) ring B is 5- to 6-membered heteroaryl, phenyl, benzo C₃₋₈ cycloalkyl, benzo 4- to 8-membered heterocyclyl, or benzo 5- to 6-membered heteroaryl, preferably 5- to 6-membered heteroaryl, phenyl, benzo C₅₋₆ cycloalkyl, benzo 5- to 6-membered heterocyclyl, or benzo 5- to 6-membered heteroaryl, and more preferably a group as follows: 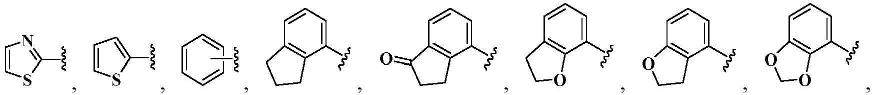 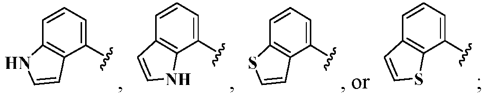
(6) R_{B} is hydrogen, deuterium, halogen, cyano, hydroxy, oxo, C₁₋₃ alkoxy, or C₁₋₃ alkyl, preferably hydrogen, deuterium, fluorine, chlorine, bromine, cyano, hydroxy, oxo, methoxy, or methyl, and more preferably hydrogen, fluorine, chlorine, cyano, hydroxy, oxo, methoxy, or methyl;
(7) R_{c} is hydrogen, deuterium, or halogen, preferably hydrogen, deuterium, fluorine, chlorine, or bromine, and more preferably hydrogen or fluorine;
(8) Rₐₐ and R_{bb} are each independently hydrogen, deuterium, C₁₋₆ alkyl, or C₁₋₆ deuterated alkyl, preferably hydrogen, deuterium, C₁₋₃ alkyl, or C₁₋₃ deuterated alkyl, and more preferably hydrogen, deuterium, methyl, ethyl, propyl, or isopropyl;
(9) z is an integer from 0 to 3;
(10) n1 is an integer from 3 to 5, preferably 4; and
(11) n2 is 0.

3. The compound of general formula (Ia), the stereoisomer thereof, or the pharmaceutically acceptable salt thereof according to claim 2, wherein one or more of the following conditions are met:
(1) 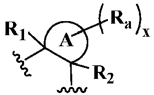 is a group as follows: 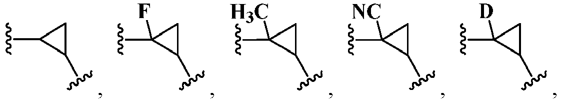 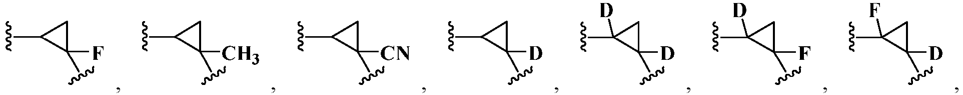 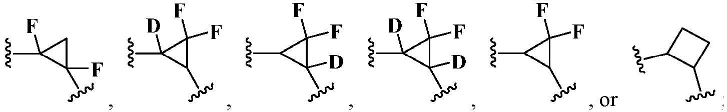 and
(2) 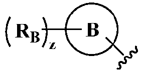 is a group as follows:

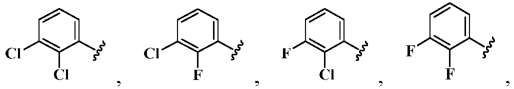

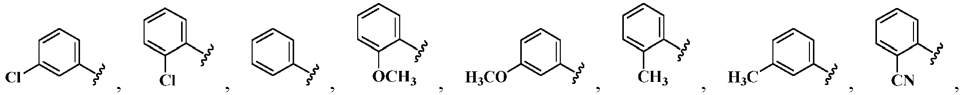

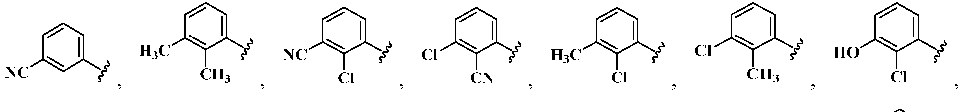

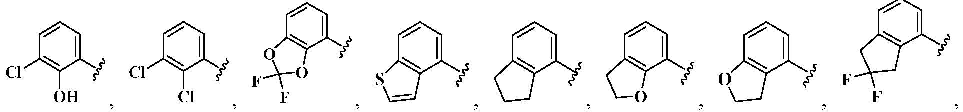

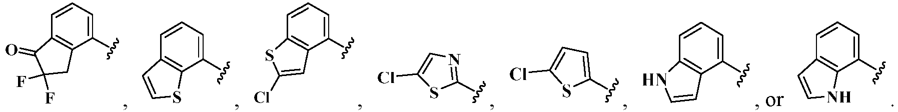

4. A compound of general formula (I), a stereoisomer thereof, or a pharmaceutically acceptable salt thereof: 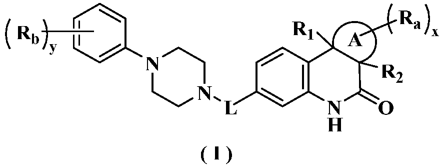 wherein:
L is -(CH₂)ₙ₁-, -(CH₂)ₙ₁O(CH₂)ₙ₂-, -(CH₂)ₙ₁S(CH₂)ₙ₂-, -(CH₂)ₙ₁NH(CH₂)ₙ₂-, -(CH₂)ₙ₁C(O)(CH₂)ₙ₂-, - (CH₂)ₙ₁S(O)₂(CH₂)ₙ₂-, -(CH₂)ₙ₁C(O)O(CH₂)ₙ₂-, -(CH₂)ₙ₁OC(O)(CH₂)ₙ₂-, -(CH₂)ₙ₁C(O)S(CH₂)ₙ₂-, - (CH₂)ₙ₁SC(O)(CH₂)ₙ₂-, -(CH₂)ₙ₁C(O)NH(CH₂)ₙ₂-, or -(CH₂)ₙ₁NHC(O)(CH₂)ₙ₂-;
ring A is C₃₋₆ cycloalkyl or 3- to 6-membered heterocyclyl;
Rₐ is hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb};
R₁ and R₂ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb};
R_{b} is hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb};
Rₐₐ and R_{bb} are each independently hydrogen, deuterium, hydroxy, cyano, amino, C₁₋₆ alkoxy, C₁₋₆ alkyl, C₁₋₆ deuterated alkyl, or C₁₋₆ haloalkyl;
x is an integer from 0 to 4;
y is an integer from 0 to 5; and
n1 and n2 are each independently an integer from 0 to 6.

5. The compound of general formula (I), the stereoisomer thereof, or the pharmaceutically acceptable salt thereof according to claim 4, wherein one or more of the following conditions are met:
(1) L is -(CH₂)ₙ₁O-, -(CH₂)ₙ₁OCH₂-, -(CH₂)₂O(CH₂)ₙ₂-, -(CH₂)ₙ₁S-, -(CH₂)ₙ₁SCH₂-, -(CH₂)₂S(CH₂)ₙ₂-, - (CH₂)ₙ₁C(O)NH-, -(CH₂)ₙ₁C(O)NHCH₂-, -(CH₂)ₙ₁NHC(O)CH₂-, or -(CH₂)₂NHC(O)(CH₂)ₙ₂-, preferably - (CH₂)ₙ₁O-, -(CH₂)ₙ₁S-, or -(CH₂)ₙ₁C(O)NH-, more preferably -(CH₂)ₙ₁O-, and further preferably -(CH₂)₃O-, - (CH₂)₄O-, or -(CH₂)₅O-,
(2) Rₐ is hydrogen, deuterium, halogen, or C₁₋₆ alkyl, preferably hydrogen, deuterium, fluorine, chlorine, bromine, or C₁₋₃ alkyl, more preferably hydrogen, deuterium, fluorine, or methyl, and further preferably hydrogen, deuterium, or fluorine;
(3) R₁ and R₂ are each independently hydrogen, deuterium, halogen, cyano, or C₁₋₆ alkyl, preferably hydrogen, deuterium, fluorine, chlorine, bromine, cyano, or C₁₋₃ alkyl, and more preferably hydrogen, deuterium, fluorine, cyano, or methyl;
(4) R_{b} is hydrogen, deuterium, halogen, cyano, hydroxy, C₁₋₃ alkoxy, or C₁₋₃ alkyl, preferably hydrogen, deuterium, fluorine, chlorine, bromine, cyano, hydroxy, methoxy, or methyl, and more preferably hydrogen, fluorine, chlorine, cyano, hydroxy, methoxy, or methyl;
(5) Rₐₐ and R_{bb} are each independently hydrogen, deuterium, C₁₋₆ alkyl, or C₁₋₆ deuterated alkyl, preferably hydrogen, deuterium, C₁₋₃ alkyl, or C₁₋₃ deuterated alkyl, and more preferably hydrogen, deuterium, methyl, ethyl, propyl, or isopropyl;
(6) ring A is C₃₋₆ cycloalkyl, preferably 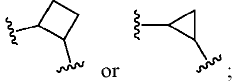
(7) y is an integer from 0 to 2;
(8) n1 is an integer from 3 to 5, preferably 4; and
(9) n2 is 0.

6. The compound of general formula (I), the stereoisomer thereof, or the pharmaceutically acceptable salt thereof according to claim 5, wherein one or more of the following conditions are met:
(1) 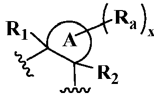 is a group as follows: 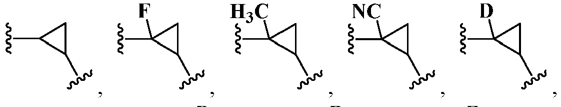 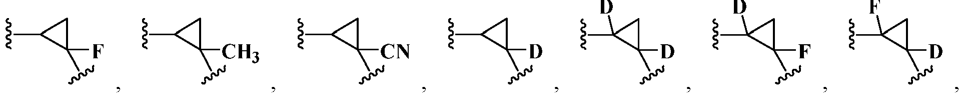 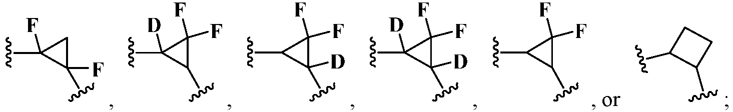 and
(2) 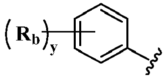 is a group as follows:

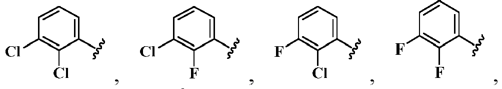

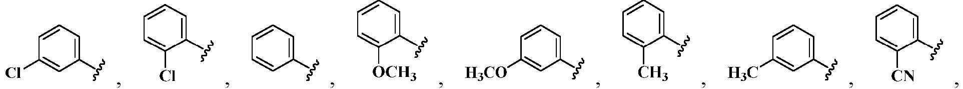

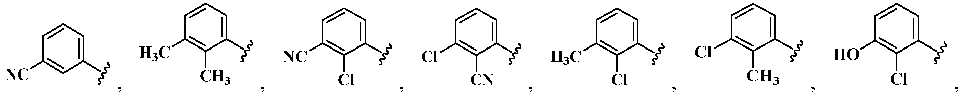

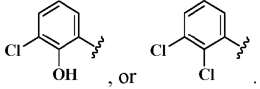

7. The compound of general formula (I), the stereoisomer thereof, or the pharmaceutically acceptable salt thereof according to any one of claims 4 to 6, wherein general formula (I) is further represented by general formula (II): 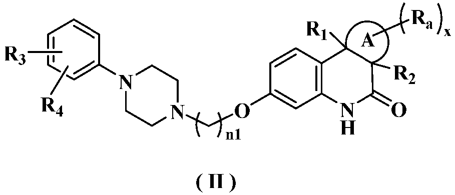 wherein:
R₃ and R₄ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, -C(O)ORₐₐ, -NRₐₐR_{bb}, or -C(O)NRₐₐR_{bb}, preferably hydrogen, deuterium, halogen, cyano, hydroxy, C₁₋₃ alkoxy, or C₁₋₃ alkyl, more preferably hydrogen, deuterium, fluorine, chlorine, bromine, cyano, hydroxy, methoxy, or methyl, and further preferably hydrogen, fluorine, chlorine, cyano, hydroxy, methoxy, or methyl;
Rₐₐ and R_{bb} are each independently hydrogen, deuterium, hydroxy, cyano, amino, C₁₋₆ alkoxy, C₁₋₆ alkyl, C₁₋₆ deuterated alkyl, or C₁₋₆ haloalkyl, preferably hydrogen, deuterium, C₁₋₆ alkyl, or C₁₋₆ deuterated alkyl, more preferably hydrogen, deuterium, C₁₋₃ alkyl, or C₁₋₃ deuterated alkyl, and further preferably hydrogen, deuterium, methyl, ethyl, propyl, or isopropyl; and
n1 is an integer from 2 to 6, preferably an integer from 3 to 5, and more preferably 4;
ring A, R₁, R₂, Rₐ, x, and 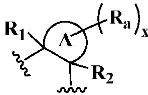 are as defined in any one of claims 4 to 6.

8. The compound of general formula (II), the stereoisomer thereof, or the pharmaceutically acceptable salt thereof according to claim 7, wherein 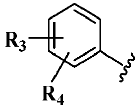 is a group as follows:

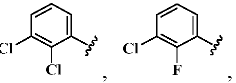

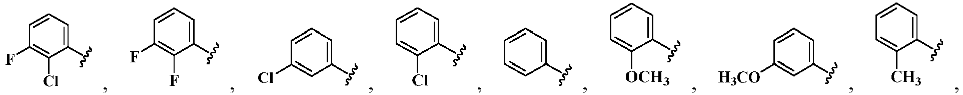

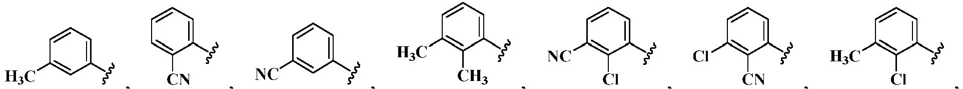

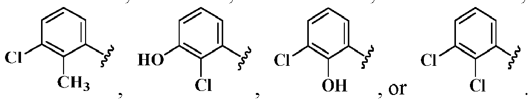

9. The compound of general formula (Ia), the stereoisomer thereof, or the pharmaceutically acceptable salt thereof according to any one of claims 1 to 3, wherein general formula (Ia) is further represented by general formula (III): 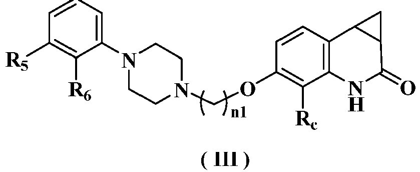 wherein:
R₅ and R₆ are each independently hydrogen, deuterium, halogen, cyano, hydroxy, C₁₋₃ alkoxy, or C₁₋₃ alkyl, preferably hydrogen, deuterium, fluorine, chlorine, bromine, cyano, hydroxy, methoxy, or methyl, and more preferably hydrogen, fluorine, chlorine, cyano, hydroxy, methoxy, or methyl;
or R₅ and R₆ are linked to the carbon atom to which they are attached to form C₃₋₈ cycloalkyl, 4- to 8-membered heterocyclyl, or 5- to 6-membered heteroaryl, the C₃₋₈ cycloalkyl, 4- to 8-membered heterocyclyl, and 5- to 6-membered heteroaryl optionally being further substituted with one or more substituents selected from hydrogen, deuterium, oxo and halogen,
preferably to form C₃₋₅ cycloalkyl, 5- to 6-membered heterocyclyl, or 5- to 6-membered heteroaryl, the C₃₋₅ cycloalkyl, 5- to 6-membered heterocyclyl, and 5- to 6-membered heteroaryl optionally being further substituted with one or more substituents selected from hydrogen, deuterium, oxo, fluorine, chlorine, and bromine,
more preferably to form a group as follows: 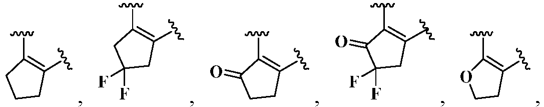 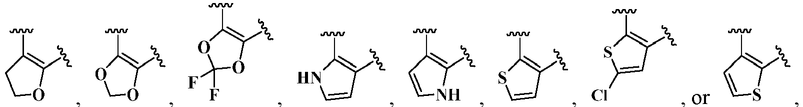
and further preferably to form a group as follows: 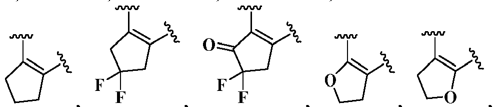 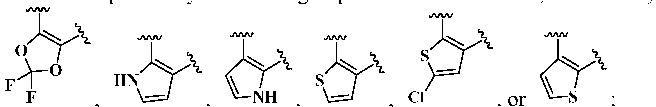
R_{c} is hydrogen, deuterium, or halogen, preferably hydrogen, deuterium, fluorine, chlorine, or bromine, and more preferably hydrogen or fluorine;
n1 is an integer from 2 to 6, preferably an integer from 3 to 5, and more preferably 4.

10. The compound of general formula (III), the stereoisomer thereof, or the pharmaceutically acceptable salt thereof according to claim 9, wherein 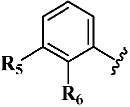 is a group as follows: 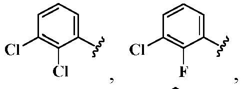 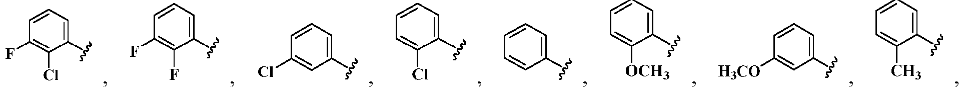 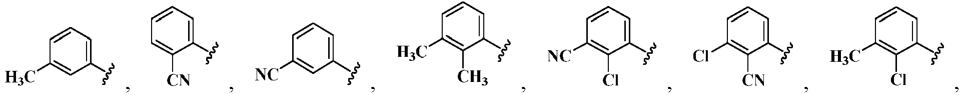 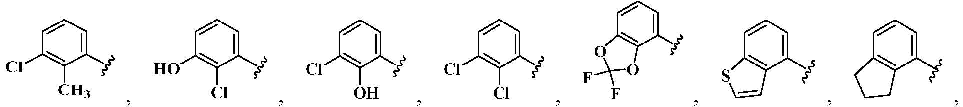 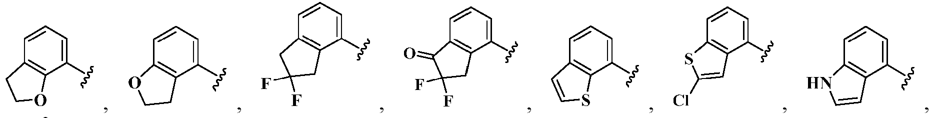 or

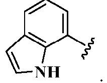

11. The compound, the stereoisomer thereof, or the pharmaceutically acceptable salt thereof according to any one of claims 1 to 10 wherein the compound is selected from any one of the following structures:

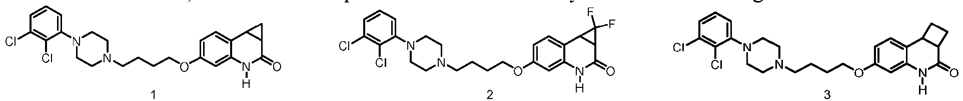

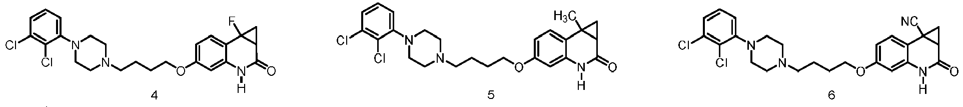

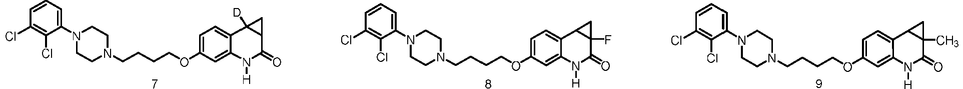

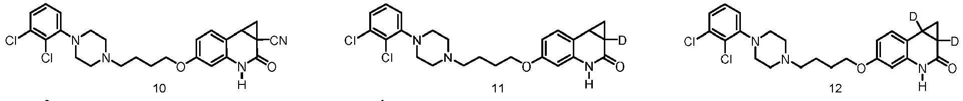

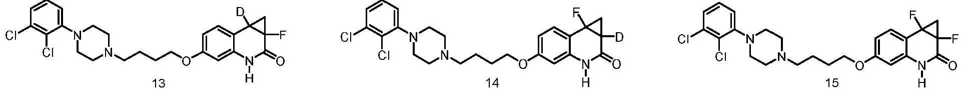

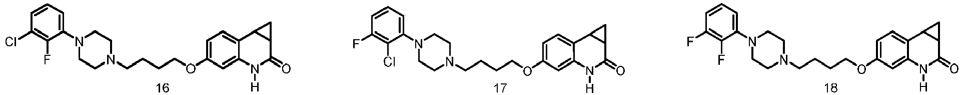

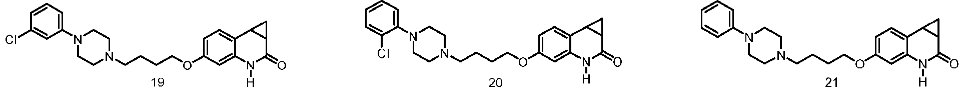

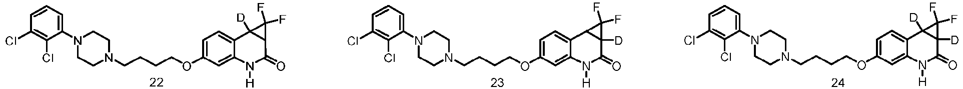

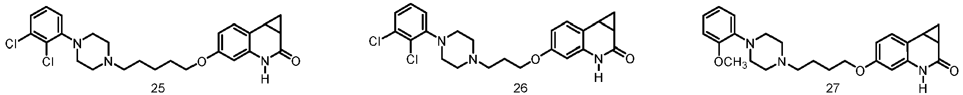

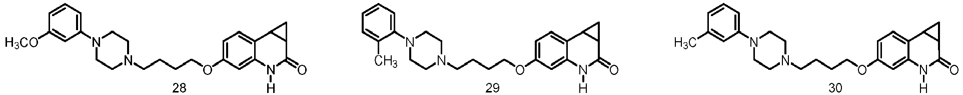

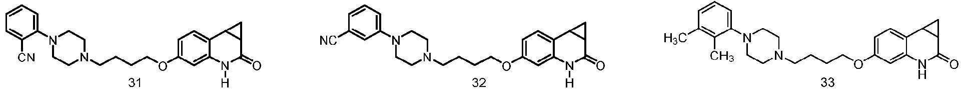

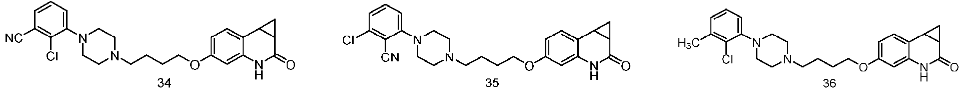

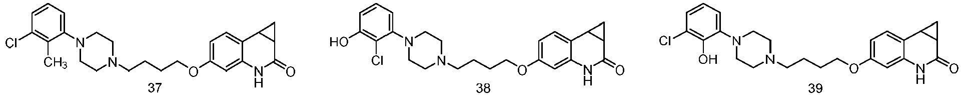

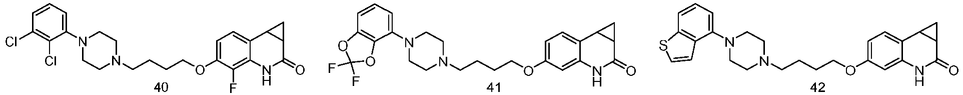

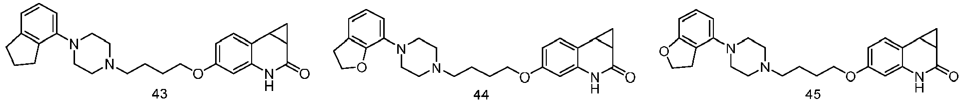

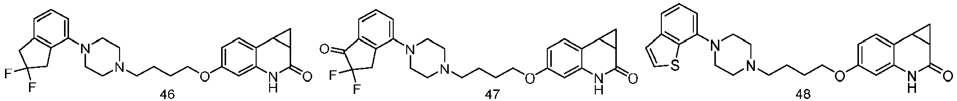

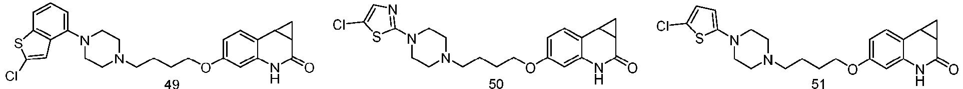

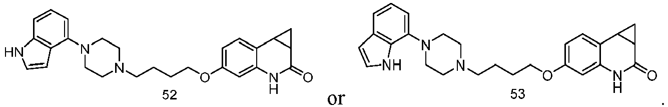

12. The compound, the stereoisomer thereof, or the pharmaceutically acceptable salt thereof according to any one of claims 1 to 10, wherein the compound is selected from any one of the following structures:

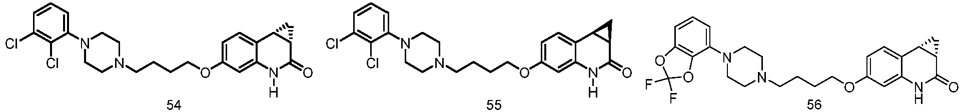

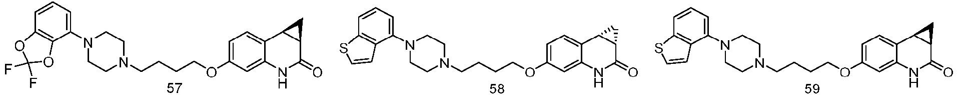

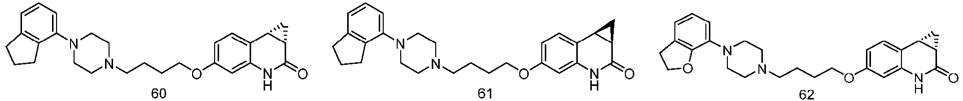

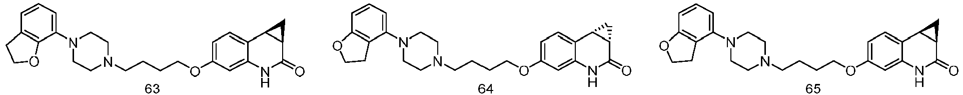

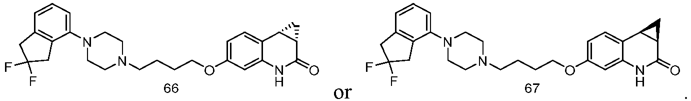

13. A method for preparing the compound of general formula (Ia), the stereoisomer thereof, or the pharmaceutically acceptable salt thereof according to claim 1, comprising the following step: 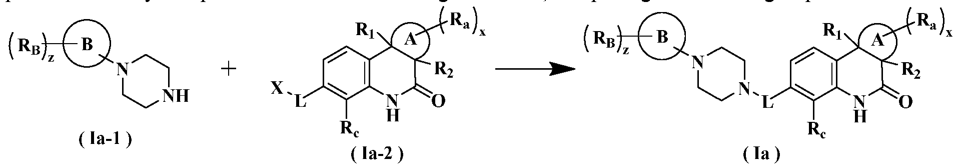 reacting a compound of general formula (Ia-1) with a compound of general formula (Ia-2) through a nucleophilic substitution reaction or reductive amination reaction to prepare the compound of general formula (Ia), the stereoisomer thereof, or the pharmaceutically acceptable salt thereof;
wherein:
X is halogen, preferably fluorine, chlorine, bromine, or iodine, and more preferably bromine;
ring A, ring B, L, R₁, R₂, Rₐ, R_{B}, R_{c}, x, and z are as described in claim 1.

14. A method for preparing the compound of general formula (I), the stereoisomer thereof, or the pharmaceutically acceptable salt thereof according to claim 4, comprising the following step: 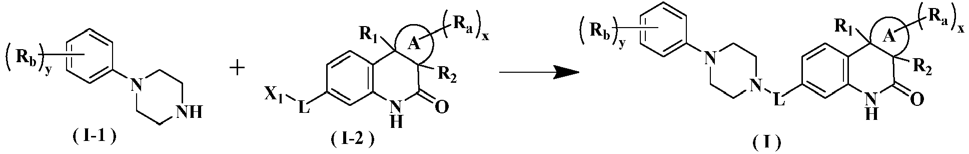
reacting a compound of general formula (1-1) with a compound of general formula (I-2) through a nucleophilic substitution reaction or reductive amination reaction to prepare the compound of general formula (I), the stereoisomer thereof, or the pharmaceutically acceptable salt thereof;
wherein:
X₁ is halogen, preferably fluorine, chlorine, bromine, or iodine, and more preferably bromine;
ring A, L, R₁, R₂, Rₐ, R_{b}, x, and y are as described in claim 4.

15. A pharmaceutical composition comprising a therapeutically effective dose of the compound, the stereoisomer thereof, or the pharmaceutically acceptable salt thereof according to any one of claims 1 to 12, and one or more pharmaceutically acceptable carriers or excipients.

16. Use of the compound, the stereoisomer thereof, or the pharmaceutically acceptable salt thereof according to any one of claims 1 to 12, or the pharmaceutical composition according to claim 15 in the preparation of a drug, wherein the drug is selected from one or more of 5-hydroxytryptamine, dopamine, and norepinephrine receptor regulators.

17. Use of the compound, the stereoisomer thereof, or the pharmaceutically acceptable salt thereof according to any one of claims 1 to 12, or the pharmaceutical composition according to claim 15 in the preparation of a drug for preventing and/or treating a central nervous system disease and/or a peripheral disease, wherein the central nervous system disease is a central nervous system disease associated with the transmission of one or more neurotransmitters selected from 5-hydroxytryptamine, dopamine, and norepinephrine in a mammal; the central nervous system disease is preferably selected from one or more of schizophrenia, epilepsy, pain, depression, anxiety, bipolar disorder, sleep disorder, neurodegenerative disease, Huntington's chorea, cognitive disorder, memory impairment, Alzheimer's disease, Parkinson's disease, post-traumatic stress syndrome, addictive diseases, withdrawal syndrome, autism, Down's syndrome, attention deficit hyperactivity disorder, Reye's syndrome, attention deficit hyperactivity disorder, panic disorder, obsessive-compulsive disorder, and sleep disorder; the peripheral disease is preferably selected from one or two of pulmonary fibrosis and pulmonary hypertension.
